(19) Europäisches Patentamt
European Patent Office
Office européen des brevets

(11) EP 4 142 703 B1

(12) # FASCICULE DE BREVET EUROPEEN

(45) Date de publication et mention de la délivrance du brevet:
**18.10.2023 Bulletin 2023/42**

(21) Numéro de dépôt: **21734182.5**

(22) Date de dépôt: **20.05.2021**

(51) Classification Internationale des Brevets (IPC):
*A61K 31/13* (2006.01) *A61K 31/14* (2006.01)
*A61K 45/06* (2006.01) *A61P 9/10* (2006.01)
*A61P 25/08* (2006.01) *A61P 25/28* (2006.01)
*A61P 25/32* (2006.01) *A61P 29/00* (2006.01)
*A61P 39/06* (2006.01)

(52) Classification Coopérative des Brevets (CPC):
(C-Sets disponibles)
**A61K 45/06; A61K 31/13; A61K 31/14; A61P 9/10; A61P 25/08; A61P 25/28; A61P 25/32; A61P 29/00; A61P 39/06** (Cont.)

(86) Numéro de dépôt international:
**PCT/FR2021/050929**

(87) Numéro de publication internationale:
**WO 2021/234324 (25.11.2021 Gazette 2021/47)**

(54) **COMPOSÉ ET COMPOSITION POUR INDUIRE UNE NEUROPROTECTION**

VERBINDUNG UND ZUSAMMENSETZUNG ZUR INDUKTION VON NEUROPROTEKTION

COMPOUND AND COMPOSITION FOR INDUCING NEUROPROTECTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.05.2020 FR 2005138**

(43) Date de publication de la demande:
**08.03.2023 Bulletin 2023/10**

(73) Titulaires:
- **REST THERAPEUTICS**
**34095 Montpellier cedex 5 (FR)**
- **UNIVERSITE DE MONTPELLIER**
**34090 Montpellier (FR)**
- **INSTITUT NATIONAL DE LA SANTE ET DE LA RECHERCHE MEDICALE (INSERM)**
**75013 Paris (FR)**
- **Ecole Pratique des Hautes Etudes**
**75014 Paris (FR)**

(72) Inventeurs:
- **RUBINSTENN, Gilles**
**75005 PARIS (FR)**
- **MAURICE, Tangui**
**34980 SAINT-GELY-DU-FESC (FR)**

(74) Mandataire: **A.P.I. Conseil**
**Immeuble Newton**
**4, rue Jules Ferry**
**64000 Pau (FR)**

(56) Documents cités:
**WO-A1-2009/062391 WO-A1-2013/064579**
**WO-A1-2014/191424 WO-A1-2017/191034**
**WO-A1-2019/115833 WO-A1-2020/232246**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



**(Cont. page suivante)**

• S. Couly ET AL: "Anti-amnesic and neuroprotective effects of Fluoroethylnormemantine (FENM) in a pharmacological mouse model of Alzheimer's disease", Int. J. Neuropsychopharmacology, 25 septembre 2020 (2020-09-25), pages 1-16, XP055764703, Extrait de l'Internet: URL:https://watermark.silverchair.com/pyaa075.pdf?token=AQECAHi208BE49Ooan9kkhW_Ercy 7Dm3ZL_9Cf3qfKAc485ysgAAAs0wggLJBgkqhkiG9w 0BBwagggK6MIICtgIBADCCAq8GCSqGSIb3DQEHATAe BglghkgBZQMEAS4wEQQM7dPBp4EGAa6g-ulbAgEQgI ICglLuJRaAZoOfeal1lETPmf5vg-8Vjqwp8PtDqT48 ZA5hkRxsBiO6l5ChSJVcN7JQQbc380Gs2ryQdpKgEi e5k_0u7Eib [extrait le 2021-01-13]

Remarques:
Le document complet y compris le(s) table(s) de référence et le(s) listage(s) de séquence sont téléchargeables sur le site internet de l'OEB

(52) Classification Coopérative des Brevets (CPC): (Cont.)

C-Sets
**A61K 31/13, A61K 2300/00;**
**A61K 31/14, A61K 2300/00**

## Description

L'invention s'intéresse au domaine des maladies neurodégénératives.

L'invention concerne un composé de formule (I) pour son utilisation pour induire une neuroprotection, chez un sujet en ayant besoin, tel que défini dans les revendications.

L'invention concerne en outre une combinaison comprenant le composé de formule (I) et au moins un autre composé tel que, mais non exclusivement, un modulateur des connexines, un inhibiteur de l'acétylcholinestérase, un modulateur positif de sigma 1, ou un anticorps visant à lutter contre la formation des plaques amyloïdes pour son utilisation pour induire une neuroprotection chez un sujet en ayant besoin, tel que défini dans les revendications.

## Art antérieur

L'Organisation Mondiale de la Santé (OMS) estime que d'ici 2050 le nombre de personnes âgées de plus de 60 ans devrait atteindre deux milliards. Ce vieillissement sans précédent de la population mondiale laisse entrevoir une forte pression des maladies chroniques liées au vieillissement sur les systèmes de santé. La démence est une de celles-ci. Ainsi, l'OMS estime que le nombre total de personnes atteintes de démence devrait dépasser 150 millions de personnes à l'horizon de 2050.

La démence se caractérise par une dégradation des fonctions cognitives notamment de la mémoire et du raisonnement qui impacte le comportement du malade et sa capacité à effectuer des tâches pourtant quotidiennes. La démence est un syndrome qui recouvre des pathologies aux étiologies très diverses qui affectent des zones différentes du cerveau et/ou d'autres régions du système nerveux central et impliquent notamment la neurodégénérescence et la mort des cellules neuronales. En lien direct avec le vieillissement, la dysfonction mitochondriale et le stress oxydatif jouent un rôle crucial dans la pathogenèse des maladies neurodégénératives. Ces maladies sont également souvent en lien avec une accumulation anormale de certaines protéines et/ou l'accumulation de protéines mutées et/ou anormalement repliées telle qu'observée dans les amyloïdoses à Aβ, les tauopathies, les synucléinopathies, l'agrégation de la superoxyde dismutase-1 (SOD1), de la polyglutamine, de la protéine TDP-43. Les maladies neurodégénératives, n'impliquent pas toutes, néanmoins au stade précoce, une détérioration des capacités cognitives. Les symptômes peuvent notamment être principalement moteurs. De même, elles ne sont pas toutes en lien avec le vieillissement, notamment quand elles sont liées à des mutations génétiques ou des accidents traumatiques.

La maladie d'Alzheimer est la forme la plus commune des pathologies neurodégénératives et est à l'origine de 60-70% des cas de démence. Les autres formes répandues de pathologies neurodégénératives sont notamment la démence vasculaire, la démence à corps de Lewy (démence parkinsonienne), la démence fronto-temporale (dégénérescence des lobes frontaux du cerveau), la maladie de Huntington, l'atrophie corticale postérieure, la maladie de Parkinson, la sclérose latérale amyotrophique, l'accident vasculaire cérébral.

L'excitotoxicité est couramment observée dans les tissus neuronaux lors de troubles neurologiques aigus et chroniques, tels que les pathologies neurodégénératives susmentionnées, mais aussi les traumatismes médullaires, les traumatismes crâniens, l'alcoolisme et le sevrage alcoolique (syndrome de Korsakoff). L'excitotoxicité correspond à la suractivation des récepteurs du glutamate sélectivement activés par le N-méthyl-D-aspartate (récepteurs NMDA) qui aboutit à la mort des cellules neuronales par apoptose, notamment, mais pas uniquement, en lien avec l'entrée massive de calcium dans les cellules et qui entraine une dysfonction mitochondriale.

Les agents bloquant les récepteurs NMDA sont donc connus comme neuroprotecteurs dans divers modèles aigus de neurotoxicité. WO 2009/062391 décrit de tels antagonistes dont la mémantine. Néanmoins, les doses requises pour atteindre l'effet de neuroprotection sont telles que des effets secondaires indésirables, voire contraires à l'effet attendu sur les capacités mnésiques, rendent ces molécules inutilisables chez l'homme. A ce jour, la mémantine (3,5-Diméthyl-1-adamantanamine), un inhibiteur non compétitif des canaux NMDA est le seul composé de cette classe à avoir été autorisé par certaines agences de santé nationales pour le traitement symptomatique de la maladie d'Alzheimer dans ses formes légères à modérément sévères, pour traiter les troubles cognitifs en lien avec la maladie d'Alzheimer. Néanmoins, chez l'homme, les effets uniquement symptomatiques s'avèrent limités. En octobre 2016, des métanalyses des données cliniques montrant, au dosage autorisé, une efficacité symptomatique au mieux modeste établie seulement à court terme et essentiellement sur les troubles cognitifs, ont conduit, en France, la Haute Autorité de Santé à juger la pertinence clinique de ces effets non clairement établie. Cela a abouti en 2018 au déremboursement de cette molécule du fait de : (i) l'absence de pertinence clinique de ses effets symptomatiques, de (ii) l'absence de démonstration d'efficacité sur les troubles du comportement, la qualité de vie, le délai d'entrée en institution, la mortalité, l'évolution de la maladie, la charge de la maladie sur les aidants, (iii) de son profil de tolérance et (iv) du risque élevé d'interactions médicamenteuses chez les sujets âgés. Ces effets modestes de la mémantine sont reportés par diverses métanalyses (*e.g.* Knight *et al.,* 2018).

Cette faible efficacité pourrait s'expliquer par les doses insuffisantes administrées pour obtenir, au niveau du système nerveux central, une concentration efficace correspondante aux $IC_{50}$ observées *in vitro* pour la molécule (Valis

*et al.* 2019). Cependant, les effets secondaires de la mémantine, notamment ses effets contraires amnésiques et neuropsychiatriques mais également secondaires et indésirables comme les troubles digestifs et cardiovasculaires, ne permettent pas d'envisager une augmentation des doses administrées. En outre, certains auteurs ont montré, dans des modèles animaux, que les effets contre la neurotoxicité n'étaient observés qu'à des doses de mémantine induisant des troubles neurocomportementaux sévères, notamment une atteinte sensorimotrice sévère ainsi qu'une altération significative de la mémoire (Creeley *et al.*, 2006).

Les inhibiteurs modérés des récepteurs NMDA constituent cependant toujours des candidats de choix dans le traitement de la neurodégénérescence. Il existe donc un fort besoin pour un tel inhibiteur, qui serait utilisable à des doses permettant d'envisager d'atteindre des doses efficaces dans le liquide céphalo-rachidien (LCR) pour induire l'inhibition de la neurotoxicité chez les patients et cela sans présenter des effets indésirables inacceptables et sans aggraver les symptômes cognitifs. En effet, les récepteurs NMDA à l'instar des autres récepteurs glutamatergiques jouent également un rôle dans la plasticité neuronale ; ainsi, si les agents bloquant ces récepteurs n'empêchent pas la transmission synaptique, il est connu que certains empêchent le déclenchement de la potentialisation à long terme : à mesure que l'on augmente la dose d'antagoniste, la plasticité synaptique diminue et les déficits mnésiques se renforcent.

Il est également connu que les différents antagonistes des récepteurs NMDA ont des modes d'actions différents, de sorte qu'il est difficile de prédire les effets d'un antagoniste à l'autre, d'un type de pathologie à l'autre et ce même pour des molécules ayant une structure proche.

La demande WO 2014/191424 décrit la 2-fluoroethyl normémantine (3-(2-fluoroéthyl)adamantan-1-amine, FENM) marquée au $^{18}F$ pour le marquage des récepteurs NMDA et leur visualisation par tomographie par émission de positons pour étudier la distribution de ces récepteurs et leur réaction à des traitements médicamenteux ou dans le développement de maladies neurodégénératives. Cette demande décrit une affinité de la FENM pour les récepteurs NMDA d'un ordre de grandeur comparable à celui de la mémantine.

La demande WO 2019/115833 décrit la FENM dans le traitement de troubles liés à l'anxiété et la dépression.

La demande WO 2013/064579 décrit une combinaison d'agent bloquant les connexines (tel que l'acide méclofénamique) avec un inhibiteur de l'acétylcholine estérase (tel que le donépézil) pour leur utilisation dans le traitement de troubles cognitifs. Néanmoins il est à noter que les inhibiteurs d'acétylcholine estérase, initialement autorisés dans le traitement symptomatique de la maladie d'Alzheimer, ont également été déremboursés en France en raison de leur faible efficacité.

Ainsi les patients font aujourd'hui face à l'absence de solution thérapeutique probante capable d'induire une neuroprotection dans le cadre de la neurodégénérescence ou d'atteinte neuronale aiguë.

**Problème technique**

L'invention a donc pour but de remédier aux inconvénients de l'art antérieur. En particulier, l'invention a pour but de proposer un composé pour son utilisation dans la neuroprotection ; ledit composé présentant un effet neuroprotecteur qui permet de prévenir les mécanismes de mort cellulaire des neurones et d'aboutir au maintien des capacités cognitives des sujets souffrant de pathologies notamment, mais pas uniquement, en lien avec l'excitotoxicité. En outre, la présente invention a pour but de proposer un composé ne présentant pas d'effets délétères sur la cognition tels que ceux observés pour les inhibiteurs des récepteurs NMDA, permettant ainsi d'élargir sans danger la gamme des doses administrables à l'homme pour le traitement symptomatique de la maladie d'Alzheimer. Enfin, la présente invention a pour but de proposer un composé efficace dans le traitement symptomatique des troubles cognitifs induits par la neurodégénérescence.

**Brève description de l'invention**

Les demandeurs ont découvert de manière tout à fait surprenante que la FENM possède des effets neuroprotecteurs significatifs dont la mémantine est dépourvue qui permettent de prévenir les mécanismes de mort cellulaire et d'aboutir au maintien des capacités cognitives dans un modèle murin comprenant par administration intracérébrale d'oligomères de peptide amyloïde-$\beta_{25-35}$ ($A\beta_{25-35}$). En outre, les demandeurs ont mis en évidence que la FENM ne présente pas les effets délétères pour la mémoire de la mémantine. Cela permet d'envisager une administration à des doses plus importantes que celles qui ont été autorisées pour la mémantine et ainsi d'en attendre un gain en efficacité. Enfin, il a été découvert que la FENM est également plus efficace que la mémantine dans le traitement symptomatique des troubles cognitifs induits par la neurodégénérescence.

Ainsi, un objet de la présente invention concerne un composé de formule (I) :

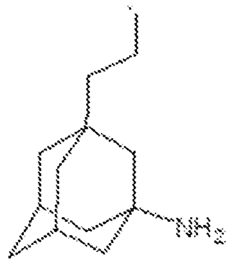 (I)

ou un de ses sels pharmaceutiquement acceptable pour son utilisation pour induire une neuroprotection, chez un sujet souffrant, étant suspecté de souffrir ou étant considéré à risque de souffrir d'une pathologie neurodégénérative.

Selon une caractéristique optionnelle, le sel pharmaceutiquement acceptable correspond à la formule (II) :

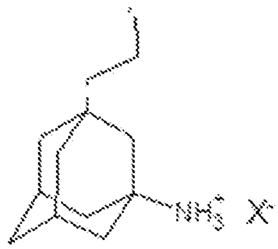 (II)

dans laquelle, $X^-$ désigne un contre anion choisi dans le groupe constitué par les ions chlorure, bromure, iodure, acétate, méthane sulfonate, benzène sulfonate, camphosulphonate, tartrate, dibenzoate, ascorbate, fumarate, citrate, phosphate, salicylate, oxalate, bromohydrate et tosylate.

Les inventeurs ont découvert que la FENM induit une protection importante contre les différentes composantes de la neurodégénération, ainsi :

- dans un des aspects de l'invention, la neuroprotection conférée par les composés de l'invention inclut la prévention ou la diminution de la neuroinflammation.

- dans un autre aspect de l'invention, la neuroprotection conférée par les composés de l'invention inclut la prévention ou la diminution du stress oxydant dans les cellules neuronales.

- dans un aspect de l'invention, la neuroprotection conférée par les composés de l'invention inclut la prévention ou la diminution de l'apoptose des cellules neuronales chez ledit sujet.

Il peut résulter de cette neuroprotection un effet positif sur les structures du cerveau, et notamment celles impliquées dans les processus cognitifs. Dans un aspect de l'invention, la neuroprotection conférée par les composés de l'invention inclut l'inhibition de la perte cellulaire de l'hippocampe chez ledit sujet. Les modifications de structure et de volume de l'hippocampe sont détectées en imagerie médicale et peuvent permettre de suivre l'efficacité du traitement.

La neuroprotection conférée par les composés de formule (I) résulte également en la préservation des capacités cognitives du sujet. Ainsi, dans un aspect de l'invention, la neuroprotection conférée par les composés de l'invention inclut la prévention ou la diminution des altérations des capacités cognitives du sujet, en particulier, la prévention ou la diminution des altérations :

- de sa mémoire à court terme,
- de sa mémoire à moyen terme,
- de sa mémoire spatiale, ou
- de ses capacités de reconnaissance et/ou d'apprentissage.

Les composés de formule (I) sont particulièrement efficaces pour induire une neuroprotection contre la toxicité des agrégats toxiques de protéines, tels que $A\beta_{25-35}$ oligomérisé Ainsi, dans un aspect de l'invention, la neuroprotection inclut la protection contre la toxicité des agrégats de β-amyloïde de ses fragments ou de ses oligomères, chez ledit sujet.

La neuroprotection conférée par les composés de formule (I) permet de lutter contre des mécanismes à la base de nombreuses pathologie neurodégénératives. Ainsi, dans un de ses aspect, l'invention concerne également le

composé de formule (I) pour son utilisation chez un sujet souffrant, étant suspecté de souffrir ou étant considéré à risque de souffrir d'une pathologie du système nerveux central sélectionnée parmi les tauopathies, synucléinopathies, les amyloïdopathies, la maladie d'Alzheimer, la maladie de Parkinson, l'atrophie multisystématisée, la maladie de Huntington, l'atrophie corticale postérieure, la maladie de Pick, l'épilepsie, la démence vasculaire, la démence fronto-temporale, la démence à corps de Lewy, la sclérose latérale amyotrophique, le syndrome de Korsakoff, le sevrage alcoolique, l'ischémie, l'ischémie néonatale, les traumatismes crânien, l'accident vasculaire cérébral, de préférence la maladie d'Alzheimer.

De par son mode d'action supposé, le composé de formule(I) est particulièrement adapté pour être utilisé en combinaison avec des composés ciblant d'autres aspects ou voies impliquées dans les processus neurodégénératifs. Ainsi un objet de l'invention concerne donc une combinaison du composé de formule (I) avec :

- au moins un inhibiteur de l'acétylcholinestérase, de préférence sélectionné parmi le donépézil, la rivastigmine, la galantamine, ou un de leurs sels pharmaceutiquement acceptables,
- au moins un agent inhibiteur des connexines, de préférence sélectionné parmi l'acide méclofénamique, enoxolone, la méfloquine et le 2-amino ethoxy diphényl borate (APB), ou un de leurs sels pharmaceutiquement acceptables,
- l'aducanumab ou un de ses fragments se liant à un antigène capable de lutter contre la toxicité de agrégats de β-amyloïde, de ses fragments ou de ses oligomères, ou
- au moins un modulateur positif de sigma 1 tel que ceux décrits dans la demande WO2017191034 ou un de leurs sels pharmaceutiquement acceptables, de préférence sélectionné parmi 2-(2-Chlorophényl)-2-oxo-3,3,5,5-tétraméthyl-[1 ,4,2]-oxazaphosphinane ; 2-(4-Chlorophényl)-2-oxo-3,3,5,5-tétraméthyl-[1 ,4,2]-oxazaphosphinane ; 2-(3,5-Dichlorophényl)-2-oxo-3,3,5,5-tétraméthyl-[1 ,4,2]-oxazaphosphinane ; 2-(2,3-Dichlorophényl)-2-oxo-3,3,5,5-tétraméthyl-[1 ,4,2]-oxazaphosphinane ; 2-(3-Fluorophényl)-2-oxo-3,3,5,5-tétraméthyl-[1 ,4,2]-oxazaphosphinane ; 2-(4-Fluorophényl)-2-oxo-3,3,5,5-tétraméthyl-[1 ,4,2]-oxazaphosphinane ; 2-(3-Nitrophényl)-2-oxo-3,3,5,5-tétramethyl-[1 ,4,2]-oxazaphosphinane ; 2-(4 benzyloxycarbamoylphényl)-2-oxo-3,3,5,5-tétraméthyl-[1 ,4,2]-oxazaphosphinane ; 2-(Pyridin-2-yl)-2-oxo-3,3,5,5-tétraméthyl[1 ,4,2]-oxazaphosphinane ; 2-(Pyridin-3-yl)-2-oxo-3,3,5,5-tétraméthyl[1 ,4,2]-oxazaphosphinane ; 2-(Pyridin-4-yl)-2-oxo-3,3,5,5-tétraméthyl[1 ,4,2]-oxazaphosphinane ; 2-(Pyrimidin-2-yl)-2-oxo-3,3,5,5-tétraméthyl-[1 ,4,2]-oxazaphosphinane ; 2-(Pyrimidin-5-yl)-2-oxo-3,3,5,5-tétraméthyl-[1 ,4,2]-oxazaphosphinane ; 2-(3-aminophényl)-2-oxo-3,3,5,5-tétraméthyl-[1 ,4,2]-oxazaphosphinane ; 2-(4-aminophényl)-2-oxo-3,3,5,5-tétraméthyl-[1 ,4,2]-oxazaphosphinane ; 2-(3-chlorophényl)-N-méthyl-2-oxo-3,3,5,5-tétraméthyl-[1 ,4,2]-oxazaphosphinane ; 2-(3-chlorophényl)-2-thiono-3,3,5,5-tétraméthyl-[1 ,4,2]-oxazaphosphinane ; 2-(3-Chlorophényl)-2-oxo-3,3,5,5-tétraméthyl-[1 ,4,2]-oxazaphosphinane ; 2-morpholin-4-yléthyl 1-phénylcyclohexane-1-carboxylate) ; (+)-pentazocine ; 1,13-diméthyl-10-prop-2-enyl-10-azatricyclo[7.3.1.02,7]trideca-2(7),3,5-trien-4-ol ; 1-[2-(3,4-diméthoxyphényl)éthyl]-4-(3-phénylpropyl)pipérazine ; 1-(2,2-Diphényltétrahydro-3-furanyl)-N,N-diméthylméthanamine ; 2-{[(E)-{5-Méthoxy-1-[4-(trifluorométhyl)phényl]pentylidène}amino]oxy}éthanamine ; N-(1-benzylpipéridin-4-yl)-4-iodobenzamide ; (5E)-N-(Cyclopropylméthyl)-N-méthyl-3,6-diphényl-5-hexén-3-amine ; 1-{3-[4-(3-Chlorophényl)-1-pipérazinyl]propyl}-5-méthoxy-3,4-dihydro-2(1H)-quinoléinone ; (1S,2R)-N-[2-(3,4-Dichlorophényl)éthyl]-N-méthyl-2-(1 - pyrrolidinyl)cyclohexanamine) ; 6-[(4-benzylpipérazin-1-yl)méthyl]-2,3-diméthoxyphénol) ; 4-(3-(méthylsulfonyl)phényl)-1-propylpipéridine ou un de leurs sels pharmaceutiquement acceptables, de préférence le 2-(3-Chlorophényl)-2-oxo-3,3,5,5-tétraméthyl-[1 ,4,2]-oxazaphosphinane ou le 2-morpholin-4-yléthyl 1-phénylcyclohexane-1-carboxylate ou un de ses sels pharmaceutiquement acceptable pour son utilisation pour induire une neuroprotection, chez un sujet souffrant. étant suspecté de souffrir ou à risque de souffrir d'une pathologie du système nerveux central susmentionnée, et particulièrement un sujet souffrant, étant suspecté de ou à risque de souffrir de la maladie d'Alzheimer.

Le profil d'activité du composé de formule (I) montre une neuroprotection sur des intervalles de doses importants, et ce sans effet délétères sur les capacités cognitives des sujets traités. Ainsi des dosages plus importants que ceux aujourd'hui autorisés pour la mémantine, et donc un traitement plus efficace, sont possibles. Selon un autre aspect, l'invention concerne donc le composé de formule (I) pour induire une neuroprotection chez un sujet en ayant besoin, tel que défini dans les revendications, ledit composé étant administré oralement à une dose supérieure à 20 mg par jour, de préférence supérieure ou égale à 30 mg par jour, optionnellement au sein d'une combinaison telle décrite ci-dessus.

D'autres avantages et caractéristiques de l'invention apparaitront à la lecture de la description suivante donnée à titre d'exemple illustratif et non limitatif, en référence aux Figures annexées qui représentent :

**Figures**

**Figure 1**, schémas expérimentaux d'administration du peptide $A\beta_{25-35}$ oligomérisé, des composés tests et de la réalisation des tests comportementaux d'évaluation des capacités cognitives des animaux. (**A, B**) Evaluation des effets symptomatiques sur l'amnésie induite par le peptide $A\beta_{25-35}$ oligomérisé. (**C**) Evaluation de l'effet neuroprotecteur des composés test. YMT, labyrinthe en Y (*Y-maze test* en langue anglo-saxonne) ; PAT, test d'évitement passif (*Passive Avoidance Test* en terminologie anglo-saxonne) ; ORT, test de reconnaissance d'objet; WMT, test d'apprentissage spatial en la piscine de Morris; †, sacrifice ; flèche noire : injection intracérébroventriculaire du peptide $A\beta_{25-35}$ oligomérisé; flèches grises : administration des composés tests/véhicule.

**Figure 2,** effet de la mémantine (MEM) et de la FENM (0.3 mg/Kg i.p.), sur les niveaux d'interleukine-6 (IL-6) (**A**), de peroxydation des lipides (**B**), ou de ratio Bax/Bcl-2 (**C**) dans des homogénats d'hippocampe de souris intoxiquées ou non par le peptide $A\beta_{25-35}$ oligomérisé. ANOVA: **A**, $F_{(3,22)} = 2.53$, $p > 0.05$ ; **B**, $F_{(3,21)} = 4.33$, $p < 0.05$ ; **C**, $F_{(3,22)} = 0.763$, $p > 0.05$.* $p < 0.05$, *** $p < 0.001$ vs. (V+V); # $p < 0.05$ vs. (V+$A\beta_{25-35}$), test de Dunnett. Le nombre de souris par groupe est indiqué dans chaque colonne. V : solution véhicule. Le pourcentage d'augmentation induit par $A\beta_{25-35}$ oligomérisé par rapport au groupe témoin non intoxiqué non traité (V+V) est indiqué au-dessus des colonnes.

**Figure 3,** effet de la mémantine (MEM) et de la FENM (0.3 mg/Kg i.p.), sur la réaction astrogliale à la suite de l'administration de peptide $A\beta_{25-35}$ oligomérisé. Quantification en inmunohistochimie de GFAP dans le *stratum radiatum* (**A**, Rad), le *stratum moleculare* (**B**, Mol), la couche polymorphe du gyrus denté (**C**, PoDT) de l'hippocampe et l'aire latérale pariétale associative (**D**, LPTA) ANOVA: **A,** $F_{(3,22)} = 5.06$, $p < 0.01$ ; **B,** $F_{(3,23)} = 4.50$, $p < 0.05$ ; **C,** $F_{(3,23)} = 3.24$, $p < 0.05$ ; **D,** $F_{(3,22)} = 3.71$, $p < 0.05$. * $p < 0.05$, *vs.* (V+V); # $p < 0.05$, ## $p < 0.01$ vs. ($A\beta_{25-35}$+V) ; test de Dunnett. V : solution véhicule. Le nombre de souris par groupe est indiqué dans chaque colonne. Le pourcentage d'augmentation induit par $A\beta_{25-35}$ oligomérisé par rapport au groupe témoin non intoxiqué non traité (V+V) est indiqué au-dessus des colonnes.

**Figure 4,** effet de la mémantine (MEM) et de la FENM (0.3 mg/Kg i.p.), sur la réaction microgliale à la suite de l'administration de peptide $A\beta_{25-35}$ oligomérisé. Quantification en inmunohistochimie du marqueur Iba-1 dans le *stratum radiatum* (**A**, Rad), *stratum moleculare* (**B**, Mol), la couche polymorphe du gyrus denté (**C**, PoDT) de l'hippocampe et l'aire latérale pariétale associative (**D**, LPTA) ANOVA: **A,** $F_{(3,23)} = 3.22$, $p < 0.05$; **B,** $F_{(3,22)} = 2.86$, $p > 0.05$ ; **C**, $F_{(3,23)} = 3.38$, $p < 0.05$; **D**, $F_{(3,23)} = 6.43$, $p < 0.01$. * $p < 0.05$, ** $p < 0.01$ *vs.* (V+V); # $p < 0.05$, ## $p < 0.01$ vs. ($A\beta_{25-35}$+V); test de Dunnett. V : solution véhicule. Le nombre de souris par groupe est indiqué dans chaque colonne. Le pourcentage d'augmentation induit par $A\beta_{25-35}$ oligomérisé par rapport au groupe témoin non intoxiqué non traité (V+V) est indiqué au-dessus des colonnes.

**Figure 5,** effet neuroprotecteur de la mémantine (MEM) et de la FENM (0.3 mg/Kg i.p.) sur l'altération de la mémoire spatiale de référence (piscine de Morris) et de l'apprentissage induite par l'intoxication aux oligomères de $A\beta_{25-35}$. Le temps passé dans le quart nord-est, dit quart d'entrainement (T), ou dans les autres quarts (o) a été analysé par surveillance vidéo.°°° $p < 0.001$ *vs.* 15 s; t-test pour un échantillon ; *** $p < 0.001$ *vs.* o quadrants. Veh : solution véhicule; Sc. $A\beta$ : peptide contrôle.

**Figure 6,** effet symptomatique de la mémantine (**A**) et de la FENM (**B**) (0.1-10 mg/Kg i.p.) sur l'altération de la mémoire induite par le peptide $A\beta_{25-35}$ oligomérisé dans le test du labyrinthe en Y. ANOVA: **A**, $F_{(6,83)} = 2.62$, $p < 0.05$ ; **B**, $F_{(6,89)} = 4.94$, $p < 0.001$. ). ** $p < 0.01$, *** $p < 0.001$ *vs.* (Sc. $A\beta$ +V); # $p < 0.05$, ## $p < 0.01$ vs. (V+$A\beta_{25-35}$), test de Dunnett, V : solution véhicule ; Sc. $A\beta$ : peptide contrôle. Le nombre de souris par groupe est indiqué dans chaque colonne.

**Figure 7,** effet symptomatique de la mémantine et de la FENM (0.1-10 mg/Kg i.p.) sur l'altération de la mémoire induite par l'intoxication aux oligomères de $A\beta_{25-35}$ dans le test de l'évitement passif. Les résultats sont des données non-paramétriques et sont présentés en médiane et écarts 25%-75%. Kruskal-Wallis ANOVA: **A**, $H = 23.4$, $p < 0.001$ ; **B,** $H = 19.5$, $p < 0.01$, in (d). * $p < 0.05$, ** $p < 0.01$, *** $p < 0.001$ vs. (Sc.$A\beta$+V) ; # $p < 0.05$, ## $p < 0.01$ vs. (V+$A\beta_{25-35}$); test de Dunn. V : solution véhicule ; Sc. $A\beta$ : peptide contrôle. Le nombre de souris par groupe est indiqué dans chaque colonne.

**Figure 8,** effet symptomatique de la mémantine et de la FENM (0.3 mg/Kg i.p.) sur l'altération des capacités

mnésiques complexes dans le test du hameau (Hamlet test®). Indice de désorientation est calculé à partir des erreurs (**A**) ou des latences (**B**). ° $p < 0.05$ *vs.* niveau zéro, t-test pour un échantillon. V: solution véhicule ; Sc. Aβ : peptide contrôle.

## Description de l'invention

La présente invention concerne la 2-fluoroéthyl normémantine (FENM) pour son utilisation pour induire une neuroprotection chez un sujet en ayant besoin, tel que défini dans les revendications. L'invention, telle que définie dans les revendications, concerne également des combinaisons de FENM pour leur utilisation pour induire ladite neuroprotection.

Il a en effet été découvert, comme le montre la partie expérimentale, que la FENM protège les cellules neuronales et évite la mort cellulaire induite en diminuant l'apoptose, le stress oxydant dans lesdites cellules et en diminuant la neuroinflammation au niveau du cerveau. Ceci aboutit à la prévention des déficits cognitifs qui en résultent. Ceci aboutit également à une efficacité dans la correction symptomatique des troubles cognitifs induits par une neurotoxicité.

### Définitions

Dans le contexte de la présente invention, la mention d'un médicament ou d'un composé spécifique inclut non seulement le médicament ou le composé spécifiquement nommé, mais également tout sel, hydrate, racémate, composition énantiomériquement pure, pharmaceutiquement acceptable, de la molécule active du médicament ou dudit composé. De manière préférée, la mention d'un composé inclut le composé spécifiquement nommé, ainsi que tout sel, hydrate, racémate, composition énantiomériquement pure, pharmaceutiquement acceptable dudit composé. De manière plus préférée, la désignation d'un composé est destinée à désigner le composé comme spécifiquement désigné en soi, ainsi que tout sel pharmaceutiquement acceptable de celui-ci.

Par « sels pharmaceutiquement acceptables », on entend au sens de l'invention, un sel d'addition d'acide inorganique ou organique pharmaceutiquement acceptable et relativement non toxique d'un composé de la présente invention. La formation de sel pharmaceutique consiste à coupler une molécule de médicament acide, basique ou zwitterionique avec un contre-ion pour créer une version saline du médicament. Une grande variété d'espèces chimiques peut être utilisée dans la réaction de neutralisation. Les sels pharmaceutiquement acceptables de l'invention comprennent donc ceux obtenus en faisant réagir le composé principal, fonctionnant comme une base, avec un acide inorganique ou organique pour former un sel, par exemple, des sels d'acide acétique, d'acide nitrique, d'acide tartrique, d'acide chlorhydrique, sulfurique acide, acide phosphorique, acide méthane sulfonique, acide camphre sulfonique, acide oxalique, acide maléique, acide succinique ou acide citrique. Les sels pharmaceutiquement acceptables de l'invention comprennent également ceux dans lesquels le composé principal fonctionne comme un acide et est mis à réagir avec une base appropriée pour former, par exemple, des sels de sodium, de potassium, de calcium, de magnésium, d'ammonium ou de choline. Bien que la plupart des sels d'un principe actif donné soient bioéquivalents, certains peuvent avoir, entre autres, des propriétés de solubilité ou de biodisponibilité accrues. La sélection du sel est maintenant une opération standard courante dans le processus de développement de médicaments comme enseigné par Stahl et Wermuth dans leur manuel (Stahl and Wermuth).

Le terme « neuroprotection » s'entend, au sens de l'invention, de la prévention ou du ralentissement de la progression d'une maladie affectant le système nerveux central ou périphérique en arrêtant ou au moins en ralentissant la perte de fonctionnalité des cellules nerveuses, la dégénérescence ou la perte des cellules de ces systèmes, notamment des neurones, conduisant à un déclin cognitif. Ainsi un sujet ayant besoin de neuroprotection se définit comme un sujet souffrant, étant suspecté de souffrir ou étant considéré à risque de souffrir d'une pathologie en lien avec la mort cellulaire neuronale et/ou la neurodégénérescence, notamment en lien, mais pas uniquement, avec l'excitotoxicité. Ces pathologies sont, par exemple, des pathologies neurodégénératives telles que des tauopathies, synucléopathies ou amyloïdopathies telles que la maladie d'Alzheimer, la maladie de Parkinson, l'atrophie multisystématisée, la démence à corps de Lewy, la dégénérescence cortico-basale, la maladie de Pick, la démence fronto-temporale, l'atrophie corticale postérieure. D'autres pathologies neurodégénératives sont par exemple la maladie de Huntington, la sclérose latérale amyotrophique, l'épilepsie, la démence vasculaire, le syndrome de Korsakoff, ou des pathologies neuronales aiguës telles que le sevrage alcoolique, l'ischémie, l'ischémie néonatale, les traumatismes crâniens, l'accident vasculaire.

Selon l'invention, un sujet ayant besoin de neuroprotection est un sujet souffrant, étant suspecté de souffrir ou étant considéré à risque de souffrir d'une pathologie neurodégénérative.

Par « sujet », on entend décrire ici n'importe quel membre du règne animal, de manière préférée les mammifères et de manière encore plus préférée l'homme. Dans un autre mode de réalisation préféré, les abeilles, insectes qui présentent également un système NMDA contrôlant la génération de la mémoire, sont également incluses.

Les termes "combinaison" ou "traitement combiné" ou "thérapie combinée" s'entendent d'une combinaison à base du composé de formule (I) ou de l'un de ses sels pharmaceutiquement acceptable (autrement dit, la FENM), et

d'au moins un autre composé ou médicament coadministré au dit sujet dans le but d'obtenir un effet biologique. La FENM et ledit au moins un autre composé, dans cette combinaison, peuvent être administrés ensemble ou séparément, concomitamment ou séquentiellement. Quand ils sont administrés ensemble, ils peuvent l'être au sein d'une composition unique comprenant la FENM et ledit au moins un autre composé ou médicament. En d'autres termes, la FENM et ledit au moins un autre composé ou médicament sont alors formulés ensemble. Alternativement, ils peuvent être administrés séparément audit sujet, par une voie d'administration identique ou différente. Ainsi, par exemple, la FENM peut être administrée oralement et ledit au moins autre composé avec lequel la FENM est coadministrée peut être injecté audit sujet, par exemple, par voie intraveineuse ou sous cutanée. Dans un autre mode de réalisation, par exemple, la FENM peut être administrée oralement et ledit au moins un autre composé avec lequel la FENM est coadministrée peut être également administré oralement audit sujet. De manière préférée, la séquence d'administration des principes actifs de la combinaison est telle que lesdits principes actifs ou les métabolites actifs exercent leurs effets biologiques au même moment, de manière que le sujet bénéficie de l'effet maximal de ladite combinaison. Ainsi, de manière particulièrement préférée, la FENM et ledit au moins un autre composé ou médicament sont administrés de façon à atteindre leur concentration maximale dans le plasma ou le liquide céphalorachidien, de préférence le liquide céphalorachidien, au même moment.

Les « peptides A bêta », ou « Aβ » ou « peptides bêta-amyloïdes », ou « peptides amyloïdes », ou encore « bêta-amyloïde » résultent du clivage par les gamma et les bêtasécrétases de la protéine APP (pour «Amyloid Protein Precursor» en langue anglaise) localisée à la membrane des neurones. Ils peuvent avoir, chez l'homme des tailles variables (principalement de 38 à 42 acides aminés) et se présenter sous forme d'assemblages oligomériques de taille et de solubilité variables. Chaque type d'oligomère présente potentiellement un caractère toxique entraînant une altération de la structure, de la fonction et de plasticité synaptique qui aboutit finalement à la mort neuronale (Pike *et al.,* 1991). Cette altération des synapses est à l'origine du dysfonctionnement des régions impliquées dans les processus de mémoire et d'apprentissage. Ces fragments se retrouvent également dans les plaques dites amyloïdes qui s'accumulent avec l'âge ou dans certaines maladies. *In vivo,* chez l'homme, $A\beta_{1-42}$ a une forte propension à s'auto-agréger ; les formes familiales de la maladie d'Alzheimer s'accompagnent d'un accroissement du niveau relatif de peptide $A\beta_{1-42}/A\beta_{1-40}$, et c'est plutôt ce niveau relatif qui serait indicatif du diagnostic de la pathologie plutôt que l'accumulation des peptides $A\beta_{1-42}$ et/ ou $A\beta_{1-40}$. $A\beta_{25-35}$ est un fragment toxique pour les cellules neuronales *in vitro.* Chez la souris et le rat, l'injection intracérébroventriculaire (icv) d'oligomères du peptide $A\beta_{25-35}$ est un des modèles utilisés pour étudier la neurodégénérescence induite par les oligomères Aβ, notamment dans le cadre de la maladie d'Alzheimer et pour tester des candidats médicaments pour cette maladie (Maurice *et al.,* 1996). Par neurotoxicité des « peptides A bêta », ou « Aβ » ou « peptides bêta-amyloïdes », ou « peptides amyloïdes », ou encore « bêta-amyloïde », on entend donc, au sens de l'invention, la neurotoxicité induite par tout oligomère et/ou agrégat formé d'un ou de peptide(s) résultant du clivage de la protéine APP.

Les demandeurs ont découvert que la FENM permet d'induire efficacement une neuroprotection contre la toxicité induite par l'injection intracérébrale d'oligomères de $A\beta_{25-35}$ dans un modèle animal. Les oligomères de $A\beta_{25-35}$ sont connus pour induire un processus inflammatoire, un stress oxydant et une apoptose des cellules neuronales quand ils sont injectés dans le cerveau de modèles animaux. De manière tout à fait surprenante l'administration de FENM aux animaux dès le jour de l'injection des oligomères de $A\beta_{25-35}$ permet de réduire les niveaux des marqueurs de l'apoptose et du stress mitochondrial, de la neuroinflammation, et de réduire la mortalité cellulaire des cellules de l'hippocampe. Cette réduction s'accompagne d'une restauration des capacités cognitives des animaux à des niveaux non statistiquement différents de ceux des animaux non injectés par $A\beta_{25-35}$. Un tel niveau de neuroprotection n'est pas observé pour l'antagoniste de référence des récepteurs NMDA pour les pathologies neurodégénératives, la mémantine, dont la structure est pourtant voisine. En outre, à la différence de la mémantine, les demandeurs ont également observé que la FENM est dépourvue d'effets amnésiques. Enfin, la FENM permet le traitement symptomatique des troubles cognitifs induits par l'injection de $A\beta_{25-35}$, de manière plus efficace que la mémantine.

Ainsi un premier objet de la présente invention concerne le composé de formule (I) :

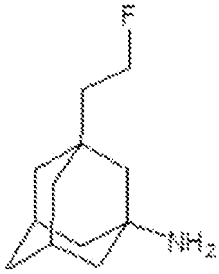

(I),

ou de l'un de ses sels pharmaceutiquement acceptable, pour son utilisation pour induire une neuroprotection chez un sujet en ayant besoin, tel que défini dans les revendications.

Dans un mode de réalisation particulier, ledit sujet souffre, est suspecté de souffrir ou est considéré à risque de souffrir d'une pathologie du système nerveux central. Dans un mode de réalisation également particulier, cette pathologie du système nerveux central est en lien avec la mort cellulaire neuronale et/ou la neurodégénérescence. Dans un mode de réalisation également particulier, cette pathologie du système nerveux central est en lien avec l'excitotoxicité. Dans un mode de réalisation encore plus particulier, le dit sujet souffre, est suspecté de souffrir ou est considéré à risque de souffrir d'une pathologie du système nerveux central sélectionnée parmi les tauopathies, d'une synucléopathies ou les amyloïdopathies telles que la maladie d'Alzheimer, la maladie de Parkinson, l'atrophie multisystématisée, la démence à corps de Lewy, la dégénérescence cortico-basale, la maladie de Pick, la démence fronto-temporale, l'atrophie corticale postérieure, ou de pathologies telles que la maladie de Huntington, la sclérose latérale amyotrophique, l'épilepsie, la démence vasculaire, le syndrome de Korsakoff, le sevrage alcoolique, l'ischémie, l'ischémie néonatale, les traumatismes crâniens, l'accident vasculaire cérébral.

Dans un autre mode particulier de réalisation, ledit sujet peut ainsi avoir été diagnostiqué comme atteint d'une pathologie du système nerveux central. Particulièrement, cette pathologie est en lien avec la mort cellulaire neuronale et/ou la neurodégénérescence. Plus particulièrement, ledit sujet peut avoir été diagnostiqué comme souffrant d'une tauopathie, d'une synucléopathie ou d'une amyloïdopathie telles que la maladie d'Alzheimer, la maladie de Parkinson, l'atrophie multisystématisée, la démence à corps de Lewy, la dégénérescence cortico-basale, la maladie de Pick, la démence fronto-temporale, l'atrophie corticale postérieure, ou de pathologies telles que la maladie de Huntington, la sclérose latérale amyotrophique, l'épilepsie, la démence vasculaire, le syndrome de Korsakoff, le sevrage alcoolique, l'ischémie, l'ischémie néonatale, les traumatismes crâniens, l'accident vasculaire cérébral. Ce diagnostic se base sur des analyses comportementales, cognitives, biologiques et/ou d'imagerie médicales bien connue de l'homme de l'art. Dans ce cas, la neuroprotection conférée par composé de formule (I) permettra de ralentir voire de stopper la progression de la maladie c'est-à-dire de stopper ou de ralentir la mort cellulaire neuronale et/ou la neurodégénérescence déjà initiée dans le contexte de la maladie ainsi diagnostiquée. En conséquence, ladite neuroprotection ainsi conférée chez ledit sujet se traduira par l'arrêt, ou le ralentissement de l'évolution des atteintes cognitives résultant de la neurodégénérescence et des symptômes associés.

Dans un autre mode particulier de réalisation, ledit sujet peut également être suspecté de souffrir d'une pathologie en lien avec la mort cellulaire neuronale et/ou la neurodégénérescence. En d'autres termes, le diagnostic établi pour ce sujet est incertain, c'est-à-dire, par exemple, que le sujet ne présente pas un niveau de symptômes (dans leur intensité), ou la totalité des différents symptômes ou signes du tableau clinique qui permettent d'établir un diagnostic formel de la pathologie. Cependant, chez ce sujet, les symptômes ou signes relevés sont pertinents dans le cadre de la pathologie. Il peut s'agir par exemple de patients au début du développement de la maladie et qui présentent alors uniquement quelques signes précurseurs, potentiellement de faible intensité. Plus particulièrement, ledit sujet présente des symptômes ou signes pertinents dans le cadre d'une tauopathie, d'une synucléopathie ou d'une amyloïdopathie telles que la maladie d'Alzheimer, la maladie de Parkinson, l'atrophie multisystématisée, la démence à corps de Lewy, la dégénérescence cortico-basale, la maladie de Pick, la démence fronto-temporale, l'atrophie corticale postérieure, ou des symptômes ou signes pertinents dans le cadre de pathologies telles que la maladie de Huntington, la sclérose latérale amyotrophique, l'épilepsie, la démence vasculaire, le syndrome de Korsakoff, le sevrage alcoolique, l'ischémie, l'ischémie néonatale, les traumatismes crâniens, l'accident vasculaire cérébral.

Les symptômes ou signes pertinents peuvent être détectés par des analyses comportementales, cognitives, biologiques et/ou d'imagerie médicale bien connues de l'homme de l'art et couramment utilisées dans le diagnostic de ces pathologies.

Les tests couramment utilisés pour l'évaluation cognitive des sujets humains, sont par exemple, le Mini-Mental State Examination (MMSE ou test de Folstein), Modified Mini-Mental State Examination ( ou échelle 3MS), Abbreviated Mental Test Score (AMTS ou Test mental abrégé), Dementia questionnaire for persons with Mental Retardation (ou questionnaire DMR), Cognitive Abilities Screening Instrument (CASI ou Instrument de dépistage des capacités cognitives), Trail- making test, Clock drawing test, Alzheimer's disease assessment scale - Cognition (ADAS-Cog ou Sous échelle cognitive de l'échelle de l'évaluation de la maladie d'Alzheimer), General Practitioner Assessment of Cognition (GPCOG ou évaluation par un praticien de la cognition), Montreal Cognitive Assessment (MoCA ou évaluation cognitive de Montréal), ou Rowland Universal Dementia Assessment Scale (RUDAS ou échelle Rowland d'évaluation de la démence universelle), ou encore Alzheimer's Disease Cooperative Study - Activities of Daily Living (ADCS-ADL ou étude coopérative de la maladie d'Alzheimer-activité de la vie quotidienne) selon leur dénomination en langue anglaise.

Plus particulièrement, le MMSE permet de dépister les personnes souffrant d'une atteinte neurocognitive majeure (démence) sans la rattacher à une pathologie particulière. Le MMSE sert également à assurer les suivis de l'état cognitif des personnes et à mesurer le déclin des fonctions cognitives chez les personnes qui souffrent d'atteintes neurocognitives. Ce test évalue l'orientation, l'enregistrement, l'attention et le calcul, la rétention mnésique, le langage et praxie de construction. Le CERAD (dans sa dénomination en langue anglaise Consortium to Establish a Registry for

Alzheimer's Disease ou Consortium pour établir un registre de la maladie d'Alzheimer) a établi une échelle de sévérité de la démence associée aux scores obtenus dans le MMSE. Un score entre 19 et 24 est associé à une démence légère, entre 10 et 18, une démence modérée, et un score inférieur à 10 correspond à une démence sévère, le score maximal étant de 30.

L'ADAS-Cog est une sous-échelle cognitive de l'échelle d'évaluation de la maladie d'Alzheimer et ne concerne donc que les aspects de la démence liés à la cognition. Ainsi il peut être utilisé pour évaluer (*i.e.* scorer) et suivre l'évolution de tout type de démence. L'ADAS-Cog évalue l'orientation, la mémoire, la fonction exécutive, la capacité visuospatiale, le langage ou la pratique, avec une plage de scores de 0 à 70, un score plus élevé indiquant une plus grande déficience. L'ADAS-Cog est considéré comme plus sensible que le MMSE. C'est un des tests les plus communément utilisés pour l'évaluation clinique des composés candidats à l'obtention d'une Autorisation de Mise sur le Marché dans le cadre de traitements anti-démence et également dans la mesure de l'évolution des atteintes cognitives.

L'imagerie médicale peut permettre d'identifier une atteinte structurale ou fonctionnelle de zones particulières du cerveau, et également ainsi aider au diagnostic des certaines de ces pathologies neurodégénératives. Par exemple, la scintigraphie cérébrale à l'ioflupane permet de caractériser l'atteinte des neurones dopaminergiques dans le cas de la maladie de Parkinson ou de la démence à corps de Lewy. La FENM marquée au $^{18}F$ est-elle même envisagée comme marqueur des récepteurs NMDA par tomographie par émission de positrons (TEP) et fait l'objet d'une étude pilote chez l'homme (Beaurain *et al.*, 2019). L'IRM ou la TEP peut permettre de diagnostiquer par exemple, la démence fronto-temporale (par l'identification d'une atrophie des lobes frontaux et temporaux) ou la maladie d'Alzheimer (atrophie corticale et/ou atrophie des hippocampes). Ainsi, dans un mode de réalisation particulier, le sujet ayant besoin de neuroprotection présente une atteinte des neurones dopaminergique, une atrophie des fronto et/ou temporaux, ou atrophie corticale et/ou atrophie d'une ou des deux hippocampes.

L'analyse de la présence de certaines protéines dans le LCR peut permettre d'établir le diagnostic, par exemple, de la maladie d'Alzheimer, dont le profil typique associe une diminution de la concentration du peptide $A\beta_{42}$ et une augmentation des protéines Tau et de leurs formes phosphorylées P-Tau. Dans certains cas, le dosage de l'$A\beta_{1-40}$ et la mesure du ratio $A\beta_{1-42}/A\beta_{1-40}$ permettent d'améliorer le diagnostic. Les marqueurs d'imagerie et du LCR permettent un diagnostic précoce des maladies, dans certains cas avant l'apparition des symptôme cognitifs. Ainsi, dans un mode de réalisation particulier, le sujet ayant besoin de neuroprotection présente un profil anormal des niveaux des peptides $A\beta_{1-42}$ et/ou $A\beta_{1-40}$ et/ou de la protéine Tau et/ou de ses formes phosphorylées et /ou du ratio $A\beta_{1-42}/A\beta_{1-40}$.

Également, l'étude des enregistrements de potentiels évoqués (ERP pour *Event-Related Potential* en langue anglaise) est d'une grande application dans l'évaluation des processus cognitifs car les résultats sont indépendants du stimulus utilisé. Les ERP sont observés en réponse à un stimulus discordant et représentent les phénomènes cognitifs activés tels que la perception, l'attention, le processus de prise de décision, le processus de mémoire, le langage, etc. Les ERP sont enregistrés, par exemple, par l'électroencéphalographie (EEG) ou magnétoencéphalographie (MEG). Les ERP fournissent des informations sur le traitement du stimulus par le cerveau même quand aucun changement de comportement n'est perceptible. Les caractéristiques de l'ERP peuvent varier en fonction de divers facteurs tels que la pertinence du stimulus, la tâche effectuée, les lésions du système nerveux ou la prise de médicaments.

Les ERP sont connus dans l'état de l'art comme des biomarqueurs cognitifs utiles pour le diagnostic de la démence, le suivi de la progression de la maladie et l'évaluation de l'effet pro-cognitif de traitements. Les ERP sont par exemple altérés chez les patients souffrant de la maladie d'Alzheimer, de démence vasculaire ou de démence associée à des symptômes parkinsoniens, comme par exemple la démence à corps de Lewy. Plus particulièrement, les mesures des ERP permettent détecter une altération de la fonction cognitive à un stade précoce et notamment dans le cas du stade précoce ou léger de la maladie d'Alzheimer. L'ERP le plus fréquemment examiné en pratique clinique est l'onde P300 (ou P3) qui est une grande positivité centropariétale qui se produit avec une latence d'environ 300 ms après le stimulus discordant. L'onde P300 peut être divisée en deux sous-composantes P3a et P3b. P3a est généralement considérée comme liée au degré d'attention focale, tandis que P3b est censée indexer la mise à jour de la mémoire de travail. L'amplitude de l'onde P300 fait notamment référence à la motivation (en lien avec la difficulté de la tâche) et à la vigilance en fonction de la probabilité d'occurrence du stimulus. La latence se réfère au temps nécessaire à la prise de décision. Une augmentation de la latence de P300 (ou de ses sous composantes) et une diminution de son amplitude (ou de ses sous composantes) sont observées chez les patients atteints de démence, notamment de la maladie d'Alzheimer. L'étude de l'évolution de la latence est utile pour le suivi de la progression de la démence, notamment de la maladie d'Alzheimer et pour évaluer la réponse au traitement de la maladie d'Alzheimer.

Ainsi, dans un mode de réalisation particulier, le sujet ayant besoin de neuroprotection présente une altération de ses capacités cognitives. De manière préférée, cette altération est mesurée par le MMSE ou l'ADAS-Cog. De manière particulièrement préférée, ledit sujet présente un score MMSE inférieur à 30, inférieur ou égale à 29, 28, 27, 26, 25, 24, 23, 22, 21, 20, 19, 18, 17, 16, 15, 14, 13, 12, 11, 10, 9, 8, 7, 6, 5, 4, 3, ou encore inférieur ou égal à 2. De manière également préférée, cette altération est mesurée chez ledit sujet par l'analyse de l'onde P300 des ERP du sujet, le sujet présentant une diminution de l'amplitude et/ou de la latence de l'onde P300 ou d'une de ses composantes.

Dans un autre mode de réalisation particulier, ledit sujet ayant besoin de neuroprotection souffre d'une démence

légère. Dans un autre mode de réalisation particulier, ledit sujet ayant besoin de neuroprotection souffre d'une démence modérée. Dans un autre mode de réalisation particulier, ledit sujet ayant besoin de neuroprotection souffre d'une démence sévère. De manière particulièrement préférée, le sujet ayant besoin de neuroprotection souffre d'une démence légère. Ainsi dans un mode de réalisation particulier, l'invention concerne le composé de formule (I) ou de l'un de ses sels pharmaceutiquement acceptable, pour son utilisation pour induire une neuroprotection chez un sujet souffrant de démence légère.

De manière particulièrement préférée, le sujet ayant besoin de neuroprotection est diagnostiqué comme souffrant d'une pathologie du système nerveux central en lien avec la mort cellulaire neuronale et/ou la neurodégénérescence telle que définie ci-dessus, mais ne présente pas de symptômes cognitifs ou bien des symptômes n'ont pas été détectés chez ce sujet. Ainsi, du fait de propriétés neuroprotectrices décrites dans la partie expérimentale, il sera possible de prévenir, de retarder ou de ralentir la survenue de ces symptômes et par conséquent de maintenir la qualité de vie des patients et d'éviter la survenue d'autres pathologies associées avec les pathologies neurodégénératives, notamment les pathologies psychiatriques telle que la dépression.

Enfin, ledit sujet ayant besoin de neuroprotection peut également être considéré comme un sujet à risque de souffrir d'une pathologie du système nerveux central, particulièrement une pathologie en lien avec la mort cellulaire neuronale et/ou la neurodégénérescence telle que décrite ci-dessus. En d'autres termes, ledit sujet ne présente pas de symptôme ou de signes associés avec ladite pathologie, mais présente un risque accru de développer cette pathologie par rapport à la population générale, car il présente des facteurs de risques liés à son mode de vie, des prédispositions génétiques ou à son histoire familiale, à la présence de comorbidités et/ou à son âge. Plus particulièrement, ledit sujet a un mode de vie, présente des prédispositions génétiques ou a une histoire familiale, souffre d'une comorbidités et/ou est d'un âge qui augmente(nt) son risque (par rapport à la population générale) de développer une tauopathie, une synucléopathie ou une amyloïdopathie telles que la maladie d'Alzheimer, la maladie de Parkinson, l'atrophie multisystématisée, la démence à corps de Lewy, la dégénérescence cortico-basale, la maladie de Pick, la démence frontotemporale, l'atrophie corticale postérieure, ou de développer des pathologies telles que la maladie de Huntington, la sclérose latérale amyotrophique, l'épilepsie, la démence vasculaire, le syndrome de Korsakoff, le sevrage alcoolique, l'ischémie, l'ischémie néonatale, les traumatismes crâniens, l'accident vasculaire cérébral.

Par exemple, et sans être limitatif, l'âge est le premier facteur de risque des démences, dont la maladie d'Alzheimer fait partie, puisque 5 à 8% des personnes âgées de plus de 60 ans en sont atteintes. Concernant les prédispositions génétiques aux maladies du SNC, pathologie en lien avec la mort cellulaire neuronale et/ou la neurodégénérescence, on peut citer, par exemple, la présence de l'allèle ε4 de l'apolipoprotéine E (ApoE) qui est associé avec un risque accru de survenue de plusieurs démences et notamment à la maladie d'Alzheimer (Liu *et al.* 2013) ; également, par exemple, des mutations dans les gènes SNCA (alphasynucléine), PRKN (parkine), LRRK2 (leucine-rich repeat kinase 2), PINK1 (PTEN-induced putative kinase 1), DJ-1 and ATP13A2 et dans les 11 loci des gènes PARK1-PARK11 sont associées à une augmentation du risque de la maladie de Parkinson pour le sujet porteur de telles prédispositions génétiques ou mutations ; la mutation du gène SOD1 et son lien avec la sclérose latérale amyotrophique ou encore les mutations du gène codant la huntingtine sont également des exemples de prédisposition génétique à des maladies du SNC. Parmi les comorbidités associées avec un risque accru de développer ces pathologies, on peut citer des maladies comme, par exemple, la trisomie 21, le trouble de stress post-traumatique (PTSD), la dépression, l'hypertension artérielle, le diabète, les traumatismes crâniens ou les accidents vasculaires cérébraux. La consommation excessive d'alcool est associée à une augmentation du risque de démence. L'exposition à certains métaux tels que le manganèse, le cuivre, le plomb ou certains composés chimiques tels que le paraquat, la roténone ou le manèbe est associée à une augmentation du risque de développer la maladie de Parkinson.

Ainsi, dans un mode de réalisation le sujet ayant besoin de neuroprotection est un sujet présentant un facteur de risque pour une pathologie du système nerveux central, plus particulièrement une pathologie en lien avec la mort cellulaire neuronale et/ou la neurodégénérescence. Plus particulièrement le sujet ne présente pas d'autres symptômes de la pathologie. De manière préférée, le sujet ne présente pas de symptôme cognitif de la pathologie. Dans un mode de réalisation préféré, le sujet présente une prédisposition génétique pour ladite pathologie. Dans un autre mode de réalisation préféré, le sujet présente une pathologie associée avec un risque accru de développer une pathologie du système nerveux central, plus particulièrement une pathologie en lien avec la mort cellulaire neuronale et/ou la neurodégénérescence. L'administration des composés de formule (I) chez ces sujets définis comme à risque ayant besoin de neuroprotection permet de prévenir ou retarder la survenue de ces pathologies et/ou de leurs symptômes, notamment une pathologie du système nerveux central, plus particulièrement une pathologie en lien avec la mort cellulaire neuronale et/ou la neurodégénérescence.

Dans un aspect non-revendiqué, la présente divulgation concerne le composé de formule (I) ou l'un de ses sels pharmaceutiquement acceptable pour son utilisation pour induire une neuroprotection chez un sujet souffrant de PTSD. Un mode de réalisation encore plus particulier de la présente divulgation concerne le composé de formule (I) ou l'un de ses sels pharmaceutiquement acceptable pour induire une neuroprotection chez un sujet souffrant de PTSD, pour par exemple, prévenir la survenue de la maladie d'Alzheimer ou de ses symptômes.

Les données expérimentales présentées montrent que le composé de formule (I) est particulièrement efficace pour prévenir la neuroinflammation, le stress oxydant et l'apoptose des cellules neuronales induits par la toxicité des oligomères Aβ et qui sont à la source de la neurodégénérescence et de la mort des cellules du SNC plus particulièrement des neurones dans de nombreuses pathologies, comme, mais pas uniquement, la maladie d'Alzheimer.

Ainsi, un objet de la présente invention concerne également le composé de formule (I) ou l'un de ses sels pharmaceutiquement acceptable pour son utilisation pour prévenir ou diminuer la toxicité des oligomères Aβ, et plus particulièrement pour prévenir ou diminuer la neurotoxicité des oligomères Aβ. Un mode de réalisation particulier de l'invention concerne également le composé de formule (I) ou l'un de ses sels pharmaceutiquement acceptable pour son utilisation pour prévenir ou diminuer la toxicité des oligomères Aβ, et plus particulièrement pour prévenir ou diminuer la neurotoxicité des oligomères Aβ, chez un sujet souffrant, suspecté de souffrir ou à risque de souffrir de la maladie d'Alzheimer ou d'une pathologie dans laquelle les agrégats ou les oligomères Aβ sont impliqués, telle que la démence à corps de Lewy.

Comme évoqué, la neuroinflammation, le stress oxydant et l'apoptose des cellules neuronales sont observés dans de nombreuses pathologies neurodégénératives.

Un autre objet de la présente invention concerne également le composé de formule (I) ou l'un de ses sels pharmaceutiquement acceptable pour son utilisation telle que définie dans les revendications indépendantes pour prévenir ou diminuer la neuroinflammation chez un sujet en ayant besoin. Un objet de la présente invention concerne également le composé de formule (I) ou de l'un de ses sels pharmaceutiquement acceptable pour son utilisation telle que définie dans les revendications indépendantes pour prévenir ou diminuer le stress oxydant chez un sujet en ayant besoin. Un objet de la présente invention est également le composé de formule (I) ou de l'un de ses sels pharmaceutiquement acceptable pour son utilisation telle que définie dans les revendications indépendantes pour prévenir ou diminuer l'apoptose dans le SNC chez un sujet en ayant besoin, comprenant l'administration audit sujet d'un composé de formule (I) ou de l'un de ses sels pharmaceutiquement acceptable. Un objet de la présente invention est également le composé de formule (I) ou de l'un de ses sels pharmaceutiquement acceptable pour son utilisation telle que définie dans les revendications indépendantes pour prévenir ou diminuer la perte cellulaire de l'hippocampe chez ledit sujet. Cette perte peut être détectée, comme mentionné précédemment, notamment, par l'observation d'une atrophie, d'une diminution du volume, d'une modification de la forme de l'hippocampe ou d'une atrophie ou d'une diminution du cortex cérébral, par exemple en Imagerie à Résonnance Magnétique (IRM). Dans un mode de réalisation particulier, les composés et combinaisons pour leur utilisation selon l'invention comprennent le suivi structurel et ou fonctionnel du SNC par imagerie médicale.

Dans un mode de réalisation particulier de chacun de ces objets, ledit sujet souffre, est suspecté de souffrir ou est considéré à risque de souffrir d'une pathologie du système nerveux central sélectionnée parmi les tauopathies, synucléinopathies, les amyloïdopathies, la maladie d'Alzheimer, la maladie de Parkinson, l'atrophie multisystématisée, la maladie de Huntington, l'atrophie corticale postérieure, la maladie de Pick l'épilepsie, la démence vasculaire, la démence fronto-temporale, la démence à corps de Lewy, la sclérose latérale amyotrophique, le syndrome de Korsakoff, le sevrage alcoolique, l'ischémie, l'ischémie néonatale, les traumatismes crânien, l'accident vasculaire cérébral, de préférence la maladie d'Alzheimer.

En lien avec la prévention de mécanismes cellulaires et biochimiques sous-jacents à la neurodégénérescence et la mort des cellules du SNC, plus particulièrement des cellules neuronales, il est observé que le composé de formule (I) est particulièrement efficace dans la prévention et le traitement symptomatique des altérations des capacités cognitives dans un modèle animal de neurodégénération et de démence. Les données expérimentales montrent que le composé de formule (I) est efficace, dans des conditions pathologiques, dans le maintien et la protection de nombreux et différents types de mémoire et de processus cognitifs qui impliquent des mécanismes neuronaux et des aires variées du cerveau.

Ainsi un objet de l'invention est également le composé de formule (I) ou de l'un de ses sels pharmaceutiquement acceptable pour son utilisation telle que définie dans les revendications indépendantes pour prévenir ou diminuer des altérations des capacités cognitives chez un sujet en ayant besoin. Les « capacités cognitives », au sens de l'invention, concernent des fonctions intellectuelles telles que la mémoire, la perception, la coordination et le raisonnement. L'ADAS-Cog est notamment un test composite qui permet de tester ces différents aspects de la cognition chez les sujets. Un autre objet particulier de l'invention concerne le composé de formule (I) ou de l'un de ses sels pharmaceutiquement acceptable pour son utilisation telle que définie dans les revendications indépendantes pour prévenir ou diminuer des altérations de la mémoire à court, moyen et/ou long terme chez un sujet en ayant besoin. Un autre objet particulier de l'invention concerne le composé de formule (I) ou de l'un de ses sels pharmaceutiquement acceptable pour son utilisation telle que définie dans les revendications indépendantes pour prévenir ou diminuer des altérations de la mémoire spatiale chez un sujet en ayant besoin comprenant l'administration audit sujet d'un composé de formule (I) ou de l'un de ses sels pharmaceutiquement acceptable. Un autre objet particulier de l'invention concerne le composé de formule (I) ou de l'un de ses sels pharmaceutiquement acceptable pour son utilisation telle que définie dans les revendications indépendantes pour prévenir ou diminuer des altérations des capacités de reconnaissance et/ou d'apprentissage chez un sujet en ayant besoin. Dans un mode de réalisation particulier de chacun de ces objets, ledit sujet souffre, est suspecté

de souffrir ou est considéré à risque de souffrir d'une pathologie du système nerveux central sélectionnée parmi les tauopathies, synucléinopathies, les amyloïdopathies, la maladie d'Alzheimer, la maladie de Parkinson, l'atrophie multisystématisée, la maladie de Huntington, l'atrophie corticale postérieure, la maladie de Pick l'épilepsie, la démence vasculaire, la démence fronto-temporale, la démence à corps de Lewy, la sclérose latérale amyotrophique, le syndrome de Korsakoff, le sevrage alcoolique, l'ischémie, l'ischémie néonatale, les traumatismes crânien, l'accident vasculaire cérébral, de préférence la maladie d'Alzheimer.

Le MMSE et/ou l'ADAS-Cog peuvent permettre de suivre aisément l'évolution des symptômes et ainsi de contrôler la neuroprotection conférée par le composé de formule (I) ou l'un de ses sels pharmaceutiquement acceptable ou combinaisons pour leur utilisation selon l'invention. Une baisse du score de MMSE ou une augmentation de l'ADAS-cog (ou de l'un de ses items) est indicative d'une dégradation des capacités cognitives de l'individu testé. Sans que cela ne soit limitatif, il a été estimé que la dégradation moyenne des performances cognitives aux stades légers et modérés de la maladie par la perte de 2 à 4 points par an au MMSE et une augmentation de 6 à 8 points par an à l'ADAS-Cog (Institut de la maladie d'Alzheimer ; <http://www.imaalzheimer.com>. ADAS-Cog). Une augmentation de l'ADS-cog lors de deux tests consécutifs signera une aggravation de la maladie et/ou des symptômes. Une diminution du MMSE lors de deux tests consécutifs signera une aggravation de la maladie et/ou des symptômes. Les tests peuvent être faits toutes les 1, 2, 3, 4 semaines, tous les mois, tous les 2, 3, 4, 5, 6, 7, 8, 9, 10, 11, 12 mois ou 3 à 4 fois par an ou même une fois par an. Par exemple, une augmentation inférieure ou égale à 20%, 15%, de préférence inférieure ou égale à 10%, voire de préférence inférieure ou égale à 5% de l'Adas-Cog pour deux tests réalisés à un mois d'intervalle sera signe de neuroprotection. Par exemple, une diminution inférieure ou égale à 20%, 15%, de préférence inférieure ou égale à 10%, voire de préférence inférieure ou égale à 5% du MMSE pour deux tests réalisés à un mois d'intervalle sera signe de neuroprotection. Alternativement, un ralentissement de l'aggravation des symptômes cognitifs par rapport à l'évolution usuelle de la pathologie sera signe d'une neuroprotection. Également, un ralentissement de la dégradation du score correspondant à une ou plusieurs catégories de mémoire ou des performances cognitives tels que mesurées dans les scores composites mais pas forcément du score global du test, sera signe de neuroprotection. Dans un mode de réalisation particulier, les composés et combinaisons pour leur utilisation selon l'invention comprennent le suivi de l'état cognitif du sujet au cours du traitement de préférence par l'application d'un test MMSE ou ADAS-Cog.

Comme mentionné précédemment, l'étude des ERP permet de suivre l'évolution des symptômes de la neurodégénérescence et ainsi de contrôler la neuroprotection conférée par le composé de formule (I) ou l'un de ses sels pharmaceutiquement acceptable ou combinaisons pour leur utilisation selon l'invention. Une augmentation de la latence dans la survenue de l'onde P300 et/ou une diminution de son amplitude est indicative d'une dégradation des capacités cognitives de l'individu testé. Ainsi, une diminution de la latence de l'onde P300 et/ou une augmentation de son amplitude sera signe de neuroprotection. Dans un mode de réalisation particulier, les composés et combinaisons pour leur utilisation selon l'invention comprennent le suivi de l'état cognitif du sujet au cours du traitement de préférence par l'étude des ERP par EGG ou MEG.

Un autre objet de l'invention concerne le composé de formule (I) ou de l'un de ses sels pharmaceutiquement acceptable pour son utilisation pour prévenir ou traiter une pathologie du système nerveux central sélectionnée parmi les tauopathies, les synucléopathies ou les amyloïdopathies telles que la maladie d'Alzheimer, la maladie de Parkinson, l'atrophie multisystématisée, la démence à corps de Lewy, la dégénérescence cortico-basale, la maladie de Pick, la démence fronto-temporale, l'atrophie corticale postérieure, ou de pathologies telles que la maladie de Huntington, la sclérose latérale amyotrophique, l'épilepsie, la démence vasculaire, le syndrome de Korsakoff, le sevrage alcoolique, l'ischémie, l'ischémie néonatale, les traumatismes crâniens, l'accident vasculaire cérébral, de préférence la maladie d'Alzheimer, chez un sujet en ayant besoin. Un objet particulier de l'invention concerne le composé de formule (I) ou de l'un de ses sels pharmaceutiquement acceptable pour son utilisation pour prévenir ou traiter la maladie d'Alzheimer, chez un sujet en ayant besoin. Ledit sujet peut souffrir de la maladie d'Alzheimer à son stade léger (dit également précoce), au stade modéré ou au stade avancé. Dans un mode de réalisation préféré, ledit sujet soufre de la maladie d'Alzheimer à son stade léger.

Un autre objet de l'invention concerne le composé de formule (I) ou de l'un de ses sels pharmaceutiquement acceptable pour son utilisation pour prévenir ou traiter la démence, chez un sujet en ayant besoin. Dans un mode de réalisation particulier, le sujet souffre de démence (*e.g.* de déclin cognitif) légère.

Un autre objet de l'invention concerne le composé de formule (I) ou de l'un de ses sels pharmaceutiquement acceptable pour son utilisation pour prévenir ou traiter l'excitotoxicité chez un sujet souffrant, étant suspecté de souffrir ou étant considéré à risque de souffrir d'une pathologie du système nerveux central sélectionnée parmi les tauopathies, les synucléopathies ou les amyloïdopathies telles que la maladie d'Alzheimer, la maladie de Parkinson, l'atrophie multisystématisée, la démence à corps de Lewy, la dégénérescence cortico-basale, la maladie de Pick, la démence fronto-temporale, l'atrophie corticale postérieure, ou de pathologies telles que la maladie de Huntington, la sclérose latérale amyotrophique, l'épilepsie, la démence vasculaire, le syndrome de Korsakoff, le sevrage alcoolique, l'ischémie, l'ischémie néonatale, les traumatismes crâniens, l'accident vasculaire cérébral, de préférence la maladie d'Alzheimer. Un objet particulier de l'invention concerne le composé de formule (I) ou de l'un de ses sels pharmaceutiquement acceptable

pour son utilisation pour prévenir ou traiter l'excitotoxicité chez un sujet souffrant la maladie d'Alzheimer, chez un sujet en ayant besoin.

Dans un mode de réalisation particulier, un sel pharmaceutiquement acceptable du composé de formule (i) est sélectionné parmi les sels de formule (II) :

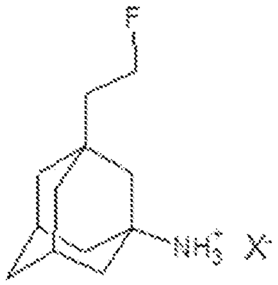

(II)

dans laquelle, $X^-$ désigne un contre anion choisi dans le groupe constitué par les ions chlorure, bromure, iodure, acétate, méthane sulfonate, benzène sulfonate, camphosulphonate, tartrate, dibenzoate, ascorbate, fumarate, citrate, phosphate, salicylate, oxalate, bromohydrate et tosylate. De manière préférée, le contre anion est l'ion chlorure.

Sans vouloir être lié par une quelconque théorie scientifique, on peut penser que cette action de la FENM est liée à son activité antagoniste des récepteurs NMDA et donc de modulation de l'excitotoxicité. Bien que structurellement proche de la mémantine, ce composé s'en différencie par des propriétés inattendues puisqu'il induit une neuroprotection significative que la mémantine ne permet pas. En outre la FENM, ne présente pas les effets délétères pour la mémoire de la mémantine quand la FENM est administrée à des doses importantes ce qui est un avantage particulier, notamment dans le cas des pathologies neurodégénératives qui résultent en une démence et/ou des troubles cognitifs chez les sujets qui en sont atteints. Il est donc possible d'administrer des doses importantes de ce composé au sujet, cela pour atteindre des doses efficaces au niveau du système nerveux central, ce qui n'était pas possibles pour la mémantine, pour laquelle la dose maximum autorisée (20 mg par jour pour l'Ebixa®) ne permet pas d'atteindre l'$IC_{50}$ pour les récepteurs NMDA dans le liquide céphalorachidien (LCR). Enfin la FENM semble présenter une courbe dose effet totalement différente, inattendue et bien plus favorable que celle de la mémantine. La FENM est active chez l'animal pour un intervalle de dose significativement plus large que celui de la mémantine. Ces propriétés inattendues permettent d'envisager des intervalles de doses utiles bien plus importants que ceux de la mémantine et ainsi faciliter la galénique et obtenir un traitement plus efficace que la mémantine.

Dans un mode de réalisation particulier, le composé de formule (I), ou l'un de ses sels pharmaceutiquement acceptable ou du composé de formule (II) pour son utilisation chez un sujet en ayant besoin, est dépourvu, à la dose administrée, d'effet cognitif indésirable, en particulier d'effet amnésique pour le patient.

De préférence, le composé de formule (I), ou l'un de ses sels pharmaceutiquement acceptable ou du composé de formule (II) est administré au sujet sous la forme de préparation pharmaceutique, par exemple, sans être limitatif, de manière orale ou de manière parentérale (sous-cutanée ou par intraveineuse). L'administration orale étant particulièrement préférée.

Dans un mode de réalisation, le composé de formule (I), ou l'un de ses sels pharmaceutiquement acceptable ou le composé de formule (II) est administré au sujet à une dose orale comprise entre 1 et 1000 mg par jour bornes comprises, de préférence entre 5 et 500 mg par jour, de manière préférée entre 10 et 100 mg par jour, de manière particulièrement préférée entre 20 et 70 mg par jour, de manière encore plus préférée entre 30 et 60 mg par jour.

Dans un autre mode de réalisation, le composé de formule (I), ou l'un de ses sels pharmaceutiquement acceptable, ou le composé de formule (II), est administré à une dose orale supérieure à 20 mg par jour, de manière préférée supérieure ou égale à 30 mg par jour, à 40 mg, à 50 mg, à 60 mg, à 70 mg, à 80 mg, à 90 mg, voire supérieure ou égale à 100 mg par jour.

S'agissant de conférer la neuroprotection dans le cas de maladies chroniques telles que des pathologies du système nerveux central sélectionnée parmi les tauopathies, les synucléopathies ou les amyloïdopathies telles que la maladie d'Alzheimer, la maladie de Parkinson, l'atrophie multisystématisée, la démence à corps de Lewy, la dégénérescence cortico-basale, la maladie de Pick, la démence fronto-temporale, l'atrophie corticale postérieure, ou des pathologies telles que la maladie de Huntington, la sclérose latérale amyotrophique, l'épilepsie, la démence vasculaire, le syndrome de Korsakoff, on comprendra aisément que le traitement du sujet avec le composé de formule (I) ou l'un de ses sels pharmaceutiquement acceptable ou le composé de formule (II), dure toute la vie de celui-ci du moins tant que le traitement fait effet. Pour les pathologies aiguës telles que le sevrage alcoolique, l'ischémie, l'ischémie néonatale, les traumatismes crâniens, l'accident vasculaire cérébral, un traitement limité dans le temps peut être envisagé. Il peut durer de 1, 2, 3, 4, 5, 6, 7 jours, ou de 1, 2, 3, 4, semaines, ou de 2, 3, 4, 5, 6, 7, 8, 9, 10, 11, 12 mois, ou de 1, 2, 3, 4,

5, 6 années ou même plus, c'est-à-dire le temps de la durée du besoin en neuroprotection, notamment contre la neurotoxicité. Pour ces pathologies aiguës, il est désirable que le traitement comprenant le composé de formule (I) ou (II) soit mis en oeuvre le plus tôt possible, pour prévenir au mieux neurodégénérescence, par exemple, le jour de leur survenue, le lendemain, ou encore le surlendemain.

Lesdites doses peuvent être unitaires, c'est-à-dire administrées en une seule fois au sujet. La dose peut également être administrée en plusieurs prises réparties dans la journée, le nombre de prises dans la journée permettant d'obtenir la dose journalière désirée. Ainsi dans un mode de réalisation particulier, les doses en question peuvent être administrées en une à quatre prises journalières, par exemple 1 fois, par exemple 2 fois, par exemple 3 fois, voire 4 fois.

Dans un mode particulier de réalisation, le composé de formule (I) ou l'un de ses sels pharmaceutiquement acceptable ou le composé de formule (II) est conditionné de manière à fournir la dose correspondant à une prise sans nécessiter de manipulation telle qu'une mesure de volume, une pesée ou la division d'une tablette, ce qui est particulièrement avantageux chez des sujets présentant des atteintes cognitives puisque cela évite tout calcul ou manipulation particulière.

Dans un mode de réalisation, le composé de formule (I) ou l'un de ses sels pharmaceutiquement acceptable ou le composé de formule (II) se présente sous forme de tablette ou comprimé. Dans un mode particulier ladite tablette ou comprimé est sécable en 1, 2, 3, voire 4 morceaux de manière à pouvoir fournir au sujet la dose nécessaire à une prise en utilisant 1, 2 ou 3 morceaux de ladite tablette. Cela est particulièrement intéressant, par exemple, dans le cas où le traitement nécessite une période d'augmentation de dose, pour arriver à la dose journalière ciblée, les morceaux pouvant correspondre aux paliers d'augmentation, et la tablette ou comprimé entier à la dose cible du traitement

Dans un mode de réalisation particulier, le traitement comprenant l'administration du composé de formule (I) ou l'un de ses sels pharmaceutiquement acceptable ou le composé de formule (II) comprend une période d'augmentation de dose, pour permettre au sujet de s'habituer au traitement. Cette période se tient au début du traitement ou lors d'une reprise du traitement lorsque celui-ci a été interrompu. Au cours de cette période les doses journalières sont augmentées régulièrement jusqu'à atteindre la dose maximale supportée par le patient ou prescrite par le praticien. Par exemple, les paliers d'augmentation de doses peuvent être de 2, 3, 4, 5, 6, 7 jours ou même plus, de préférence les paliers d'augmentation sont de 7 jours, et les doses peuvent être augmentées de 5 mg par palier, 10 mg ou même plus, de préférence 5 mg. Ainsi dans un mode de réalisation particulier, les doses sont augmentées de 5 mg de semaine en semaine jusqu'à atteindre la dose maximale supportée par le sujet ou la dose prescrite par le praticien. Dans un autre mode de réalisation, les doses sont augmentées de 10 mg de semaine en semaine jusqu'à atteindre la dose maximale supportée par le sujet ou la dose prescrite par le praticien.

Dans un autre mode de réalisation, le composé de formule (I) ou l'un de ses sels pharmaceutiquement acceptable ou le composé de formule (II) est formulé sous forme liquide. Il peut alors être conditionné sous forme de dose unitaire dans des contenants tels que des ampoules, ou alors dans un contenant tel qu'une bouteille ou un flacon associé à un dispositif permettant le prélèvement et, optionnellement, l'administration du volume désiré pour obtenir la dose adéquate.

Bien que la FENM soit efficace utilisée seule pour induire une neuroprotection chez un sujet en ayant besoin, il peut être intéressant de la combiner avec au moins un autre composé connu pour être bénéfique également dans la neuroprotection ou le traitement symptomatique des pathologie neurodégénératives. En effet, ces maladies sont des maladies complexes impliquant de nombreuses voies biochimiques et mécanismes cellulaires différents, qu'il est pertinent de cibler, dans certaines instances, par une action pléiotrope *via* des combinaisons pour obtenir une plus grande efficacité thérapeutique et/ou permettre la diminution des dosages et/ou la diminution d'effets indésirables.

Les inhibiteurs de l'acétylcholine estérase sont des molécules autorisées depuis plus de 20 années dans le traitement symptomatique de la maladie d'Alzheimer, de démences associées à la maladie de Parkinson ou d'autres démences neurodégénératives. En ralentissant/inhibant la dégradation de l'acétylcholine libérée par les neurones cholinergiques encore intacts, ces inhibiteurs sont supposés faciliter la neurotransmission cholinergique et donc avoir un effet favorable sur les déficits cognitifs dépendants de ces voies cholinergiques au cours de la maladie d'Alzheimer, ou d'une démence associée à la maladie de Parkinson. Les inhibiteurs de l'acétylcholine estérase utilisés sont le donépézil, la rivastigmine, la galanthamine. La tacrine, du fait notamment de son hépatotoxicité, est moins désirable.

Un objet de la présente invention est donc une combinaison comprenant un composé de formule (I) ou l'un de ses sels pharmaceutiquement acceptable ou le composé de formule (II) et au moins un inhibiteur de l'acétylcholinestérase, de préférence sélectionné parmi le donépézil, la rivastigmine, la galantamine ou un de leurs sels pharmaceutiquement acceptables, pour son utilisation pour induire une neuroprotection chez un sujet en ayant besoin, tel que défini dans les revendications.

Dans un mode de réalisation particulier ledit sujet souffre, est suspecté de ou à risque de souffrir de la maladie d'Alzheimer.

Les modulateurs des connexines, plus précisément la méfloquine, font l'objet d'un essai clinique dans le traitement symptomatique de la maladie d'Alzheimer. L'objet de l'essai est un traitement combinatoire du donépézil avec la méfloquine. Il a été découvert que la méfloquine avait un effet modulateur sur les connexines et notamment la

connexine 43, impliquées dans les jonctions communicantes des astrocytes. Les astrocytes jouent un rôle de soutien des neurones dans le SNC mais aussi dans le transfert d'informations et dans l'activité neuronale *via* les synapses tripartites. Un effet synergique entre le donépézil et la méfloquine a été reporté, permettant un effet à faible dose et plus rapide du donépézil.

Un objet de la présente invention est donc une combinaison comprenant un composé de formule (I) ou l'un de ses sels pharmaceutiquement acceptable ou le composé de formule (II) et au moins un inhibiteur modulateur des connexines pour son utilisation pour induire une neuroprotection chez un sujet en ayant besoin, tel que défini dans les revendications.

Un objet particulier de la présente invention est une combinaison comprenant un composé de formule (I) ou l'un de ses sels pharmaceutiquement acceptable ou le composé de formule (II) et au moins un modulateur des connexines tel que ceux listés dans la demande WO 2013/064579 ou WO 2010/029131. pour son utilisation telle que définie dans les revendications. Dans un mode de réalisation préféré, ledit au moins un modulateur de connexine est sélectionné parmi l'acide méclofénamique, l'énoxolone, la méfloquine et le 2-amino éthoxy diphényl borate (APB), ou un de leurs sels pharmaceutiquement acceptables. De manière encore plus préférée, ledit modulateur de connexine est l'acide méclofénamique. Dans un mode de réalisation particulier ledit sujet souffre, est suspecté de ou à risque de souffrir de la maladie d'Alzheimer.

Un objet particulier de la présente invention est également une combinaison comprenant un composé de formule (I) ou l'un de ses sels pharmaceutiquement acceptable ou de formule (II), au moins un modulateur des connexines tel que ceux listés dans la demande WO 2013/064579 ou WO 2010/029131 et au moins un inhibiteur de l'acétylcholine estérase, pour son utilisation pour induire une neuroprotection chez un sujet en ayant besoin, tel que défini dans les revendications. Dans un mode de réalisation préféré, ledit au moins un modulateur de connexine est sélectionné parmi l'acide méclofénamique, l'énoxolone, la méfloquine et le 2-amino éthoxy diphényl borate (APB), ou un de leurs sels pharmaceutiquement acceptables. De manière encore plus préférée, ledit modulateur de connexine est l'acide méclofénamique. Dans un mode de réalisation préféré ledit au moins un inhibiteur de l'acétylcholine estérase est sélectionné parmi donépézil, la rivastigmine et la galantamine. Dans une mode de réalisation encore plus préféré ledit au moins un inhibiteur de l'acétylcholine estérase est le donépézil. Dans un mode de réalisation particulièrement préféré, ladite combination pour son utilisation pour induire une neuroprotection chez un sujet en ayant besoin comprend un composé de formule (I) ou l'un de ses sels pharmaceutiquement acceptable ou le composé de formule (II), de l'acide méclofénamique l'un de ses sels pharmaceutiquement acceptable et du donépézil ou l'un de ses sels pharmaceutiquement acceptable. Dans un mode de réalisation particulier ledit sujet souffre, est suspecté de ou à risque de souffrir de la maladie d'Alzheimer.

Malgré des échecs en essai clinique de plusieurs thérapies basées sur des anticorps monoclonaux visant à diminuer la formation de plaque amyloïdes ou à empêcher ou diminuer l'hyperphosphorylation de la protéine Tau, cette stratégie reste une voie active dans la recherche de traitement contre la maladie d'Alzheimer. L'aducanumab est un anticorps humain recombinant qui montre une excellente sélectivité pour les agrégats fibrillaires oligomères solubles et insolubles d'A$\beta$, par rapport aux monomères non pathogènes d'A$\beta$ (Arndt *et al.,* 2018). Il est actuellement en essai clinique dans le traitement de la maladie d'Alzheimer.

Un objet de la présente invention est donc une combinaison comprenant un composé de formule (I) ou l'un de ses sels pharmaceutiquement acceptable ou le composé de formule (II) et une thérapie basée sur un anticorps visant à perturber la formation de plaque amyloïdes pour son utilisation telle que définie dans les revendications.

Un objet particulier de l'invention concerne une combinaison comprenant un composé de formule (I) ou l'un de ses sels pharmaceutiquement acceptable ou le composé de formule (II) et l'aducanumab ou un de ses fragments se liant à un antigène capable de lutter contre la toxicité de agrégats de $\beta$-amyloïde, de ses fragments ou de ses oligomères pour son utilisation pour induire une neuroprotection chez un sujet en ayant besoin, tel que défini dans les revendications.

Dans un mode de réalisation particulier, ledit sujet souffre, est suspecté de souffrir ou a risque de souffrir de la maladie d'Alzheimer, ou de démence à corps de Lewy.

Des agonistes des récepteurs $\sigma_1$ sont actuellement développés dans plusieurs indications neurodégénératives. Des effets neuroprotecteurs conférés par des agonistes des récepteurs $\sigma_1$ ont été observés dans certains modèles de la maladie d'Alzheimer mais aussi pour d'autres pathologies telles que la maladie de Parkinson, de Huntington, la sclérose latérale amyotrophique. Il a été montré que certains de ces agonistes ont des effets neuroprotecteurs (Meunier *et al.*, 2006 ; Maurice *et al.*, 2019). Des familles de composés agonistes des récepteurs $\sigma_1$ et neuroprotecteurs sont présentés dans la demande WO2017/191034. Egalement, le PRE-084 (2-morpholin-4-yléthyl 1-phénylcyclohexane-1-carboxylate), la (+)-pentazocine), le (+)-SKF10,047 (1,13-diméthyl-10-prop-2-enyl-10-azatricyclo[7.3.1.0$^{2,7}$]trideca-2(7),3,5-trien-4-ol), le SA4503 (1-[2-(3,4-diméthoxyphényl)éthyl]-4-(3-phénylpropyl)pipérazine), le 1-(2,2-Diphényltétrahydro-3-furanyl)-N,N-diméthylméthanamine, la fluvoxamine (2-{[(E)-{5-Méthoxy-1-[4-(trifluorométhyl)phényl]pentylidène}amino]oxy}éthanamine), le 4-IBP (*N*-(1-benzylpipéridin-4-yl)-4-iodobenzamide), l'igmesine ((5E)-N-(Cyclopropylméthyl)-N-méthyl-3,6-diphényl-5-hexén-3-amine), l'OPC-14523 (1-{3-[4-(3-Chlorophényl)-1-pipérazinyl]propyl}-5-méthoxy-3,4-dihydro-2(1H)-quinoléinone), le BD-737 ((1S,2R)-N-[2-(3,4-Dichlorophényl)éthyl]-N-méthyl-2-(1-pyrrolidinyl)cyclohexanamine), le BHDP (6-[(4-benzylpipérazin-1-yl)méthyl]-2,3-diméthoxyphénol, la pridopidine (4-(3-(méthylsulfonyl)phényl)-1-propylpipéridine) sont connus comme d'autres agonistes des récepteurs $\sigma_1$.

Ainsi un objet de la présente invention est donc une combinaison comprenant un composé de formule (I) ou l'un de ses sels pharmaceutiquement acceptable ou le composé de formule (II) et au moins un agoniste des récepteurs $\sigma_1$, tel que ceux listés dans la demande WO 2017191034, ou sélectionné parmi le PRE-084 (2-morpholin-4-yléthyl 1-phénylcyclohexane-1-carboxylate), la (+)-pentazocine, le (+)-SKF10,047 (1,13-diméthyl-10-prop-2-enyl-10-azatricyclo[7.3.1.0$^{2,7}$]trideca-2(7),3,5-trien-4-ol), le SA4503 (1-[2-(3,4-diméthoxyphényl)éthyl]-4-(3-phénylpropyl)pipérazine), le 1-(2,2-Diphényltétrahydro-3-furanyl)-N,N-diméthylméthanamine, la fluvoxamine (2-{[(E)-{5-Méthoxy-1-[4-(trifluorométhyl)phényl]pentylidène}amino]oxy}éthanamine), le 4-IBP (*N*-(1-benzylpipéridin-4-yl)-4-iodobenzamide), l'igmesine ((5E)-N-(Cyclopropylméthyl)-N-méthyl-3,6-diphényl-5-hexén-3-amine), l'OPC-14523 (1-{3-[4-(3-Chlorophényl)-1-pipérazinyl]propyl}-5-méthoxy-3,4-dihydro-2(1H)-quinoléinone), le BD-737 ((1S,2R)-N-[2-(3,4-Dichlorophényl)éthyl]-N-méthyl-2-(1-pyrrolidinyl)cyclohexanamine), le BHDP (6-[(4-benzylpipérazin-1-yl)méthyl]-2,3-diméthoxyphénol), la pridopidine (4-(3-(méthylsulfonyl)phényl)-1-propylpipéridine) ou un de leurs sels pharmaceutiquement acceptable, pour son utilisation pour induire une neuroprotection chez un sujet en ayant besoin, tel que défini dans les revendications.

Dans un mode de réalisation particulier, ledit au moins un agoniste des récepteurs $\sigma_1$ est choisi parmi le 2-(2-Chlorophényl)-2-oxo-3,3,5,5-tétraméthyl-[1 ,4,2]-oxazaphosphinane ; 2-(4-Chlorophényl)-2-oxo-3,3,5,5-tétraméthyl-[1 ,4,2]-oxazaphosphinane ; 2-(3,5-Dichlorophényl)-2-oxo-3,3,5,5-tétraméthyl-[1 ,4,2]-oxazaphosphinane ; 2-(2,3-Dichlorophényl)-2-oxo-3,3,5,5-tétraméthyl-[1 ,4,2]-oxazaphosphinane ; 2-(3-Fluorophényl)-2-oxo-3,3,5,5-tétraméthyl-[1 ,4,2]-oxazaphosphinane ; 2-(4-Fluorophényl)-2-oxo-3,3,5,5-tétraméthyl-[1 ,4,2]-oxazaphosphinane ; 2-(3-Nitrophényl)-2-oxo-3,3,5,5-tétramethyl-[1 ,4,2]-oxazaphosphinane ; 2-(4 benzyloxycarbamoylphényl)-2-oxo-3,3,5,5-tétraméthyl-[1 ,4,2]-oxazaphosphinane ; 2-(Pyridin-2-yl)-2-oxo-3,3,5,5-tétraméthyl[1 ,4,2]-oxazaphosphinane ; 2-(Pyridin-3-yl)-2-oxo-3,3,5,5-tétraméthyl[1 ,4,2]-oxazaphosphinane ; 2-(Pyridin-4-yl)-2-oxo-3,3,5,5-tétraméthyl[1 ,4,2]-oxazaphosphinane ; 2-(Pyrimidin-2-yl)-2-oxo-3,3,5,5-tétraméthyl-[1 ,4,2]-oxazaphosphinane ; 2-(Pyrimidin-5-yl)-2-oxo-3,3,5,5-tétraméthyl-[1 ,4,2]-oxazaphosphinane ; 2-(3-aminophényl)-2-oxo-3,3,5,5-tétraméthyl-[1 ,4,2]-oxazaphosphinane ; 2-(4-aminophényl)-2-oxo-3,3,5,5-tétraméthyl-[1 ,4,2]-oxazaphosphinane ; 2-(3-chlorophényl)-N-méthyl-2-oxo-3,3,5,5-tétraméthyl-[1 ,4,2]-oxazaphosphinane ; 2-(3-chlorophényl)-2-thiono-3,3,5,5-tétraméthyl-[1 ,4,2]-oxazaphosphinane ; 2-(3-Chlorophényl)-2-oxo-3,3,5,5-tétraméthyl-[1 ,4,2]-oxazaphosphinane, le PRE-084 (2-morpholin-4-yléthyl 1-phénylcyclohexane-1-carboxylate), la (+)-pentazocine, le (+)-SKF10,047 (1,13-diméthyl-10-prop-2-enyl-10-azatricyclo[7.3.1.0$^{2,7}$]trideca-2(7),3,5-trien-4-ol), le SA4503 (1-[2-(3,4-diméthoxyphényl)éthyl]-4-(3-phénylpropyl)pipérazine), le 1-(2,2-Diphényltétrahydro-3-furanyl)-N,N-diméthylméthanamine, la fluvoxamine (2-{[(E)-{5-Méthoxy-1-[4-(trifluorométhyl)phényl]pentylidène}amino]oxy}éthanamine), le 4-IBP (*N*-(1-benzylpipéridin-4-yl)-4-iodobenzamide), l'igmesine ((5E)-N-(Cyclopropylméthyl)-N-méthyl-3,6-diphényl-5-hexén-3-amine), l'OPC-14523 (1-{3-[4-(3-Chlorophényl)-1-pipérazinyl]propyl}-5-méthoxy-3,4-dihydro-2(1H)-quinoléinone), le BD-737 ((1S,2R)-N-[2-(3,4-Dichlorophényl)éthyl]-N-méthyl-2-(1-pyrrolidinyl)cyclohexanamine), le BHDP (6-[(4-benzylpipérazin-1-yl)méthyl]-2,3-diméthoxyphénol), la pridopidine (4-(3-(méthylsulfonyl)phényl)-1-propylpipéridine) ou un de leurs sels pharmaceutiquement acceptable, de préférence le 2-(3-Chlorophényl)-2-oxo-3,3,5,5-tétraméthyl-[1 ,4,2]-oxazaphosphinane, ou un de ses sels pharmaceutiquement acceptable. Le PRE-084 (2-morpholin-4-yléthyl 1-phénylcyclohexane-1-carboxylate) ou un de ses sels pharmaceutiquement acceptable est également préféré. Dans un mode de réalisation particulier, ledit au moins un agoniste des récepteurs $\sigma_1$ est dans une forme énantiomériquement pure. Dans un mode de réalisation préféré, ledit au moins un agoniste des récepteurs $\sigma_1$ est sous forme d'un mélange racémique. Dans un mode de réalisation particulier ledit sujet souffre, est suspecté de souffrir ou à risque de souffrir de la maladie d'Alzheimer.

Pour chacune des combinaisons selon invention, chacun des composés est adapté pour une administration simultanée, séparée ou étalée dans le temps comme définie plus haut, en fonction des spécificités de ces composés mais aussi, de préférence, de manière que ces composés ou leurs métabolites actifs exercent leurs effets biologiques au même moment, de manière que le sujet bénéficie de l'effet maximal de ladite combinaison.

Un objet de la présente invention concerne également une composition pharmaceutique comprenant au moins un composé de formule (I) ou l'un de ses sels pharmaceutiquement acceptable ou le composé de formule (II) seul ou en combinaison avec un autre agent actif, pour son utilisation telle que définie dans les revendications, ladite composition étant particulièrement adaptée pour conférer une neuroprotection selon les différentes et modes de réalisation évoqués.

**Exemples**

Abréviations

$A\beta_{25-35}$ : fragment de 11 acides aminés de séquence Nt-GSNKGAIIGLM-Ct (SEQ ID NO 1) du peptide APP.
Sc $AC_{25-35}$ : ou Sc Aβ, peptide contrôle comprenant les 11 acides aminés de $A\beta_{25-35}$ dans un ordre aléatoire de séquence Nt-MAKGINGISGL-Ct (SEQ ID NO 2).
YMT : test d'alternance spontanée dans le labyrinthe en Y ; « Y-maze test » en langue anglaise.
PAT : test d'évitement passif ; « Passive Avoidance Test » ou « step-through passive avoidance test » en langue anglaise.
ORT : test de reconnaissance d'objet ou « Object Recognition Test » en langue anglaise.
WMT : test d'apprentissage spatial en piscine ou « Morris Water Maze Test » en langue anglaise.
icv : intracérébroventiculaire.
i.p. : intrapéritonéal(e).
SNC : Système Nerveux Central.
Iba-1 : ionized calcium-binding adapter molécule 1
GFAP : Glial Fibrillary Acidic Protein.
*RaD : stratum radiatum* (Rad).
Mol : *stratum moleculare* (Mol).
PoDG : Couche polymorphe du gyrus denté.
LPtA : aire latérale pariétale associative (LPtA).

**1. Matériel et méthodes**

Les expérimentations animales sont conduites en accord avec les dispositions de la directive de l'Union Européenne N° 2010/63 et ont été dûment autorisées par le Comité Consultatif National d'Ethique (CCNE) de la république française.

**1.1. Animaux**

Les expérimentations *in vivo* ont été menées sur des souris mâles Swiss CD-1 (RjOrl:SWISS) ou C57Bl/6j (Janvier, Le Genest-Saint-Isle, France) âgées de 7 à 9 semaines. Les animaux sont hébergés par groupe de 8 à 10 individus dans des cages en plastiques avec un accès à volonté à la boisson et à la nourriture, dans un environnement contrôlé (23 ± 1°C, 40-60% d'humidité, cycle jour/nuit de 12 h). Toutes les expérimentations ont été menées sur les souris Swiss, excepté le test du hameau (cf. ci-dessous) qui a été effectué sur les souris de génotype C57Bl/6j.

**1.2. Composés tests et peptides**

<u>*Solutions stocks de mémantine et FENM (composés test).*</u> La FENM est le composé selon l'invention; la mémantine est le composé de référence.
Le chlorhydrate de mémantine a été utilisé (Sigma-Aldrich, Saint-Quentin-Fallavier, France). Le chlorhydrate de FENM (FENM HCl) a été synthétisée par M2i Life Sciences (Saint-Cloud, France). Les solutions stock des composés ont été obtenues par solubilisation en tampon NaCl (0,9%, véhicule) à une concentration de 2 ng/mL qui correspond à une dose de 10 mg/Kg pour 100 μL. Les solutions stocks sont conservées à 4°C pendant 2 semaines maximum.

<u>*Solution stock de peptide amyloide [25-35] ; formation des oliaomères.*</u>

Le peptide amyloïde [25-35], noté $A\beta_{25-35}$ (fournisseurs : Polypeptide, Illkirch, France ou Eurogentec, Angers, France), a été solubilisé dans de l'eau distillée stérile à une concentration de 3 mg/mL, la solution stock ainsi formée est aliquotée et conservée à -20°C jusqu'à utilisation.
L'injection de solution véhicule (eau distillée) donne la même absence d'effet que l'injection de peptide contrôle Sc Aβ (peptide contrôle), qui comprend les mêmes acides aminés que $A\beta_{25-35}$ dans un ordre aléatoire et qui ne s'oligomérise pas.
Les oligomères de $A\beta_{25-35}$ sont formés tel que décrit par Maurice *et al.* (1996), lors d'une incubation à 37°C pendant 4 jours. La solution véhicule ou le peptide contrôle sont soumis au même traitement avant administration.

<u>Administration aux animaux</u>

Les composés test (mémantine, FENM HCl) sont administrés aux animaux par la voie intrapéritonéale dans un intervalle de doses de 0.1 to 10 mg/Kg.
3 μL de solution d'oligomères de $A\beta_{25-35}$ (le véhicule, de l'eau distillée stérile, ou Sc Aβ) sont administrés aux souris par injection intracérébroventriculaire (icv), tel que décrit par, entre autres, Maurice *et al.* (1996), Meunier *et al.*

(2006) ou Villard *et al.* (2009).

### 1.3. Schémas expérimentaux : traitement symptomatique et neuroprotection

Le modèle d'injection icv d'oligomères d'$A\beta_{25-35}$ est un modèle bien connu dans l'état de l'art. Les oligomères d'$A\beta_{25-35}$ sont connus pour être cytotoxiques pour les cellules neuronales chez la souris et induire des troubles de la mémoire spatiale et de travail. Ce déficit s'accompagne par la génération d'un stress mitochondrial, d'un stress oxydant et d'une apoptose des cellules, notamment dans l'hippocampe, et par une inflammation du système nerveux central. Le peptide d'$A\beta_{25-35}$ est d'ailleurs compris dans le peptide d'$A\beta_{1-40}$ ou d'$A\beta_{1-42}$ et des auto-anticorps contre ces fragments courts ont été détectés chez l'Homme (Gruden *et al.,* 2007). Par ailleurs, le modèle a une validité prédictive remarquable vis-à-vis des études dans les modèles de souris transgéniques les plus utilisés de la maladie d'Alzheimer (Maurice *et al.*, 2013; Rodriguez-Cruz *et al.,* 2017). Ainsi, bien que l'installation de la maladie soit rapide, ce modèle est considéré comme un modèle pertinent de criblage pour l'activité neuroprotectrice de composés, et plus particulièrement un modèle de première intention pertinent de la maladie d'Alzheimer. Au moins une molécule essentiellement étudiée sur ce modèle (Villard *et al.,* 2009, 2011) se révèle efficace en clinique (Hampel *et al.,* 2020) et est aujourd'hui en phase 3.

La Figure 1 est une description schématique du déroulement des différents tests en fonction du jour de l'administration icv des oligomères $A\beta_{25-35}$ et des composés tests.

Les composés test ont été testés pour leur capacité à diminuer les symptômes cognitifs de la neurodégénération induite par les oligomères $A\beta_{25-35}$, c'est-à-dire leur capacité de traitement symptomatique anti amnésique (Figures 1A et 1B). Brièvement, pour tester l'effet symptomatique des composés test, ceux-ci ont été administrés 8 jours après l'injection icv des oligomères $A\beta_{25-35}$ et ce, 30 minutes avant la réalisation des tests de mémoire YMT et PAT ou 30 minutes avant le test, mais après la session d'entrainement, dans le cas des tests MWT ou ORT (Figure 1A). Le test du hameau (Hamlet test®, Figure 1B) implique une session d'entrainement de 2 semaines (4 h/jour) puis un test (10 min max) en condition de privation d'eau et en condition normale ; après ce premier test, le peptide $A\beta_{25-35}$ est injecté. Après une semaine, les composés sont administrés puis un second test en conditions de privation d'eau et en condition normale est effectué 30 min après injection. Ce test mesure la désorientation spatio-temporelle, un signe d'alerte majeur dans la maladie d'Alzheimer (Crouzier *et al.*, 2018).

L'effet neuroprotecteur des composés c'est-à-dire leur capacité à protéger les cellules de la neurotoxicité des oligomères $A\beta_{25-35}$ et de la neurodégénérescence est également testé (Figure 1C). Pour cela l'administration des composés test a lieu le même jour que l'injection des oligomères $A\beta_{25-35}$ et se poursuit sur une base journalière jusqu'au jour 7 après l'injection des oligomères ; les souris sont alors soumises aux tests YMT, PAT, MWT ou ORT. L'analyse de l'anatomie du cerveau et le dénombrement des cellules neuronales par immunohistochimie est effectuée sur des souris sacrifiées à J13. Les analyses biochimiques de quantification des marqueurs du stress oxydant et de l'apoptose ont été effectuées à J16 sur un groupe de souris ayant été soumis au WMT (Figure 1C).

Ainsi, dans un cas, on laisse les oligomères $A\beta_{25-35}$ exercer leur neurotoxicité et induire la neurodégénérescence avant de mettre en oeuvre le traitement, alors que dans l'autre cas on évalue la capacité des composés à empêcher cette cascade d'événements et donc à prévenir la neurodégénérescence et la mort cellulaire.

### 1.4. Tests cognitifs/ comportementaux

*Test d'alternance spontanée dans le labyrinthe en Y (YMT)*

Le test d'alternance spontanée est utilisé pour étudier la mémoire spatiale de travail (à très court terme) chez les rongeurs. Le labyrinthe est fait de chlorure de polyvinyle (PVC) opaque gris. Chacun de ses bras mesure 40 cm de long, 13 cm de haut et 3 cm de large à sa base, 10 cm à son sommet. Les bras convergent l'un vers l'autre, avec un angle égal entre les différents bras. Brièvement, chaque souris est placée à l'extrémité d'un bras et est laissée libre de se mouvoir pour une session de 8 min. Les entrées de la souris dans chacun des bras, y compris le bras à l'extrémité duquel elle a été déposée, sont enregistrées. Une alternance est définie comme l'entrée successive de l'animal dans trois bras différents. Le nombre d'alternance maximale est donc le nombre total d'entrées dans chacun des bras moins 2 et le pourcentage d'alternance est calculé selon la formule :

$$\%\text{Alt} = \text{nombre d'alternance réalisées} / _{nombre\ d'alternance\ maximum} \times 100$$

Les paramètres mesurés comprennent le pourcentage d'alternance (indice de mémoire) et le nombre total d'entrée dans les bras du labyrinthe (indice d'exploration, Maurice *et al.,* 1994, 1996; Meunier *et al.,* 2006, 2013; Villard *et al.,* 2009, 2011). Les données des animaux montrant des comportements extrêmes (pourcentage d'alternance < 25% ou > 90%, ou bien un nombre d'entrée inférieur à 10) ne sont pas prises en compte dans les calculs. Le taux d'attrition

est d'ordinaire de 5%. En conditions normales, une souris va spontanément alterner les explorations des chacun des bras. Une souris ayant des capacités de mémoire et / ou d'orientation altérées verra son pourcentage d'alternance diminuer.

*Test d'évitement passif (PAT)*

Ce test mesure la mémoire non-spatiale (contextuelle) à long-terme. L'appareil utilisé pour ce test est une boîte à deux compartiments (15 x 20 x 15 cm de haut) l'un éclairé avec des parois en PVC blanc et l'autre à l'obscurité avec des parois de PVC noir et un sol grillagé. Une porte guillotine sépare les compartiments. Une lampe de 60 W est positionnée à 40 cm au-dessus de la boite et éclaire le compartiment blanc. Des chocs électriques (0,3 mA pendant 3 s) peuvent être appliqués au sol de la grille *via* un générateur (Lafayette Instruments, Lafayette, USA). Le test comprend une session d'apprentissage et une session de test. La porte à guillotine est fermée pendant la session de formation. Chaque souris est placée dans le compartiment blanc. Après 5 secondes, la porte se lève. Quand la souris entre dans le compartiment obscur de façon que ses quatre pattes soient en contact avec le grillage, la porte se ferme et des décharges électriques sont appliquées pendant 3 secondes. Le temps mis par la souris pour rentrer dans le compartiment obscur (STL-Tg) et le nombre de vocalises sont relevés. La session de test est effectuée 24 heures après la session d'apprentissage. Chaque souris est replacée dans le compartiment blanc éclairé. Après 5 s, la portes se soulève, le temps de latence (STL-R), c'est-à-dire le temps que met la souris pour aller dans le compartiment sombre est mesuré. La durée maximum est 300 s. Les souris présentant un STL-Tg > 30 s ou un STL-Tg et un STL-R < 10 s ne sont pas prises en compte pour le test. Le taux d'attrition est habituellement de 5% (Meunier *et al.,* 2006, 2013 ; Villard *et al.,* 2009, 2011). Une souris ayant des capacités de mémoire altérée aura un STL-R inférieur à celui d'une souris aux capacités normales.

*Test de reconnaissance d'objet*

Ce test est utilisé pour évaluer les capacités de mémoire de reconnaissance (Rodríguez Cruz *et al.,* 2017; Maurice *et al.*, 2019). Les souris sont chacune placées dans une arène carrée (50 x 50 $cm^2$). Lors de la session 1, on laisse les souris s'acclimater à l'environnement pendant 10 minutes. Lors de la session 2, 24 heures après la session 1, deux objets identiques sont placés au ¼ (position 1) et au ¾ (position 2) de l'une des diagonales de l'arène. Le comportement exploratoire, activité et position du nez de l'animal, sont enregistrés pendant 10 minutes (logiciel Nosetrack®, Viewpoint, Lissieu, France). Le nombre de contacts avec les objets ainsi que leur durée sont mesurés. Lors de la session 3, une heure après la session 2, l'objet en position 2 est remplacé par un nouvel objet différent par sa forme, sa couleur et sa texture. Le comportement exploratoire de chaque souris est alors également enregistré pendant 10 minutes. Les animaux n'ayant aucun contact ou moins de 10 contacts avec un objet sont écartés de l'étude.

Un index exploratoire est calculé selon la formule suivante :

$$Index\ exploratoire = \frac{nombre\ (ou\ durée\ )des\ contacts\ avec\ l'objet\ 2}{nombre\ total\ (ou\ durée)des\ contacts\ avec\ les\ deux\ objets}$$

Ce test se base sur le comportement exploratoire inné des souris. Il est utilisé pour mesurer la mémoire de reconnaissance qui implique de nombreuses aires du cerveau. Une souris ayant des capacités de mémoire altérées aura moins tendance à préférer l'objet 2 dans son comportement exploratoire.

*Test d'apprentissage spatial en piscine (ou Labyrinthe de Morris)*

Ce test est utilisé pour évaluer la mémoire spatiale de référence à long-terme. Ce test est bien connu de l'état de l'art et est notamment décrit par Rodríguez Cruz *et al.* (2017) et Maurice *et al.* (2019). Il comprend deux phases, une phase d'acquisition et une phase de test. La piscine est de forme circulaire (d'un diamètre de 140 cm) et une plateforme de 10 cm est immergée sous la surface de l'eau lors de la phase d'acquisition. Des repères sont placés autour de la piscine pour permettre aux animaux de s'orienter. La nage des animaux est enregistrée (logiciel Videotrack®, Viewpoint), et les trajectoires, temps de latence et distances parcourues mesurées. La phase d'acquisition comprend des sessions de 3 nages par jour pendant 5 jours. La position de départ de l'animal est déterminée aléatoirement chaque à partir des points cardinaux nord, sud, est, ouest. Chaque animal a 90 secondes pour trouver la plateforme immergée au milieu du quart nord-est, l'eau étant rendue opaque par suspension de blanc de Meudon. Les animaux sont laissés 20 secondes sur la plateforme. Les animaux n'ayant pas trouvé la plateforme après 90 secondes sont placés sur la plateforme et y sont laissés pour 20 secondes également. La médiane du temps de latence (temps mis pour trouver la plateforme) est calculée à chaque jour d'entrainement et exprimée pour le groupe en moyenne du temps de latence ± l'écart type. Un

test de rappel est effectué 72 heures après la dernière session d'entrainement. Dans ce test, la plateforme a été retirée. Chaque souris est laissée 60 secondes dans l'eau et sa nage est enregistrée. Le temps passé dans le quart nord-est, qui contenait à l'origine la plateforme (T), est mesuré et comparé à la moyenne du temps passé dans les autres quarts (o).

Une souris ayant des capacités de mémoire spatiale de référence altérées passera moins de temps (en proportion par rapport au temps passé dans les autres quarts) dans le quart qui contenait la plateforme lors des phases d'entrainement, voire pas significativement plus que le niveau de hasard (15 secondes).

*Mémoire topographique, test du hameau*

Ce test est destiné à mesurer la mémoire topographique des souris dans un environnement complexe. Il repose sur la familiarisation de l'animal face à un environnement complexe et enrichi, et sur l'apprentissage, dans ce contexte enrichi, par l'acquisition de fonctions mémorielles et sociales supérieures (Crouzier *et al.,* 2018).

Le dispositif du Hamlet Test®, d'un diamètre de 1,2 m comprend une place centrale (l'agora) et des rues qui en rayonnent et mènent à des compartiments fonctionnalisés ou maisons permettant de satisfaire une fonction physiologique ou d'enrichissement du milieu. Les murs et les rues du hameau sont faits de PVC transparent aux infrarouges, la pièce d'expérimentation est uniformément éclairée (200 Lux), des diodes infrarouges sont placées sous le sol du hameau et une caméra infrarouge enregistre le comportement des animaux. L'agora sert de lieu de rencontre et de point de départ pour les sessions d'entrainement et de test. Les maisons encodent des fonctions physiologiques de base et contiennent soit des granules (maison Manger), de l'eau (maison Boire), un labyrinthe Novomaze® (Viewpoint) (maison Se Cacher), une roue d'entrainement (maison Courir) ou une grille de séparation isolant une souris inconnue (maison Interagir).

Ce test a été décrit par Crouzier *et al.* (2018 a et b). Brièvement, les animaux d'une même cage sont placés dans le hameau, pendant 4 h par jour pendant les deux semaines d'entrainement. La mémoire topographique est testée lors d'une phase de test (PT0), 72 heures près la dernière session d'entrainement, après que les souris ont eu subi une privation d'eau (biberon retiré la veille au soir, soit 15 h avant le test). Les performances de animaux pour atteindre la maison Boire sont comparées aux performances des mêmes animaux, retestés le lendemain sans privation d'eau. Les animaux sont placés individuellement dans le hameau pour une session de 10 minutes. Le comportement exploratoire est enregistré et le temps mis pour trouver la maison Boire et le nombre d'erreur consignés (entrée dans une rue ne menant pas à la maison Boire). Les peptides $A\beta_{25-35}$ oligomérisés, ou le témoin sont injectés 2 heures après la phase de test et les composés tests également (Figure 1B). La phase de test est réitérée après 7 jours. Un indice de désorientation (DI) est calculé pour chaque variable (temps pour trouver la maison eau, nombre d'erreurs) :

$$DI = \frac{performance\ avant\ injection\ des\ oligomères\ A\beta 25-35}{performance\ après\ injection\ des\ oligomères\ A\beta 25-35} - \frac{performance\ avant\ injection\ du\ véhicule}{performance\ après\ injection\ ddu\ véhicule}$$

**1.5. Analyses biochimiques**

*Peroxydation des lipides membranaires*

Comme mentionné sur la Figure 1C les souris sont sacrifiées après le test comportemental par décapitation 15 jours après injection de $A\beta_{25-35}$. Les cerveaux sont prélevés, les hippocampes isolés, pesés et congelés à -80°C jusqu'aux analyses biochimiques.

La quantité d'hydroperoxyde présent dans les échantillons est mesurée par la quantification du complexe $Fe^{3+}$ avec le xylénol orange : en milieu acide, les hydroperoxydes oxydent le $Fe^{2+}$ en $Fe^{3+}$. Ce dernier forme un complexe coloré avec le xylénol orange, dont la formation est quantifiée par la mesure de son absorbance à 580 nm.

Après décongélation, les hippocampes sont homogénéisés dans du méthanol froid (1/10 w/v), centrifugés à 1 000 *g* pendant 5 min et le surnageant prélevé. Les homogénats sont ajoutés à une solution contenant du $FeSO_4$ (0.25 mM), d'$H_2SO_4$ (25 mM), et d'orange de xylénol (0.1 mM) et incubés 30 min à température ambiante. L'absorbance à 580 nm ($A_{580}1$) est mesurée puis 10 $\mu$l d'hydroperoxide de cumène (CHP, 1 mM) sont ajoutés et le tout est incubé 30 min à température ambiante. L'absorbance à 580 nm est une nouvelle fois mesurée ($A_{580}2$). Le niveau de peroxydation des lipides est déterminé en équivalent CHP (CHP eq.) selon la formule :

$$\text{CHP eq.} = \frac{A_{580}1}{A_{580}2} \times [\text{CHP (nmol)}]\text{x facteur de dilution}$$

CHP eq. est exprimé par masse de tissu et présenté en pourcentage de la valeur du groupe contrôle.

*Tests ELISA, quantification des marqueurs de l'inflammation et de l'apoptose*

L'interleukine 6 (IL-6), cytokine marqueur de l'inflammation, et les niveaux des protéines Bcl2 et Bax, marqueurs de l'apoptose ont été mesurés dans les hippocampes de souris par les tests ELISA listés dans le tableau 1 ci-dessous. Bcl2 est souvent désigné comme un marqueur anti apoptotique et Bax, comme un marqueur pro-apoptotique. Les deux hippocampes de 6-8 animaux par conditions ont été utilisés. Les tissus ont été homogénéisés après décongélation dans en tampon de lyse cellulaire (1 mL, 3 IS007, Cloud-Clone) et sonication sur la glace (2 x 10 s). Après centrifugation (10 000 *g,* 5 min, 4°C), les surnageants contenant les marqueurs à mesurer ont été aliquotés et stockés à -80°C jusqu'au test ELISA réalisé selon les instructions du fournisseur. Les résultats sont exprimés en ng de marqueur par mg de protéines totales et en pourcentage du contrôle (souris non intoxiquées par $A\beta_{25-35}$ et non traitées avec les composés tests).

Tableau 1

| Marqueur | Fournisseur | Référence |
|---|---|---|
| IL-6 | Cloud-Clone Corp | SEA079MU |
| Bax | Cloud-Clone Corp | SEB343MU |
| Bcl-2 | Cloud-Clone Corp | SEA778MU |

**1.6. Analyses immunohistochimiques**

*Préparation des coupes de tissus*

Comme indiqué Figure 1C, au jour 13, pour chaque condition expérimentale, 5-6 souris ont été euthanasiées pour mener ces études. Elles ont été préalablement anesthésiées par injection i.p. de 200 µL d'une solution de kétamine (80 mg/Kg) et de xylazine (10 mg/Kg), puis les tissus fixés par perfusion intracardiaque de 50 mL d'une solution saline puis de 50 mL de fixateur Antigenfix® (Diapath). Les échantillons sont conservés pour 48 heures supplémentaires dans le fixateur à 4°C, puis les cerveaux des souris ont été conservés dans une solution de saccharose 30% en PBS, avant d'être coupés en tranches.

Des tranches de chaque aire du cerveau ont été réalisées, notamment du cortex, du noyau *basalis* magnocellulaire et de l'hippocampe (*i.e.,* entre le bregma +1,8 et le bregma -2,8 selon les coordonnées stéréotaxiques du cerveau de souris, Paxinos *et al.,* 2004). Les coupes coronales en série d'une épaisseur de 25 µm ont été réalisées au cryomicrotome (Microm HM 450, Thermo Fisher) et conservées à -20°C dans une solution cryoprotectrice.

*Quantification des neurones viables de l'aire CA 1*

Chez les malades d'Alzheimer, une diminution des neurones dans la région CA1 (et dans une moindre mesure CA3) a été reportée. Une telle diminution s'observe également dans les modèles murins d'injection icv d'oligomères de $A\beta_{25-35}$. Pour quantifier les neurones viables de la région CA1 de l'hippocampe des souris, les coupes correspondantes ont été colorées au crésyl violet (0,12%, Sigma-Aldrich), puis déshydratées à l'éthanol, traitées au xylène et montées avec du milieu de montage (Mountex medium, BDH Laboratory Supplies) et séchées à 24°C à température ambiante. Les images ont été enregistrées et analysées (Nanozoomer virtual microscopy system, Hamamatsu, Massy, France). L'épaisseur de l'aire CA1 ainsi que le nombre de neurones pyramidaux ont été mesurés au grossissement 20x (cell count macro of ImageJ v1.46 software (NIH)). Les données sont exprimées en nombre de cellules viables par $mm^2$ pour 4-6 coupes de l'hippocampe par souris (Rodríguez Cruz *et al.,* 2017 ; Maurice *et al.*, 2019).

*Quantification Immunohistochimique des marqueurs de l'inflammation GFAP et Iba-1*

Les cellules microgliales sont souvent présentées comme les macrophages du SNC. Elles prolifèrent et s'activent pour faire face à une situation pathologique. Elles peuvent sécréter des cytokines ou des espèces réactives de l'oxygène et sont ainsi partiellement responsables du phénomène d'excitotoxicité. Elles sont aussi un marqueur de la

neuroinflammation pathologique. Le marquage Iba-1 est spécifique des cellules microgliales dans le SNC. La protéine GFAP est un des composants des filaments intermédiaires des astrocytes et est utilisée comme marqueur des astrocytes. Les astrocytes jouent un rôle de support fonctionnel et structurel des neurones. Néanmoins, les astrocytes participent également à la neuroinflammation et peuvent produire de nombreuses cytokines qui exercent une activité neurotoxique.

Les marquages d'immunohistochimie sont effectués de manière conventionnelle. Brièvement, des anticorps polyclonaux de lapin anti-Iba-1 (référence 019-19741, Wako) et des anticorps monoclonaux de souris anti-GFAP (référence G3893, Sigma-Aldrich) dilués respectivement au $250^{ème}$ et au $400^{ème}$ ont été utilisés pour marquer les cellules microgliales et les astrocytes. Le marquage a eu lieu sur la nuit à 4°C. L'hybridation avec des anticorps secondaires anti-lapin couplés au Cy3 et des anticorps secondaires anti-souris couplés à l'Alexa fluor 488, dilués au 1000ème, a lieu pendant une heure à température ambiante. Les coupes sont ensuite incubées 5 min dans une solution de DAPI à 10 ug/mL. Après un rinçage en PBS les coupes ont été montées dans un liquide de montage (ProLong, ThermoFischer) et des images de chaque coupe ont été prises au microscope confocal à fluorescence (Leica SPE) pour différentes sous régions de l'hippocampe telles que le *stratum radiatum* (Rad), le *stratum moleculare* (Mol), la couche polymorphe du gyrus denté (PoDG) et, pour le cortex, l'aire latérale pariétale associative (LPtA). Ces régions sont connues pour être des sites de l'astrogliose et de la microgliose induites par les oligomères $A\beta_{25-35}$ (Maurice *et al.,* 2019).

**1.7. Analyses statistiques**

Les analyses ont été effectuées en utilisant Prism v5.0 (GraphPad Software, San Diego, CA, USA). Les données ont été analysées à l'aide d'analyses de variance unidirectionnelles (ANOVA, valeur F), suivies d'un test de Dunnett. Les latences dans le test d'évitement passif ont été analysées par un test non-paramétrique de Kruskal-Wallis (ANOVA (valeur H), suivies d'un test de Dunn. Dans le test de la piscine de Morris, le temps passé dans les quadrants T ou o sont analysés en utilisant un t-test pour un échantillon pour les données du test de rétention vs. 15 s. Il en est de même pour les données de la session 3 du test de reconnaissance d'un objet, la préférence étant analysée suivant le temps passé à explorer l'objet et le nombre de contacts avec l'objet vs 50%. Les niveaux statistiques de significativité sont : $p < 0.05$, $p < 0.01$ et $p < 0.001$.

**2. Résultats**

**2.1. Neuroprotection et prévention de l'inflammation, l'apoptose et le stress oxydant**

*Marqueurs biochimiques de l'inflammation, de l'apoptose et du stress oxydant dans l'hippocampe.*

L'effet neuroprotecteur de la mémantine et de la FENM a été évalué au niveau biochimique par la mesure, dans l'hippocampe, du niveau de marqueurs de l'inflammation (IL-6, Figure 2A), de l'apoptose (Bax et Bcl-2, et le rapport Bax/BI-2, Figure 2C) et du stress oxydant (peroxydation des lipides, Figure 2B).

Dans l'hippocampe des souris non traitées, mais intoxiquées avec des oligomères de $A\beta_{25-35}$, une augmentation significative de 83% des niveaux d'IL-6 (cytokine pro-inflammatoire) est observée 5 jours après l'intoxication par rapport aux souris non intoxiquées, non traitées confirmant l'induction de la neuroinflammation par intoxication aux oligomères $A\beta_{25-35}$. Le traitement des souris avec la mémantine (0.3 mg/Kg) ne permet pas d'obtenir une diminution significative des niveaux d'IL-6, par rapport au souris intoxiquées mais non traitées. A l'inverse, le niveau d'IL-6 observé dans les hippocampes des souris traitées à la FENM est significativement inférieur à celui des souris intoxiquées, non traitées, et atteint des niveaux du même ordre que ceux observés pour les souris non traitées non intoxiquées. (Figure 2A). Ainsi la FENM permet de prévenir l'inflammation induite par les oligomères de $A\beta_{25-35}$.

De manière attendue (Maurice *et al.,* 2013), aucune augmentation significative du niveau de la protéine anti apoptotique Bcl2 dans les groupes intoxiqués n'est observée (non montré). Les niveaux de la protéine Bax sont eux significativement augmentés du fait de l'intoxication aux oligomères $A\beta_{25-35}$ (non montré). Cette augmentation est significativement diminuée (par rapport au groupe intoxiqué, non traité) par la mémantine (0,3 mg/Kg) et par la FENM (0,3 mg/Kg) (non montré). Néanmoins, seul le traitement des souris avec la FENM (0,3 mg/Kg) permet de diminuer significativement le rapport Bax/Bcl-2 qui est significativement augmenté du fait de l'intoxication $A\beta_{25-35}$dans les souris non traitées à la FENM (figure 2C) et qui est connu dans l'état de l'art comme un marqueur du potentiel apoptotique des cellules. Ainsi seule la FENM permet de prévenir l'apoptose induite par les oligomères $A\beta_{25-35}$ déterminée par l'augmentation rapport Bax/Bcl-2.

Il a été observé par ailleurs que le traitement à la FENM (0,3 mg/Kg i.p.) permet le maintien du nombre de neurones viables dans l'aire CA1 de l'hippocampe de même que de contrer l'augmentation de l'épaisseur de cette aire chez les souris intoxiquées par les oligomères, alors que les souris intoxiquées non traitées présentent une diminution de 13 % du nombre de cellules viables dans l'aire CA1, associée à une augmentation de 12 % de l'épaisseur de l'aire CA1 (non montré). Ainsi, la FENM permet de prévenir les altérations cellulaires et structurales de l'hippocampe induites

par les oligomères $A\beta_{25-35}$.

En lien avec l'apoptose, la dysfonction mitochondriale est aussi à la source du stress oxydant. La peroxydation des lipides est un marqueur à long terme du stress oxydant. Comme attendu, l'intoxication aux oligomères $A\beta_{25-35}$ induit une augmentation significative (+47%, Figure 2B) du niveau de peroxydation des lipides dans les cellules de l'hippocampe des souris intoxiquées. La mémantine (0,3mg/Kg) ne permet pas de diminuer le niveau de peroxydation des lipides de manière significative et est donc inefficace à prévenir le stress oxydant induit par l'intoxication aux oligomères $A\beta_{25-35}$. Un niveau équivalent à celui du groupe témoin (non intoxiqué, non traité) et significativement plus faible de celui des souris intoxiquées mais non traitées est observé dans l'hippocampe des souris traitées à la FENM (0,3 mg/Kg). Ainsi, comparé à la mémantine, seule la FENM est capable de prévenir le stress oxydant observé chez les souris intoxiquées tel que mesuré par la peroxydation des lipides.

Ainsi seule la FENM est capable d'assurer une neuroprotection efficace (au sens de l'intensité et de la significativité statistique) contre l'apoptose et l'inflammation induites par les oligomères $A\beta_{25-35}$.

*Observation de la neuroinflammation en immunohistochimie.*

L'intoxication aux oligomères $A\beta_{25-35}$ induit une augmentation importante et significative (par rapport aux souris non intoxiquées) des marquages GFAP (Figure 3, astrogliose) et Iba-1 (Figure 4, microgliose) dans le cortex (LPtA, respectivement +110% et +55%). Une augmentation de GFAP et de Iba-1 est également observée dans l'hippocampe des souris intoxiquées dans les trois zones étudiées (Rad, Mol et PoDG) ; elle est significative, concernant le marquage GFAP pour le Rad (+68%) et le Mol (+52%) et, pour le marquage Iba-1, pour le Rad (+68 %) (Figures 3 et 4). Bien que la mémantine présente un effet positif dans la zone PoDG, seule la FENM permet une diminution significative de la réaction astrocytaire et microgliale dans l'ensemble des zones du cerveau testées (Figures 3 et 4). La mémantine est en outre dépourvue de tout effet concernant l'astrogliose dans le Mol (Figure 3 B) ou la microgliose dans le cortex (Figure 4D). Ainsi la FENM est nettement plus efficace que la mémantine dans la prévention de la réaction inflammatoire déclenchées par l'intoxication aux oligomères $A\beta_{25-35}$.

La FENM se montre donc particulièrement plus efficace au regard de la mémantine pour prévenir le développement des mécanismes inflammatoires, de stress oxydant et d'apoptose observés dans la neurodégénération au niveau moléculaire (marqueurs biochimiques) et cellulaire et morphologique dans un plan expérimental de neuroprotection.

En outre, seule la FENM permet d'obtenir une amélioration significative pour l'ensemble des marqueurs étudiés.

2.2. Neuroprotection et préservation des capacités cognitives.

Il a ensuite été vérifié si les capacités neuroprotectrices spécifiques de la FENM détectées au niveau biochimique et cellulaire dans le cerveau se répercutaient sur les capacités cognitives des animaux traités avec la FENM, en comparaison avec la mémantine.

Les effets neuroprotecteurs de la FENM et de la mémantine ont été évalués et comparés, dans le cadre de test cognitifs et comportementaux de mesure de la mémoire de travail, de la mémoire à moyen terme, de la mémoire de reconnaissance, de la mémoire spatiale à long terme (apprentissage) et des capacités d'orientation. Ces tests ont été réalisés selon les schémas expérimentaux de la Figure 1C. Sauf spécification contraire la mémantine et la FENM (composés tests) ont été administrées aux doses de 0,03 ; 0,1 ; 0,3 ; 1,0 et 3 mg/Kg. Les doses testées sont ainsi réparties de façon logarithmique dans l'intervalle de doses testé, ce qui permet de comparer les profils d'activité des deux molécules.

*Effet supérieur de la FENM dans le test de la piscine de Morris.*

Ce test correspond à l'évaluation de la mémoire spatiale à long terme et aux capacités d'apprentissage.

Les oligomères $A\beta_{25-35}$ induisent une perte des fonctions d'apprentissage et de mémoire à long terme (Figure 5). Chez les souris non intoxiquées non traitées (groupe témoin) il est observé que les souris passent un temps significativement supérieur à 15 s dans le quadrant d'entrainement ; une différence significative est également observée entre le temps passé dans le quadrant d'entrainement par rapport au temps passé dans les autres quadrants. Les souris intoxiquées non traitées passent, elles, quasiment autant de temps dans les autres quadrants que dans le quadrant d'entrainement et le temps passé dans le quadrant d'entrainement n'est plus significativement différent de 15 s. L'administration de mémantine à 0,3 mg/Kg ne permet pas de prévenir l'atteinte des fonctions d'apprentissage et de mémoire. En revanche, les souris intoxiquées mais traitées par la FENM 0,3 mg/Kg maintiennent leur capacité d'apprentissage et de mémoire puisqu'elles passent significativement plus de temps dans le quadrant d'apprentissage que les autres quadrants et que ce temps est significativement supérieur à 15 s (Figure 5).

Ainsi seule la FENM permet une protection significative des capacités cognitives des animaux mesurées par

ce test.

*Effet supérieur de la FENM dans le test d'évitement passif*

Comme mentionné dans la partie matériel et méthodes, ce test est un test de motivation par la peur classiquement utilisé pour évaluer la mémoire à moyen terme.

Une diminution importante du temps de latence à rentrer dans le compartiment obscur est observé pour les souris intoxiquées par les oligomères $A\beta_{25-35}$ en comparaison avec les souris non intoxiquées (non montré). Un effet protecteur de la mémantine n'est observé que deux des doses testées (0,1 et 1 mg/Kg i.p.). *A contrario,* il est observé que la FENM confère une neuroprotection de la mémoire pour toutes les doses testées à partir de 0,1 mg/Kg i.p. jusque, compris, 3mg/Kg.

La FENM est donc plus efficace en neuroprotection que la mémantine, y compris pour des doses plus importantes auxquelles la mémantine ne présente pas d'effet. Ainsi, la FENM est plus efficace dans la prévention contre les troubles cognitifs dans le modèle de neurodégénérescence induite par injection icv des oligomères $A\beta_{25-35}$.

**2.3. La FENM est dépourvue de l'effet amnésique de la mémantine aux mêmes doses.**

Comme mentionné précédemment, il a été observé que la mémantine présente des effets délétères pour la cognition quand administrée à des doses importantes, dans les modèles animaux mais aussi chez l'homme. Dans cette expérimentation, la mémantine et la FENM ont été administrées à une dose de 10 mg/Kg i.p. et les performances des souris ont été testées dans le test d'évitement passif et le labyrinthe en Y, chez des souris non intoxiquées. Les résultats sont reportés dans le tableau 2 ci-dessous :

Tableau 2

| | **Véhicule** | **Mémantine (10 mg/Kg i.p.)** | **FENM (10 mg/Kg i.p.)** |
|---|---|---|---|
| **Labyrinthe en Y** | | | |
| Alternance (%) | 70.8 ± 3.8 | 48.8 ± 3.0*** | 63.7 ± 4.2 |
| Nombre d'entrées | 30.8 ± 1.3 | 28.2 ± 2.9 | 32.7 ± 3.4 |
| **Evitement passif** | | | |
| Temps de latence (s) | 220.8 ± 39.3 | 56.0 ± 31.1** | 183.0 ± 38.4 |
| N | 8 | 9 | 7 |
| ANOVA: $F_{(2,23)} = 10.3$, $p < 0.001$ pour l'alternance; $F_{(2,23)} = 0.728$, $p > 0.05$ pour le nombre d'entrées. Kruskal-Wallis ANOVA: $H = 11.4$, $p <0.01$ pour le test d'évitement passif. ** $p < 0.0034$ *vs.* véhicule, test de Dunnett. | | | |

Les données rassemblées dans le tableau 2 montrent bien un effet négatif de la mémantine sur la mémoire de travail et à moyen terme de l'animal. Une diminution significative du nombre d'alternances dans le labyrinthe en Y et du temps de latence dans le test d'évitement passif sont observées chez les animaux administrés avec la mémantine. On notera que l'effet délétère de la mémantine sur les performances des animaux dans ces tests est du même ordre de grandeur que celui des oligomères $A\beta_{25-35}$ observé, par exemple, Figure 6. A l'inverse, aucune atteinte mnésique significative n'est reportée pour les animaux administrés avec la FENM (tableau 2).

Cette absence d'effet amnésique est particulièrement intéressante car, outre les effets neuroprotecteurs (et symptomatiques cf. ci-dessous) supérieurs de la FENM, elle permet d'envisager des traitements avec des doses plus importantes que celles aujourd'hui acceptées pour la mémantine et donc l'obtention d'une concentration plus efficace dans le SNC, ce qui, joint à une efficacité supérieure, permet d'envisager un traitement plus efficace chez l'homme.

**2.4. Traitement symptomatique des atteintes cognitives par la FENM.**

Pour évaluer le traitement symptomatique des déficiences cognitives résultant de la neurotoxicité induite par les oligomères $A\beta_{25-35}$, les molécules ont été administrées selon le schéma expérimental de la Figure 1 (A) et (B). L'amélioration symptomatique des troubles de la mémoire est connue, pour la mémantine, dans les modèles animaux mais sont décevants chez l'homme ; ses effets ont été comparés avec ceux de la FENM. Les effets anti amnésiques de la FENM à la suite de l'intoxication par les oligomères $A\beta_{25-35}$ ont été confirmés dans tous les tests comportementaux testés (labyrinthe en Y, évitement passif, reconnaissance d'objet (non montré), piscine de Morris (non montré)). Sauf spécification contraire la mémantine et la FENM (composés tests) ont été administrées aux doses de 0,1 ; 0,3 ; 1,0 ; 3

et 10 mg/Kg. Les doses testées sont ainsi réparties de façon logarithmique dans l'intervalle de doses testé, ce qui permet de comparer les profils d'activité des deux molécules.

D'une manière générale, il est observé que la FENM est efficace dans un intervalle de doses plus large que la mémantine et notamment pour les doses les plus importantes de l'intervalle. Par exemple, le traitement par la FENM s'est avéré supérieur dans le test du labyrinthe en Y (Figure 6) puisqu'efficace à toutes les doses testées à partir de 0.1 mg/Kg, là où la mémantine n'est efficace qu'à 0,3 mg/Kg pour restaurer un Alt% significativement différent des souris intoxiquées non traitées.

Dans le test d'évitement passif (Figure 7), une amélioration significative des troubles cognitifs par la mémantine est observée uniquement pour la dose de 0,3 mg/Kg de mémantine. En outre, la mémantine aggrave les symptômes cognitifs des souris intoxiquées quand injectée à 10 mg/Kg. A l'inverse, la FENM est efficace à 0,1 ; 0,3 et 1 mg/Kg et n'exerce pas d'effet aggravant sur les symptômes amnésiques comme observé dans le cas du traitement par la mémantine (Figure 7).

Les effets des traitements symptomatiques par la mémantine et la FENM des atteintes mnésiques induites par les oligomères $A\beta_{25-35}$ ont également été comparés dans le test du hameau. Ce test évalue les processus mnésiques complexes des animaux incluant la mémoire topographique, l'orientation spatiale et l'apprentissage. La dose de composés test administrée aux souris pour ce test est de 0,3 mg/Kg. Pour le groupe traité à la FENM, les index de désorientation calculés pour les erreurs et la latence sont ramenés au niveau des souris non intoxiquées non traitées, et pas pour le groupe d'animaux traités à la mémantine (Figure 8).

La FENM est donc également plus efficace dans le traitement symptomatique des altérations mnésiques induites par la neurodégénérescence et peut être utilisée à des doses plus importantes pour lesquelles la mémantine est inefficace voire même délétère pour la mémoire. En outre la FENM permet le maintien de processus mnésiques complexes (test du hameau), ce que ne permet pas la mémantine.

**3. Conclusions**

Les données expérimentales montrent donc que la FENM, à la différence de la mémantine, est efficace pour induire une neuroprotection qui est détectée tant au niveau biochimique que cellulaire des événements sous-jacents à la mort des cellules du SNC et à la neuro dégénérescence. Cette neuroprotection aboutit à une prévention des atteintes cognitives chez les animaux. La neuroprotection conférée par le traitement à la FENM n'est pas observée pour la mémantine. En outre, la FENM est dépourvue des effets amnésiques de la mémantine quand elle est administrée à des doses importantes et elle reste efficace à ces doses dans le traitement symptomatique. Ces données montrent donc que la FENM est d'un intérêt particulier pour les traitements préventifs (de neuroprotection) et symptomatiques des pathologies neurodégénératives. Les données obtenues montrent un comportement de la FENM complètement atypique et non prévisible au regard des intervalles de doses trouvées efficaces et de effets biologiques obtenus pour la mémantine dans les modèles animaux.

**REFERENCES**

Arndt, J.W., Qian, F., Smith, B.A. et al. Structural and kinetic basis for the selectivity of aducanumab for aggregated forms of amyloid-β. Sci Rep 8, 6412 (2018).

Beaurain M., Salabert A.S., Ribeiro M.J., Arlicot N., Damier P., Le Jeune F., Demonet J.F., Payoux P. Innovative Molecular Imaging for Clinical Research, Therapeutic Stratification, and Nosography in Neuroscience. Frontiers in Medicine, 2019,6:268. Creeley C, Wozniak DF, Labruyere J, Taylor GT, Olney JW. Low doses of memantine disrupt memory in adult rats. J Neurosci. 2006;26(15):3923-3932. doi:10.1523/JNEUROSCI.4883-05.2006

Crouzier L, Gilabert D, Rosse! M, Trousse F, Maurice T. Topographical memory analyzed in mice using the Hamlet Test, a novel complex maze. Neurobiol Learn Mem. 2018;149:118-134.

Crouzier L, Maurice T. Assessment of topographie memory in mice in a complex environment using the Hamlet test. Current Protoc Mouse Biol. 2018;8:e43.

Gruden MA, Davidova TB, Malisauskas M, Sewell RD, Voskresenskaya NI, Wilhelm K, Elistratova EI, Sherstnev VV, Morozova-Roche LA Differential neuroimmune markers to the onset of Alzheimer's disease neurodegeneration and dementia: autoantibodies to Abeta((25-35)) oligomers, S100b and neurotransmitters. J Neuroimmunol. 2007 May;186(1-2):181-92. Epub 2007 May 2.

Knight R, Khondoker M, Magill N, Stewart R, Landau S: A Systematic Review and Meta-Analysis of the Effectiveness of Acetylcholinesterase Inhibitors and Memantine in Treating the Cognitive Symptoms of Dementia. Dement Geriatr Cogn Disord 2018;45:131-151. doi: 10.1159/000486546.

Liu CC, Liu CC, Kanekiyo T, Xu H, Bu G. Apolipoprotein E and Alzheimer disease: risk, mechanisms and therapy [published correction appears in Nat Rev Neurol. 2013. doi: 10.1038/nmeurol.2013.32. Liu, Chia-Chan [corrected to Liu, Chia-Chen]]. Nat Rev Neurol. 2013;9(2):106-118.

Maurice T, Hiramatsu M, Itoh J, Kameyama T, Hasegawa T, Nabeshima T. Behavioral evidence for a modulating rôle of sigma ligands in memory processes. I. Atténuation of dizocilpine (MK-801)-induced amnesia. Brain Res. 1994; 647: 44-56.

Maurice T, Lockhart BP, Privat A. Amnesia induced in mice by centrally administered β-amyloid peptides involves cholinergic dysfunction. Brain Res. 1996;706:181-93.

Maurice T, Mustafa MH, Desrumaux C, Keller E, Naert G, de la C. García-Barceló M, Rodríguez Cruz Y, Garcia Rodríguez JC. Intranasal formulation of erythropoietin (EPO) showed potent protective activity against amyloid toxicity in the Aβ25-35 nontransgenic mouse model of Alzheimer's disease. J Psychopharmacol. 2013;27;1044-57.

Maurice T, Volle JN, Strehaiano M, Crouzier L, Pereira C, Kaloyanov N, Virieux D, Pirat JL. Neuroprotection in non-transgenic and transgenic mouse models of Alzheimer's disease by positive modulation of σ1 receptors. Pharmacol Res. 2019;144:315-330.

Meunier J, Ieni J, Maurice T. The anti-amnesic and neuroprotective effects of donepezil against amyloid β25-35 peptide-induced toxicity in mice involve an interaction with the σ1 receptor. Br J Pharmacol. 2006;149: 998-1012.

Meunier J, Villard V, Givalois L, Maurice T. The γ-secretase inhibitor 2-[(1R)-1-[(4-chlorophenyl)sulfonyl] (2,5-difluorophenyl)amino]ethyl-5-fluorobenzenebutanoic acid (BMS-299897) alleviates Aβ1-42 seeding and short-term memory déficits in the AP25-35 mouse model of Alzheimer's disease. Eur J Pharmacol. 2013;698:193-9.

Paxinos G, Franklin KBJ. The Mouse Brain in Stereotaxic Coordinates. Academic Press, San Diego, CA, USA. 2004.

Pike CJ, Walencewicz AJ, Glabe CG, Cotman CW. In vitro aging of beta-amyloid protein causes peptide aggregation and neurotoxicity. Brain Res. 1991 Nov 1;563(1-2):311-4.

Rodríguez Cruz Y, Strehaiano M, Rodríguez Obaya T, García Rodríguez JC, Maurice T. An intranasal formulation of erythropoietin (Neuro-EPO) prevents memory déficits and amyloid toxicity in the APPSwe transgenic mouse model of Alzheimer's disease. J Alz Dis. 2017;55:231-248.

Stahl HP and Wermuth CG. Handbook of Pharmaceutical Salts: Properties, Sélection, and Use Edited by VHCA, Verlag Helvetica Chimica Acta, Zürich, Switzerland, and Wiley-VCH, Weinheim, Germany.

Villard V, Espallergues J, Keller E, Alkam T, Nitta A, Yamada K, Nabeshima T, Vamvakides A, Maurice T. Anti-amnesic and neuroprotective effects of the aminotetrahydrofuran derivative ANAVEX1-41 against amyloid ß25-35-induced toxicity in mice. Neuropsychopharmacology. 2009 34:1552-66.

Villard V, Espallergues J, Keller E, Vamvakides A, Maurice T. Anti-amnesic and neuroprotective potentials of the mixed muscarinic receptor/sigma1 (σ1) ligand ANAVEX2-73, a novel aminotetrahydrofuran derivative. J Psychopharmacol. 2011;25:1101-17.

## Revendications

**1.** Composé de formule (I) :

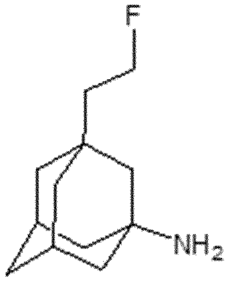

(I)

ou un de ses sels pharmaceutiquement acceptable pour son utilisation pour induire une neuroprotection, chez un sujet souffrant, étant suspecté de souffrir ou étant considéré à risque de souffrir d'une pathologie neurodégénérative.

2. Composé pour son utilisation selon la revendication 1, **caractérisé en ce que** le sel pharmaceutiquement acceptable correspond à la formule (II) :

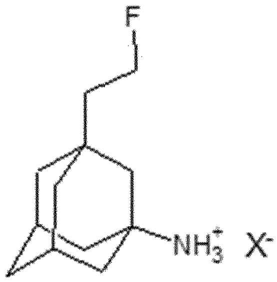

(II)

dans laquelle, $X^-$ désigne un contre anion choisi dans le groupe constitué par les ions chlorure, bromure, iodure, acétate, méthane sulfonate, benzène sulfonate, camphosulphonate, tartrate, dibenzoate, ascorbate, fumarate, citrate, phosphate, salicylate, oxalate, bromohydrate et tosylate.

3. Composé pour son utilisation selon la revendication 1 ou 2, où ladite neuroprotection inclut la prévention ou la diminution de la neuroinflammation.

4. Composé pour son utilisation selon l'une quelconque des revendications 1 à 3, où ladite neuroprotection inclut la prévention ou la diminution du stress oxydant dans les cellules neuronales.

5. Composé pour son utilisation selon l'une quelconque des revendications 1 à 4, où ladite neuroprotection inclut la prévention ou la diminution de l'apoptose des cellules neuronales chez ledit sujet.

6. Composé pour son utilisation selon l'une quelconque des revendications 1 à 5, où ladite neuroprotection inclut l'inhibition de la perte cellulaire de l'hippocampe chez ledit sujet.

7. Composé pour son utilisation selon l'une quelconque des revendications 1 à 6, où ladite neuroprotection inclut la prévention ou la diminution des altérations des capacités cognitives dudit sujet.

8. Composé pour son utilisation selon l'une quelconque des revendications 1 à 7, où ladite neuroprotection inclut la prévention ou la diminution des altérations de la mémoire à court terme dudit sujet.

9. Composé pour son utilisation selon l'une quelconque des revendications 1 à 8, où ladite neuroprotection inclut la prévention ou la diminution des altérations de la mémoire à moyen terme dudit sujet.

10. Composé pour son utilisation selon l'une quelconque des revendications 1 à 9, où ladite neuroprotection inclut la prévention ou la diminution des altérations de la mémoire spatiale dudit sujet.

11. Composé pour son utilisation selon l'une quelconque des revendications 1 à 10, où ladite neuroprotection inclut la prévention ou la diminution des altérations des capacités de reconnaissance et/ou d'apprentissage dudit sujet.

**12.** Composé pour son utilisation selon l'une quelconque des revendications 1 à 11, où ladite neuroprotection inclut la protection contre la toxicité des agrégats de β-amyloïde de ses fragments ou de ses oligomères, chez ledit sujet.

**13.** Composé pour son utilisation selon l'une quelconque des revendications 1 à 12, ledit sujet souffrant, étant suspecté de souffrir ou étant considéré à risque de souffrir d'une pathologie du système nerveux central sélectionnée parmi les tauopathies, synucléinopathies, les amyloïdopathies, la maladie d'Alzheimer, la maladie de Parkinson, l'atrophie multisystématisée, la maladie de Huntington, l'atrophie corticale postérieure, la maladie de Pick, l'épilepsie, la démence vasculaire, la démence fronto-temporale, la démence à corps de Lewy, la sclérose latérale amyotrophique, le syndrome de Korsakoff, le sevrage alcoolique, l'ischémie, l'ischémie néonatale, les traumatismes crânien, l'accident vasculaire cérébral, de préférence la maladie d'Alzheimer.

**14.** Combinaison comprenant un composé de formule (I) telle que définie dans la revendication 1 ou l'un de ses sels pharmaceutiquement acceptable ou de formule (II) telle que définie dans la revendication 2 et :

- au moins un inhibiteur de l'acétylcholinestérase, de préférence sélectionné parmi le donépézil, la rivastigmine, la galantamine, ou un de leurs sels pharmaceutiquement acceptables,
- au moins un agent inhibiteur des connexines, de préférence sélectionné parmi l'acide méclofénamique, enoxolone, la méfloquine et le 2-amino ethoxy diphényl borate (APB), ou un de leurs sels pharmaceutiquement acceptables,
- l'aducanumab ou un de ses fragments se liant à un antigène capable de lutter contre la toxicité de agrégats de β-amyloïde, de ses fragments ou de ses oligomères, ou
- au moins un modulateur positif de sigma 1 ou un de leurs sels pharmaceutiquement acceptables, de préférence sélectionné parmi 2-(2-Chlorophényl)-2-oxo-3,3,5,5-tétraméthyl-[1 ,4,2]-oxazaphosphinane ; 2-(4-Chlorophényl)-2-oxo-3,3,5,5-tétraméthyl-[1 ,4,2]-oxazaphosphinane ; 2-(3,5-Dichlorophényl)-2-oxo-3,3,5,5-tétraméthyl-[1 ,4,2]-oxazaphosphinane ; 2-(2,3-Dichlorophényl)-2-oxo-3,3,5,5-tétraméthyl-[1 ,4,2]-oxazaphosphinane ; 2-(3-Fluorophényl)-2-oxo-3,3,5,5-tétraméthyl-[1 ,4,2]-oxazaphosphinane ; 2-(4-Fluorophényl)-2-oxo-3,3,5,5-tétraméthyl-[1 ,4,2]-oxazaphosphinane ; 2-(3-Nitrophényl)-2-oxo-3,3,5,5-tétramethyl-[1 ,4,2]-oxazaphosphinane ; 2-(4 benzyloxycarbamoylphényl)-2-oxo-3,3,5,5-tétraméthyl-[1 ,4,2]-oxazaphosphinane ; 2-(Pyridin-2-yl)-2-oxo-3,3,5,5-tétraméthyl[1 ,4,2]-oxazaphosphinane ; 2-(Pyridin-3-yl)-2-oxo-3,3,5,5-tétraméthyl[1 ,4,2]-oxazaphosphinane ; 2-(Pyridin-4-yl)-2-oxo-3,3,5,5-tétraméthyl[1 ,4,2]-oxazaphosphinane ; 2-(Pyrimidin-2-yl)-2-oxo-3,3,5,5-tétraméthyl-[1 ,4,2]-oxazaphosphinane ; 2-(Pyrimidin-5-yl)-2-oxo-3,3,5,5-tétraméthyl-[1 ,4,2]-oxazaphosphinane ; 2-(3-aminophényl)-2-oxo-3,3,5,5-tétraméthyl-[1 ,4,2]-oxazaphosphinane ; 2-(4-aminophényl)-2-oxo-3,3,5,5-tétraméthyl-[1 ,4,2]-oxazaphosphinane ; 2-(3-chlorophényl)-N-méthyl-2-oxo-3,3,5,5-tétraméthyl-[1 ,4,2]-oxazaphosphinane ; 2-(3-chlorophényl)-2-thiono-3,3,5,5-tétraméthyl-[1 ,4,2]-oxazaphosphinane ; 2-(3-Chlorophényl)-2-oxo-3,3,5,5-tétraméthyl-[1 ,4,2]-oxazaphosphinane ; 2-morpholin-4-yléthyl 1-phénylcyclohexane-1-carboxylate) ; (+)-pentazocine ; 1,13-diméthyl-10-prop-2-enyl-10-azatricyclo[7.3.1.02,7]trideca-2(7),3,5-trien-4-ol ; 1-[2-(3,4-diméthoxyphényl)éthyl]-4-(3-phénylpropyl)pipérazine ; 1-(2,2-Diphényltétrahydro-3-furanyl)-N,N-diméthylméthanamine ; 2-{[(E)-{5-Méthoxy-1-[4-(trifluorométhyl)phényl]pentylidène}amino]oxy}éthanamine ; N-(1-benzylpipéridin-4-yl)-4-iodobenzamide ; (5E)-N-(Cyclopropylméthyl)-N-méthyl-3,6-diphényl-5-hexén-3-amine ; 1-{3-[4-(3-Chlorophényl)-1-pipérazinyl]propyl}-5-méthoxy-3,4-dihydro-2(1H)-quinoléinone ; (1S,2R)-N-[2-(3,4-Dichlorophényl)éthyl]-N-méthyl-2-(1-pyrrolidinyl)cyclohexanamine) ; 6-[(4-benzylpipérazin-1-yl)méthyl]-2,3-diméthoxyphénol) ; 4-(3-(méthylsulfonyl)phényl)-1-propylpipéridine ou un de leurs sels pharmaceutiquement acceptables, de préférence le 2-(3-Chlorophényl)-2-oxo-3,3,5,5-tétraméthyl-[1 ,4,2]-oxazaphosphinane ou le 2-morpholin-4-yléthyl 1-phénylcyclohexane-1-carboxylate ou un de ses sels pharmaceutiquement acceptable,

pour son utilisation pour induire une neuroprotection chez un sujet souffrant, étant suspecté de souffrir ou étant considéré à risque de souffrir d'une pathologie neurodégénérative.

**15.** Combinaison pour son utilisation selon la revendication 14, ledit sujet souffrant, étant suspecté de souffrir ou à risque de souffrir de la maladie d'Alzheimer.

**16.** Composé pour son utilisation selon l'une quelconque des revendications 1 à 13, ladite neuroprotection comprenant le traitement symptomatique des troubles cognitifs chez ledit sujet, ledit composé étant administré oralement à une

dose supérieure à 20 mg par jour, de préférence supérieure ou égale à 30 mg par jour.

**17.** Composé pour son utilisation selon la revendication 16, en combinaison avec:

- au moins un inhibiteur de l'acétylcholinestérase, de préférence sélectionné parmi le donépézil, la rivastigmine, la galantamine, ou un de leurs sels pharmaceutiquement acceptables,
- au moins un agent inhibiteur des connexines, de préférence sélectionné l'acide méclofénamique, enoxolone, la méfloquine et le 2-amino ethoxy diphényl borate (APB), ou un de leurs sels pharmaceutiquement acceptables,
- l'aducanumab ou un de ses fragments se liant à un antigène capable de lutter contre la toxicité de agrégats de β-amyloïde, de ses fragments ou de ses oligomères, ou
- au moins un modulateur positif de sigma 1 ou un de leurs sels pharmaceutiquement acceptables, de préférence sélectionné parmi les composés suivant : 2-(2-Chlorophényl)-2-oxo-3,3,5,5-tétraméthyl-[1 ,4,2]-oxazaphosphinane ; 2-(4-Chlorophényl)-2-oxo-3,3,5,5-tétraméthyl-[1 ,4,2]-oxazaphosphinane ; 2-(3,5-Dichlorophényl)-2-oxo-3,3,5,5-tétraméthyl-[1 ,4,2]-oxazaphosphinane ; 2-(2,3-Dichlorophényl)-2-oxo-3,3,5,5-tétraméthyl-[1 ,4,2]-oxazaphosphinane ; 2-(3-Fluorophényl)-2-oxo-3,3,5,5-tétraméthyl-[1 ,4,2]-oxazaphosphinane ; 2-(4-Fluorophényl)-2-oxo-3,3,5,5-tétraméthyl-[1 ,4,2]-oxazaphosphinane ; 2-(3-Nitrophényl)-2-oxo-3,3,5,5-tétramethyl-[1 ,4,2]-oxazaphosphinane ; 2-(4 benzyloxycarbamoylphényl)-2-oxo-3,3,5,5-tétraméthyl-[1 ,4,2]-oxazaphosphinane ; 2-(Pyridin-2-yl)-2-oxo-3,3,5,5-tétraméthyl[1 ,4,2]-oxazaphosphinane ; 2-(Pyridin-3-yl)-2-oxo-3,3,5,5-tétraméthyl[1 ,4,2]-oxazaphosphinane ; 2-(Pyridin-4-yl)-2-oxo-3,3,5,5-tétraméthyl[1 ,4,2]-oxazaphosphinane ; 2-(Pyrimidin-2-yl)-2-oxo-3,3,5,5-tétraméthyl-[1 ,4,2]-oxazaphosphinane ; 2-(Pyrimidin-5-yl)-2-oxo-3,3,5,5-tétraméthyl-[1 ,4,2]-oxazaphosphinane ; 2-(3-aminophényl)-2-oxo-3,3,5,5-tétraméthyl-[1 ,4,2]-oxazaphosphinane ; 2-(4-aminophényl)-2-oxo-3,3,5,5-tétraméthyl-[1 ,4,2]-oxazaphosphinane ; 2-(3-chlorophényl)-N-méthyl-2-oxo-3,3,5,5-tétraméthyl-[1 ,4,2]-oxazaphosphinane ; 2-(3-chlorophényl)-2-thiono-3,3,5,5-tétraméthyl-[1 ,4,2]-oxazaphosphinane ; 2-(3-Chlorophényl)-2-oxo-3,3,5,5-tétraméthyl-[1 ,4,2]-oxazaphosphinane ; 2-morpholin-4-yléthyl 1-phénylcyclohexane-1-carboxylate) ; (+)-pentazocine ; 1,13-diméthyl-10-prop-2-enyl-10-azatricyclo[7.3.1.02,7]trideca-2(7),3,5-trien-4-ol ; 1-[2-(3,4-diméthoxyphényl)éthyl]-4-(3-phénylpropyl)pipérazine ; 1-(2,2-Diphényltétrahydro-3-furanyl)-N,N-diméthylméthanamine ; 2-{[(E)-{5-Méthoxy-1-[4-(trifluorométhyl)phényl]pentylidène}amino]oxy}éthanamine ; N-(1-benzylpipéridin-4-yl)-4-iodobenzamide ; (5E)-N-(Cyclopropylméthyl)-N-méthyl-3,6-diphényl-5-hexén-3-amine ; 1-{3-[4-(3-Chlorophényl)-1-pipérazinyl]propyl}-5-méthoxy-3,4-dihydro-2(1H)-quinoléinone ; (1S,2R)-N-[2-(3,4-Dichlorophényl)éthyl]-N-méthyl-2-(1 - pyrrolidinyl)cyclohexanamine) ; 6-[(4-benzylpipérazin-1-yl)méthyl]-2,3-diméthoxyphénol) ; 4-(3-(méthylsulfonyl)phényl)-1-propylpipéridine ou un de leurs sels pharmaceutiquement acceptable, de préférence le 2-(3-Chlorophényl)-2-oxo-3,3,5,5-tétraméthyl-[1 ,4,2]-oxazaphosphinane ou le 2-morpholin-4-yléthyl 1-phénylcyclohexane-1-carboxylate ou l'un de ses sels pharmaceutiquement acceptable.

**18.** Composé de formule (I) :

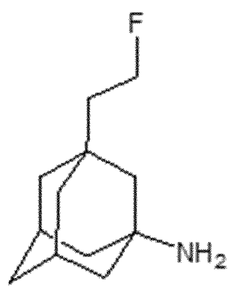

(I)

ou un de ses sels pharmaceutiquement acceptable pour son utilisation dans la prévention ou le traitement d'une pathologie neurodégénérative.

**19.** Composé de formule (I) :

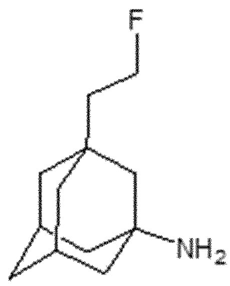

(I)

ou un de ses sels pharmaceutiquement acceptable pour son utilisation dans la prévention ou la diminution des altérations des capacités cognitives chez un sujet souffrant, étant suspecté de souffrir ou étant considéré à risque de souffrir d'une pathologie neurodégénérative.

## Patentansprüche

1. Verbindung der Formel (1):

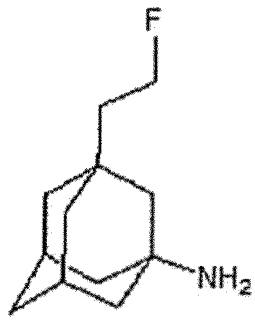

(I)

oder ein pharmazeutisch annehmbares Salz davon zur Verwendung bei der Induzierung eines Neuroschutzes bei einem Patienten, der an einer neurodegenerativen Erkrankung leidet, vermutlich an solch einer leidet oder dafür als gefährdet gilt.

2. Verbindung zur Verwendung nach Anspruch 1, **dadurch gekennzeichnet, dass** das pharmazeutisch annehmbare Salz der Formel (II) entspricht:

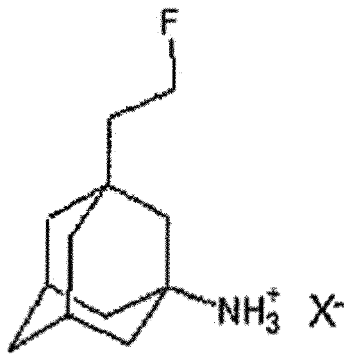

(II)

wobei $X^-$ ein Gegenanion bezeichnet, ausgewählt aus der Gruppe bestehend aus den Ionen Chlorid, Bromid, Iodid, Acetat, Methansulfonat, Benzolsulfonat, Camphosulfonat, Tartrat, Dibenzoat, Ascorbat, Fumarat, Citrat, Phosphat, Salicylat, Oxalat, Hydrobromid und Tosylat.

3. Verbindung zur Verwendung nach Anspruch 1 oder 2, wobei der Neuroschutz die Vorbeugung oder Reduzierung

der Neuroinflammation umfasst.

4. Verbindung zur Verwendung nach einem der Ansprüche 1 bis 3, wobei der Neuroschutz die Vorbeugung oder Reduzierung von oxidativem Stress in den neuronalen Zellen umfasst.

5. Verbindung zur Verwendung nach einem der Ansprüche 1 bis 4, wobei der Neuroschutz die Vorbeugung oder Reduzierung der Apoptose neuronaler Zellen bei dem Patienten umfasst.

6. Verbindung zur Verwendung nach einem der Ansprüche 1 bis 5, wobei der Neuroschutz die Hemmung des Zellverlusts im Hippocampus bei dem Patienten umfasst.

7. Verbindung zur Verwendung nach einem der Ansprüche 1 bis 6, wobei der Neuroschutz die Vorbeugung oder Reduzierung von Veränderungen der kognitiven Fähigkeiten des Patienten umfasst.

8. Verbindung zur Verwendung nach einem der Ansprüche 1 bis 7, wobei der Neuroschutz die Vorbeugung oder Reduzierung von Veränderungen des Kurzzeitgedächtnisses des Patienten umfasst.

9. Verbindung zur Verwendung nach einem der Ansprüche 1 bis 8, wobei der Neuroschutz die Vorbeugung oder Reduzierung von Veränderungen im Mittelzeitgedächtnis des Patienten umfasst.

10. Verbindung zur Verwendung nach einem der Ansprüche 1 bis 9, wobei der Neuroschutz die Vorbeugung oder Reduzierung von Veränderungen des räumlichen Gedächtnisses des Patienten umfasst.

11. Verbindung zur Verwendung nach einem der Ansprüche 1 bis 10, wobei der Neuroschutz die Vorbeugung oder Reduzierung von Veränderungen der Erkennungs- und/oder Lernfähigkeiten des Patienten umfasst.

12. Verbindung zur Verwendung nach einem der Ansprüche 1 bis 11, wobei der Neuroschutz den Schutz vor der Toxizität von β-Amyloid-Aggregaten, Fragmenten oder Oligomeren davon bei dem Patienten umfasst.

13. Verbindung zur Verwendung nach einem der Ansprüche 1 bis 12, wobei der Patient an einer Erkrankung des zentralen Nervensystems leidet, vermutlich leidet oder als gefährdet gilt, an einer solchen zu leiden, die ausgewählt ist aus Tauopathien, Synukleinopathien, Amyloidopathien, Alzheimer-Krankheit, Parkinson-Krankheit, Multisystematrophie, Huntington-Krankheit, posteriore kortikale Atrophie , Pick-Krankheit, Epilepsie, vaskulärer Demenz, frontotemporaler Demenz, Lewy-Körperchen-Demenz, amyotropher Lateralsklerose, Korsakow-Syndrom, Alkoholentzug, Ischämie, neonataler Ischämie, Schädel-Hirn-Trauma, Schlaganfall, vorzugsweise der Alzheimer-Krankheit.

14. Kombination umfassend eine Verbindung der Formel (I) wie in Anspruch 1 definiert oder ein pharmazeutisch annehmbares Salz davon oder der Formel (II) wie in Anspruch 2 definiert und:

- mindestens einen Acetylcholinesterase-Hemmer, vorzugsweise ausgewählt aus Donepezil, Rivastigmin, Galantamin oder einem pharmazeutisch annehmbaren Salz davon,
- mindestens ein Connexin-hemmendes Mittel, vorzugsweise ausgewählt aus Meclofenaminsäure, Enoxolon, Mefloquin und 2-Aminoethoxydiphenylborat (APB) oder einem pharmazeutisch annehmbaren Salz davon,
- Aducanumab oder eines seiner Fragmente, die an ein Antigen binden, das die Toxizität von β-Amyloid-Aggregaten, Fragmenten oder Oligomeren davon bekämpfen kann, oder
- mindestens einen positiven Modulator von Sigma 1 oder ein pharmazeutisch annehmbares Salz davon, vorzugsweise ausgewählt aus 2-(2-Chlorphenyl)-2-oxo-3,3,5,5-tetramethyl-[1,4,2]-oxazaphosphinan, 2-(4-Chlorphenyl)-2-oxo-3,3,5,5-tetramethyl-[1,4,2]-oxazaphosphinan; 2-(3,5-Dichlorphenyl)-2-oxo-3,3,5,5-tetramethyl-[1,4,2]-oxazaphospinan; 2-(2,3-Dichlorphenyl)-2-oxo-3,3,5,5-tetramethyl-[1,4,2]-oxazaphospinan; 2-(3-Fluorphenyl)-2-oxo-3,3,5,5-tetramethyl-[1,4,2]-oxazaphospinan; 2-(4-Fluorphenyl)-2-oxo-3,3,5,5-tetramethyl-[1,4,2]-oxazaphospinan; 2-(3-Nitrophenyl)-2-oxo-3,3,5,5-tetramethyl-[1,4,2]-oxazaphospinan; 2-(4-Benzyloxycarbamoylphenyl)-2-oxo-3,3,5,5-tetramethyl-[1,4,2]-oxazaphospinan; 2-(Pyridin-2-yl)-2-oxo-3,3,5,5-tetramethyl-[1,4,2]-oxazaphospinan; 2-(Pyridin-3-yl)-2-oxo-3,3,5,5-tetramethyl-[1,4,2]-oxazaphospinan; 2-(Pyridin-4-yl)-2-oxo-3,3,5,5-tetramethyl-[1,4,2]-oxazaphospinan; 2-(Pyrimidin-2-yl)-2-oxo-3,3,5,5-tetramethyl-[1,4,2]-oxazaphospinan; 2-(Pyrimidin-5-yl)-2-oxo-3,3,5,5-tetramethyl-[1,4,2]-oxazaphospinan; 2-(3-Aminophenyl)-2-oxo-3,3,5,5-tetramethyl-[1,4,2]-oxazaphospinan; 2-(4-Aminophenyl)-2-oxo-3,3,5,5-tetramethyl-[1,4,2]-oxazaphospinan; 2-(3-Chlorphenyl)-N-methyl-2-oxo-3,3,5,5-tetramethyl-[1,4,2]-oxazaphospinan; 2-(3-Chlorphenyl)-2-thiono-3,3,5,5-tetramethyl-[1,4,2]-oxazaphospinan; 2-(3-Chlorphenyl)-2-oxo-3,3,5,5-tetramethyl-[1,4,2]-oxazaphospinan; 2-Morpholin-4-ylethyl-1-phenylcyclohexan-1-carboxylat); (+)-Pentazocin; 1,13-Dimethyl-10-prop-2-enyl-10-azatricyclo[7.3.1.02,7]trideca-2(7),3,5-trien-4-ol; 1-[2-(3,4-Dimethoxyphenyl)ethyl]-4-(3-phenylpropyl)piperazin; 1-(2,2-Diphenyltetrahydro-3-furanyl)-N,N-dimethylmethanamin; 2-{[(E)-{5-Methoxy-1-[4-(trifluormethyl)phenyl]pentyliden}amino]oxy}ethanamin; N-(1-Benzylpiperidin-4-yl)-4-iodobenzamid; (5E)-N-(Cyclopropylmethyl)-N-methyl-3,6-diphenyl-5-hexen-3-amin; 1-{3-[4-(3-Chlorphenyl)-1-piperazinyl]propyl}-5-methoxy-3,4-dihydro-2(1H)-chinolin; (1S,2R)-N-[2-(3,4-Dichlorphenyl)ethyl]-N-methyl-2-(1-pyrrolidinyl)cyclohexanamin); 6-[(4-Benzylpiperazin-1-yl)methyl]-2,3-dimethoxyphenol); 4-(3-(Methylsulfonyl)phenyl)-1-propylpiperidin oder ein pharmazeutisch annehmbares Salz davon, vorzugsweise 2-(3-Chlorphenyl)-2-oxo-3,3,5,5-tetramethyl-[1,4,2]-
- oxazaphosphinan oder 2-Morpholin-4-ylethyl 1-phenylcyclohexan-1-carboxylat oder ein pharmazeutisch annehmbares Salz davon,

zur Verwendung bei der Induzierung eines Neuroschutzes bei einem Patienten, der an einer neurodegenerativen Erkrankung leidet, vermutlich an solch einer leidet oder als gefährdet dafür gilt.

**15.** Kombination zur Verwendung nach Anspruch 14, wobei der Patient an der Alzheimer-Krankheit leidet, vermutlich an dieser leidet oder als gefährdet dafür gilt.

**16.** Verbindung zur Verwendung nach einem der Ansprüche 1 bis 13, wobei der Neuroschutz die symptomatische Behandlung kognitiver Störungen bei dem Patienten umfasst, wobei die Verbindung oral in einer Dosis von mehr als 20 mg pro Tag, vorzugsweise mehr als oder gleich 30 mg pro Tag, verabreicht wird.

**17.** Verbindung zur Verwendung nach Anspruch 16, in Kombination mit:

- mindestens einem Acetylcholinesterase-Hemmer, vorzugsweise ausgewählt aus Donepezil, Rivastigmin, Galantamin oder einem pharmazeutisch annehmbaren Salz davon,
- mindestens ein Connexin-hemmendes Mittel, vorzugsweise ausgewählt aus Meclofenaminsäure, Enoxolon, Mefloquin und 2-Aminoethoxydiphenylborat (APB) oder einem pharmazeutisch annehmbaren Salz davon,
- Aducanumab oder eines seiner Fragmente, die an ein Antigen binden, das die Toxizität von β-Amyloid-Aggregaten, Fragmenten oder Oligomeren davon bekämpfen kann, oder
- mindestens einen positiven Modulator von Sigma 1 oder ein pharmazeutisch annehmbares Salz davon, vorzugsweise ausgewählt aus den folgenden Verbindungen:

2-(2-Chlorphenyl)-2-oxo-3,3,5,5-tetramethyl-[1,4,2]-oxazaphosphinan, 2-(4-Chlorphenyl)-2-oxo-3,3,5,5-tetramethyl-[1,4,2]-oxazaphosphinan; 2-(3,5-Dichlorphenyl)-2-oxo-3,3,5,5-tetramethyl-[1,4,2]-oxazaphospinan; 2-(2,3-Dichlorphenyl)-2-oxo-3,3,5,5-tetramethyl-[1,4,2]-oxazaphospinan; 2-(3-Fluorphenyl)-2-oxo-3,3,5,5-tetramethyl-[1,4,2]-oxazaphospinan; 2-(4-Fluorphenyl)-2-oxo-3,3,5,5-tetramethyl-[1,4,2]-oxazaphospinan; 2-(3-Nitrophenyl)-2-oxo-3,3,5,5-tetramethyl-[1,4,2]-oxazaphospinan; 2-(4-Benzyloxycarbamoylphenyl)-2-oxo-3,3,5,5-tetramethyl-[1,4,2]-oxazaphospinan; 2-(Pyridin-2-yl)-2-oxo-3,3,5,5-tetramethyl-[1,4,2]-oxazaphospinan; 2-(Pyridin-3-yl)-2-oxo-3,3,5,5-tetramethyl-[1,4,2]-oxazaphospinan; 2-(Pyridin-4-yl)-2-oxo-3,3,5,5-tetramethyl-[1,4,2]-oxazaphospinan; 2-(Pyrimidin-2-yl)-2-oxo-3,3,5,5-tetramethyl-[1,4,2]-oxazaphospinan; 2-(Pyrimidin-5-yl)-2-oxo-3,3,5,5-tetramethyl-[1,4,2]-oxazaphospinan; 2-(3-Aminophenyl)-2-oxo-3,3,5,5-tetramethyl-[1,4,2]-oxazaphospinan; 2-(4-Aminophenyl)-2-oxo-3,3,5,5-tetramethyl-[1,4,2]-oxazaphospinan; 2-(3-Chlorphenyl)-N-methyl-2-oxo-3,3,5,5-tetramethyl-[1,4,2]-oxazaphospinan;
2-(3-Chlorphenyl)-2-thiono-3,3,5,5-tetramethyl-[1,4,2]-oxazaphospinan; 2-(3-Chlorphenyl)-2-oxo-3,3,5,5-tetramethyl-[1,4,2]-oxazaphospinan; 2-Morpholin-4-ylethyl-1-phenylcyclohexan-1-carboxylat); (+)-Pentazocin;
1,13-Dimethyl-10-prop-2-enyl-10-azatricyclo[7.3.1.02,7]trideca-2(7),3,5-trien-4-ol; 1-[2-(3,4-Dimethoxyphenyl)ethyl]-4-(3-phenylpropyl)piperazin;
1-(2,2-Diphenyltetrahydro-3-furanyl)-N,N-dimethylmethanamin; 2-{[(E)-{5-Methoxy-1-[4-(trifluormethyl)phenyl]pentyliden}amino]oxy}ethanamin; N-(1-Benzylpiperidin-4-yl)-4-iodobenzamid; (5E)-N-(Cyclopropylmethyl)-N-methyl-3,6-diphenyl-5-hexen-3-amin;
1-{3-[4-(3-Chlorphenyl)-1-piperazinyl]propyl}-5-methoxy-3,4-dihydro-2(1H)-chinolin; (1S,2R)-N-[2-(3,4-Dichlorphenyl)ethyl]-N-methyl-2-(1-pyrrolidinyl)cyclohexanamin); 6-[(4-Benzylpiperazin-1-yl)methyl]-2,3-dimethoxyphenol); 4-(3-(Methylsulfonyl)phenyl)-1-propylpiperidin oder ein pharmazeutisch annehmbares Salz davon, vorzugsweise 2-(3-Chlorphenyl)-2-oxo-3,3,5,5-tetramethyl-[1,4,2]

- oxazaphosphinan oder 2-Morpholin-4-ylethyl 1-phenylcyclohexan-1-carboxylat oder ein pharmazeutisch annehmbares Salz davon.

**18.** Verbindung der Formel (I):

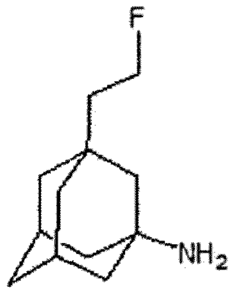

(I)

oder ein pharmazeutisch annehmbares Salz davon zur Verwendung bei der Vorbeugung oder Behandlung einer neurodegenerativen Erkrankung.

**19.** Verbindung der Formel (I):

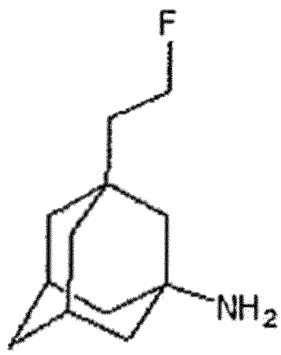

(I)

oder ein pharmazeutisch annehmbares Salz davon zur Verwendung bei der Vorbeugung oder Reduzierung von Veränderungen der kognitiven Fähigkeiten bei einem Patienten, der an einer neurodegenerativen Erkrankung leidet, vermutlich an solch einer leidet oder dafür als gefährdet gilt.

## Claims

**1.** Compound of formula (I):

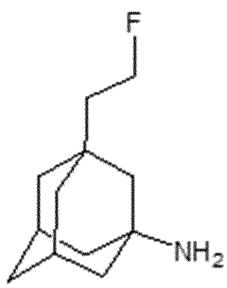

(I)

or a pharmaceutically acceptable salt thereof for use in inducing neuroprotection, in a subject suffering from, suspected of suffering from or considered to be at risk of suffering from a neurodegenerative pathology.

**2.** Compound for use according to claim 1, **characterised in that** the pharmaceutically acceptable salt corresponds

to the formula (II):

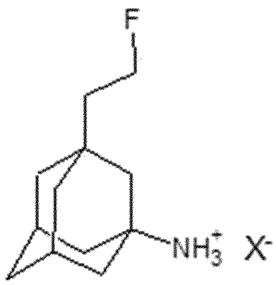

(II)

wherein $X^-$ denotes a counteranion selected from the group consisting of chloride, bromide, iodide, acetate, methane sulphonate, benzene sulphonate, camphosulphonate, tartrate, dibenzoate, ascorbate, fumarate, citrate, phosphate, salicylate, oxalate, bromohydrate, and tosylate ions.

**3.** Compound for use according to claim 1 or 2, wherein said neuroprotection includes the prevention or reduction of neuroinflammation.

**4.** Compound for use according to any of claims 1 to 3, wherein said neuroprotection includes the prevention or reduction of oxidative stress in neuronal cells.

**5.** Compound for use according to any of claims 1 to 4, wherein said neuroprotection includes the prevention or reduction of neuronal apoptosis in said subject.

**6.** Compound for use according to any of claims 1 to 5, wherein said neuroprotection includes the inhibition of hippocampal cell loss in said subject.

**7.** Compound for use according to any of claims 1 to 6, wherein said neuroprotection includes the prevention or reduction of impairments to said subject's cognitive abilities.

**8.** Compound for use according to any of claims 1 to 7, wherein said neuroprotection includes the prevention or reduction of impairments to said subject's short-term memory.

**9.** Compound for use according to any of claims 1 to 8, wherein said neuroprotection includes the prevention or reduction of impairments to said subject's intermediate-term memory.

**10.** Compound for use according to any of claims 1 to 9, wherein said neuroprotection includes the prevention or reduction of impairments to said subject's spatial memory.

**11.** Compound for use according to any of claims 1 to 10, wherein said neuroprotection includes the prevention or reduction of impairments to said subject's recognition and/or learning abilities.

**12.** Compound for use according to any of claims 1 to 11, wherein said neuroprotection includes protection against the toxicity of β-amyloid aggregates, of its fragments or of its oligomers, in said subject.

**13.** Compound for use according to any of claims 1 to 12, said subject suffering from, being suspected of suffering from, or being considered to be at risk of suffering from a pathology of the central nervous system selected from tauopathies, synucleinopathies, amyloidopathies, Alzheimer's disease, Parkinson's disease, multiple system atrophy, Huntington's disease, posterior cortical atrophy, Pick's disease, epilepsy, vascular dementia, frontotemporal dementia, dementia with Lewy bodies, amyotrophic lateral sclerosis, Korsakoff's syndrome, alcohol withdrawal, ischaemia, neonatal ischaemia, head injury, or stroke, preferably Alzheimer's disease.

**14.** Combination comprising a compound of formula (I) as defined in claim 1 or a pharmaceutically acceptable salt thereof or of formula (II) as defined I claim 2 and:

- at least one acetylcholinesterase inhibitor, preferably selected from donepezil, rivastigmine, galantamine, or

a pharmaceutically acceptable salt thereof,
- at least one connexin inhibitor, preferably selected from meclofenamic acid, enoxolone, mefloquine and 2-amino ethoxy diphenyl borate (APB), or a pharmaceutically acceptable salt thereof,
- aducanumab or an antigen-binding fragment thereof capable of fighting against the toxicity of β-amyloid aggregates, its fragments or its oligomers, or
- at least one positive modulator of sigma-1 receptors or a pharmaceutically acceptable salt thereof, preferably selected from 2-(2-Chlorophenyl)-2-oxo-3,3,5,5-tetramethyl-[1 ,4,2]-oxazaphosphinane; 2-(4-Chlorophenyl)-2-oxo-3,3,5,5-tetramethyl-[1 ,4,2]-oxazaphosphinane; 2-(3,5-Dichlorophenyl)-2-oxo-3,3,5,5-tetramethyl-[1 ,4,2]-oxazaphosphinane; 2-(2,3-Dichlorophenyl)-2-oxo-3,3,5,5-tetramethyl-[1 ,4,2]-oxazaphosphinane; 2-(3-Fluorophenyl)-2-oxo-3,3,5,5-tetramethyl-[1 ,4,2]-oxazaphosphinane; 2-(4-Fluorophenyl)-2-oxo-3,3,5,5-tetramethyl-[1 ,4,2]-oxazaphosphinane; 2-(3-Nitrophenyl)-2-oxo-3,3,5,5-tetramethyl-[1 ,4,2]-oxazaphosphinane; 2-(4-benzyloxycarbamoylphenyl)-2-oxo-3,3,5,5-tetramethyl-[1 ,4,2]-oxazaphosphinane; 2-(Pyridin-2-yl)-2-oxo-3,3,5,5-tetramethyl-[1 ,4,2]-oxazaphosphinane; 2-(Pyridin-3-yl)-2-oxo-3,3,5,5-tetramethyl[1 ,4,2]-oxazaphosphinane; 2-(Pyridin-4-yl)-2-oxo-3,3,5,5-tetramethyl[1 ,4,2]-oxazaphosphinane; 2-(Pyrimidin-2-yl)-2-oxo-3,3,5,5-tetramethyl-[1 ,4,2]-oxazaphosphinane; 2-(Pyrimidin-5-yl)-2-oxo-3,3,5,5-tetramethyl-[1 ,4,2]-oxazaphosphinane; 2-(3-aminophenyl)-2-oxo-3,3,5,5-tetramethyl-[1 ,4,2]-oxazaphosphinane; 2-(4-aminophenyl)-2-oxo-3,3,5,5-tetramethyl-[1 ,4,2]-oxazaphosphinane; 2-(3-chlorophenyl)-N-methyl-2-oxo-3,3,5,5-tetramethyl-[1 ,4,2]-oxazaphosphinane; 2-(3-chlorophenyl)-2-thiono-3,3,5,5-tetramethyl-[1 ,4,2]-oxazaphosphinane; 2-(3-Chlorophenyl)-2-oxo-3,3,5,5-tetramethyl-[1 ,4,2]-oxazaphosphinane; 2-morpholin-4-ylethyl 1-phenylcyclohexane-1-carboxylate); (+)-pentazocine; 1,13-dimethyl-10-prop-2-enyl-10-azatricyclo[7.3.1.02,7]trideca-2(7),3,5-trien-4-ol; 1-[2-(3,4-dimethoxyphenyl)ethyl]-4-(3-phenylpropyl)piperazine; 1-(2,2-Diphenyltetrahydro-3-furanyl)-N,N-dimethylmethanamine; 2-{[(E)-{5-Methoxy-1-[4-(trifluoromethyl)phenyl]pentylidene]amino]oxy}ethanamine; N-(1-benzylpiperidin-4-yl)-4-iodobenzamide; (5E)-N-(Cyclopropylmethyl)-N-methyl-3,6-diphenyl-5-hexen-3-amine; 1-{3-[4-(3-Chlorophenyl)-1-piperazinyl]propyl}-5-methoxy-3,4-dihydro-2(1H)-quinolinone; (1S,2R)-N-[2-(3,4-Dichlorophenyl)ethyl]-N-methyl-2-(1-pyrrolidinyl)cyclohexanamine); 6-[(4-benzylpiperazin-1-yl)methyl]-2,3-dimethoxyphenol); 4-(3-(methylsulfonyl)phenyl)-1-propylpiperidine or a pharmaceutically acceptable salt thereof, preferably 2-(3-Chlorophenyl)-2-oxo-3,3,5,5-tetramethyl-[1 ,4,2]-oxazaphosphinane or 2-morpholin-4-ylethyl 1-phenylcyclohexane-1-carboxylate or a pharmaceutically acceptable salt thereof, for use in inducing neuroprotection in a subject suffering from, suspected of suffering from or considered to be at risk of suffering from a neurodegenerative pathology.

**15.** Combination for use according to claim 14, said subject suffering from, being suspected of suffering from, or being at risk of suffering from Alzheimer's disease.

**16.** Compound for use according to any of claims 1 to 13, said neuroprotection comprising the symptomatic treatment of cognitive disorders in said subject, said compound being administered orally at a dose greater than 20 mg per day, preferably greater than or equal to 30 mg per day.

**17.** Compound for use according to claim 16, in combination with:

- at least one acetylcholinesterase inhibitor, preferably selected from donepezil, rivastigmine, galantamine, or a pharmaceutically acceptable salt thereof,
- at least one connexin inhibitor, preferably selected from meclofenamic acid, enoxolone, mefloquine and 2-amino ethoxy diphenyl borate (APB), or a pharmaceutically acceptable salt thereof,
- aducanumab or an antigen-binding fragment thereof capable of fighting against the toxicity of β-amyloid aggregates, its fragments or its oligomers, or
- at least one positive modulator of sigma-1 receptors or a pharmaceutically acceptable salt thereof, preferably selected from the following: 2-(2-Chlorophenyl)-2-oxo-3,3,5,5-tetramethyl-[1 ,4,2]-oxazaphosphinane; 2-(4-Chlorophenyl)-2-oxo-3,3,5,5-tetramethyl-[1 ,4,2]-oxazaphosphinane; 2-(3,5-Dichlorophenyl)-2-oxo-3,3,5,5-tetramethyl-[1 ,4,2]-oxazaphosphinane; 2-(2,3-Dichlorophenyl)-2-oxo-3,3,5,5-tetramethyl-[1 ,4,2]-oxazaphosphinane; 2-(3-Fluorophenyl)-2-oxo-3,3,5,5-tetramethyl-[1 ,4,2]-oxazaphosphinane; 2-(4-Fluorophenyl)-2-oxo-3,3,5,5-tetramethyl-[1 ,4,2]-oxazaphosphinane; 2-(3-Nitrophenyl)-2-oxo-3,3,5,5-tetramethyl-[1 ,4,2]-oxazaphosphinane; 2-(4-benzyloxycarbamoylphenyl)-2-oxo-3,3,5,5-tetramethyl-[1 ,4,2]-oxazaphosphinane; 2-(Pyridin-2-yl)-2-oxo-3,3,5,5-tetramethyl-[1 ,4,2]-oxazaphosphinane; 2-(Pyridin-3-yl)-2-oxo-3,3,5,5-tetramethyl[1 ,4,2]-oxazaphosphinane; 2-(Pyridin-4-yl)-2-oxo-3,3,5,5-tetramethyl[1 ,4,2]-oxazaphosphinane; 2-(Pyrimidin-2-yl)-2-oxo-3,3,5,5-tetramethyl-[1 ,4,2]-oxazaphosphinane; 2-(Pyrimidin-5-yl)-2-oxo-3,3,5,5-tetramethyl-[1 ,4,2]-oxazaphosphinane; 2-(3-aminophenyl)-2-oxo-3,3,5,5-tetramethyl-[1 ,4,2]-oxazaphosphinane; 2-(4-aminophenyl)-2-oxo-3,3,5,5-tetramethyl-[1 ,4,2]-oxazaphosphinane; 2-(3-chlorophenyl)-N-methyl-2-oxo-3,3,5,5-tetramethyl-[1 ,4,2]-oxazaphosphinane; 2-(3-chlorophenyl)-2-thiono-3,3,5,5-tetramethyl-[1 ,4,2]-oxazaphosphinane; 2-(3-Chlorophenyl)-2-oxo-3,3,5,5-tetramethyl-[1 ,4,2]-oxazaphosphinane; 2-morpholin-4-ylethyl 1-phenylcyclohexane-1-carboxylate); (+)-pentazocine; 1,13-dimethyl-10-prop-2-enyl-10-azatricyclo[7.3.1.02,7]trideca-2(7),3,5-trien-4-ol; 1-[2-(3,4-dimethoxyphenyl)ethyl]-4-(3-phenylpropyl)piperazine; 1-(2,2-Diphenyltetrahydro-3-furanyl)-N,N-dimethylmethanamine; 2-{[(E)-{5-Methoxy-1-[4-(trifluoromethyl)phenyl]pentylidene]amino]oxy}ethanamine; N-(1-benzylpiperidin-4-yl)-4-iodobenzamide; (5E)-N-(Cyclopropylmethyl)-N-methyl-3,6-diphenyl-5-hexen-3-amine; 1-{3-[4-(3-Chlorophenyl)-1-piperazinyl]propyl}-5-methoxy-3,4-dihydro-2(1H)-quinolinone; (1S,2R)-N-[2-(3,4-Dichlorophenyl)ethyl]-N-methyl-2-(1-pyrrolidinyl)cyclohexanamine); 6-[(4-benzylpiperazin-1-yl)methyl]-2,3-dimethoxyphenol); 4-(3-(methylsulfonyl)phenyl)-1-propylpiperidine or a pharmaceutically acceptable salt thereof, preferably 2-(3-Chlorophenyl)-2-oxo-3,3,5,5-tetramethyl-[1 ,4,2]-oxazaphosphinane or 2-morpholin-4-ylethyl 1-phenylcyclohexane-1-carboxylate or a pharmaceutically acceptable salt thereof.

**18.** Compound of formula (I):

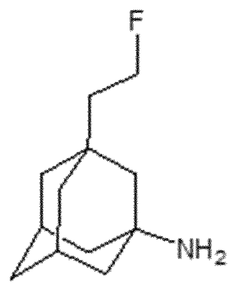

(I)

or a pharmaceutically acceptable salt thereof for use in preventing or treating a neurodegenerative pathology.

**19.** Compound of formula (I):

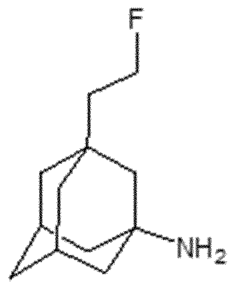

(I)

or a pharmaceutically acceptable salt thereof for use in preventing or decreasing cognitive impairment in a subject suffering from, suspected of suffering from or considered to be at risk of suffering from a neurodegenerative pathology.

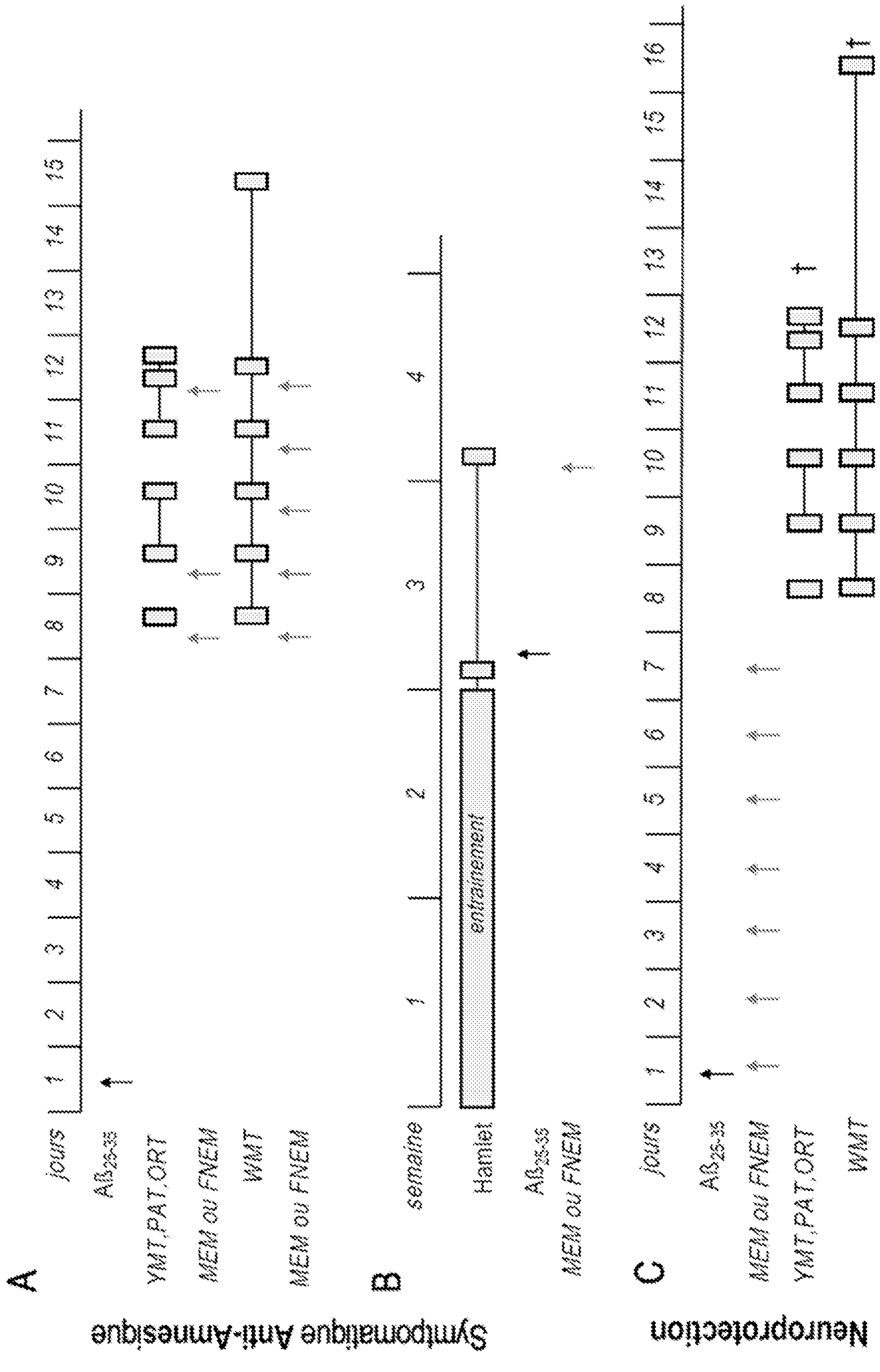
Symptomatique Anti-Amnesique
Neuroprotection
A
jours
$A\beta_{25-35}$
YMT,PAT,ORT
MEM ou FNEM
WMT
MEM ou FNEM
1 2 3 4 5 6 7 8 9 10 11 12 13 14 15
B
semaine
Hamlet
entrainement
$A\beta_{25-35}$
MEM ou FNEM
1 2 3 4
C
jours
$A\beta_{25-35}$
MEM ou FNEM
YMT,PAT,ORT
WMT
1 2 3 4 5 6 7 8 9 10 11 12 13 14 15 16

FIGURE 1

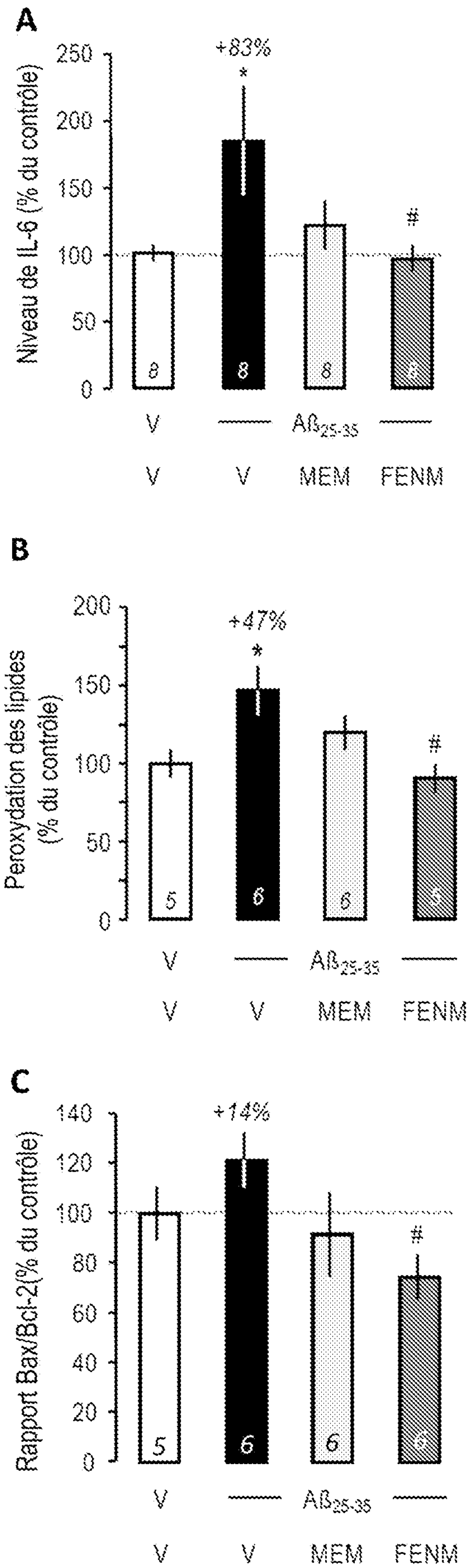
A
Niveau de IL-6 (% du contrôle)
250
200
150
100
50
0
+83%
*
#
8
8
8
8
V
Aß25-35
V
V
MEM
FENM
B
Peroxydation des lipides
(% du contrôle)
200
150
100
50
0
+47%
*
#
5
6
6
5
V
Aß25-35
V
V
MEM
FENM
C
Rapport Bax/Bcl-2(% du contrôle)
140
120
100
80
60
40
20
0
+14%
#
5
6
6
6
V
Aß25-35
V
V
MEM
FENM

FIGURE 2

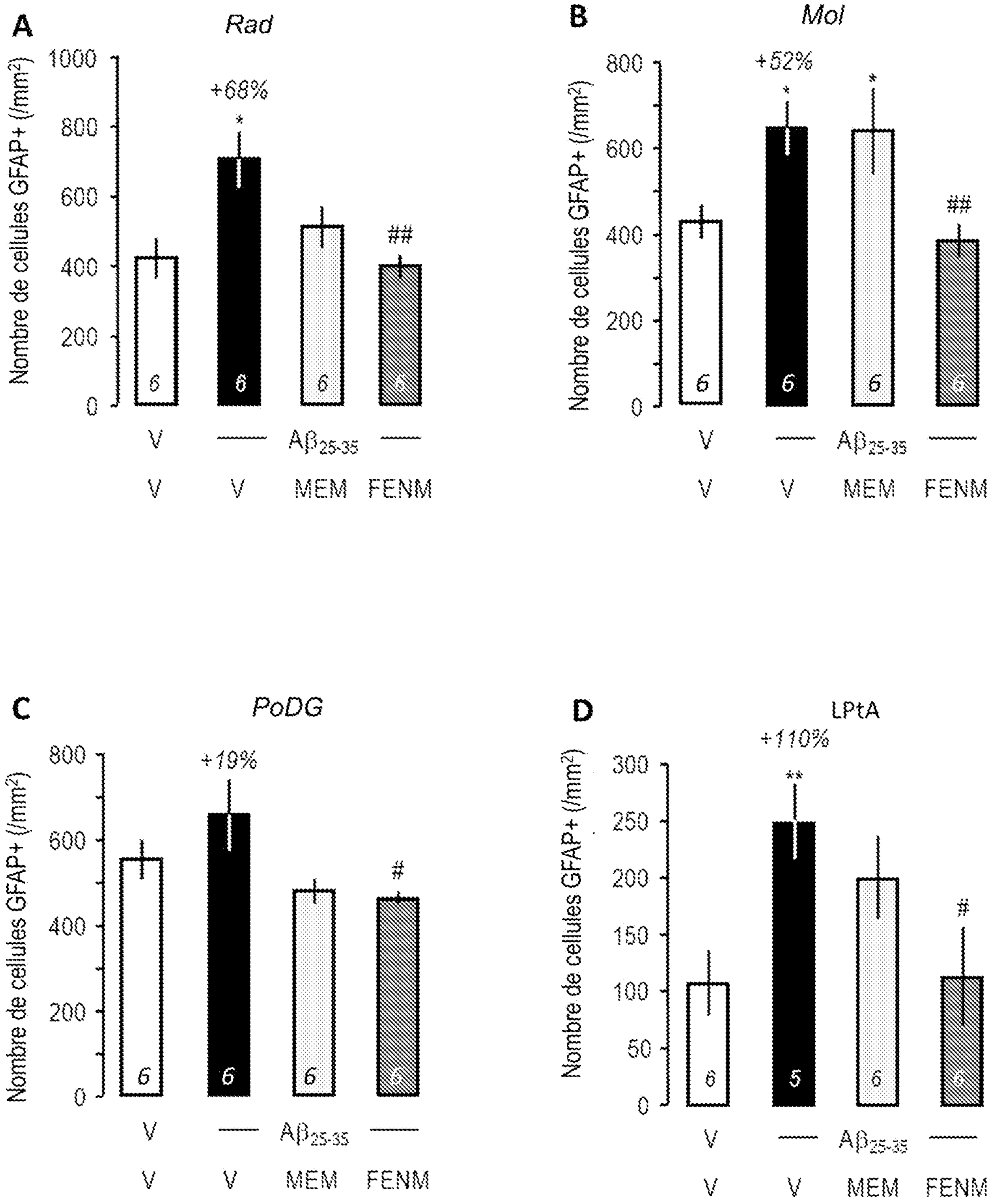
A
Rad
Nombre de cellules GFAP+ (/mm²)
1000
800
600
400
200
0
+68%
*
##
6
6
6
6
V
Aβ25-35
V
V
MEM
FENM
B
Mol
Nombre de cellules GFAP+ (/mm²)
800
600
400
200
0
+52%
*
*
##
6
6
6
6
V
Aβ25-35
V
V
MEM
FENM
C
PoDG
Nombre de cellules GFAP+ (/mm²)
800
600
400
200
0
+19%
#
6
6
6
6
V
Aβ25-35
V
V
MEM
FENM
D
LPtA
Nombre de cellules GFAP+ (/mm²)
300
250
200
150
100
50
0
+110%
**
#
6
5
6
6
V
Aβ25-35
V
V
MEM
FENM

**FIGURE 3**

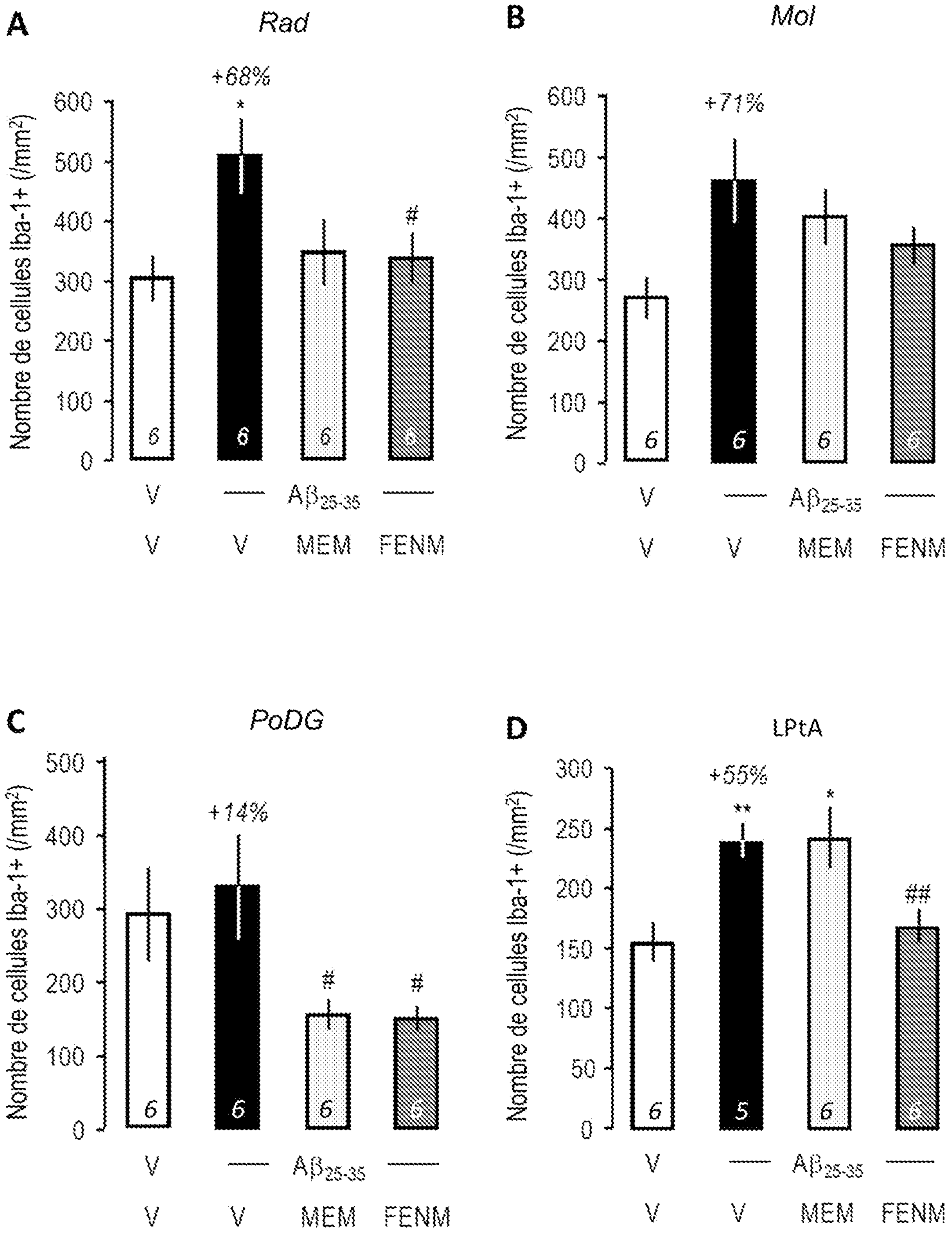
A
Rad
Nombre de cellules Iba-1+ (/mm²)
600
500
400
300
200
100
0
+68%
*
#
6
6
6
6
V
Aβ25-35
V
V
MEM
FENM
B
Mol
Nombre de cellules Iba-1+ (/mm²)
600
500
400
300
200
100
0
+71%
6
6
6
6
V
Aβ25-35
V
V
MEM
FENM
C
PoDG
Nombre de cellules Iba-1+ (/mm²)
500
400
300
200
100
0
+14%
#
#
6
6
6
6
V
Aβ25-35
V
V
MEM
FENM
D
LPtA
Nombre de cellules Iba-1+ (/mm²)
300
250
200
150
100
50
0
+55%
**
*
##
6
5
6
6
V
Aβ25-35
V
V
MEM
FENM

**FIGURE 4**

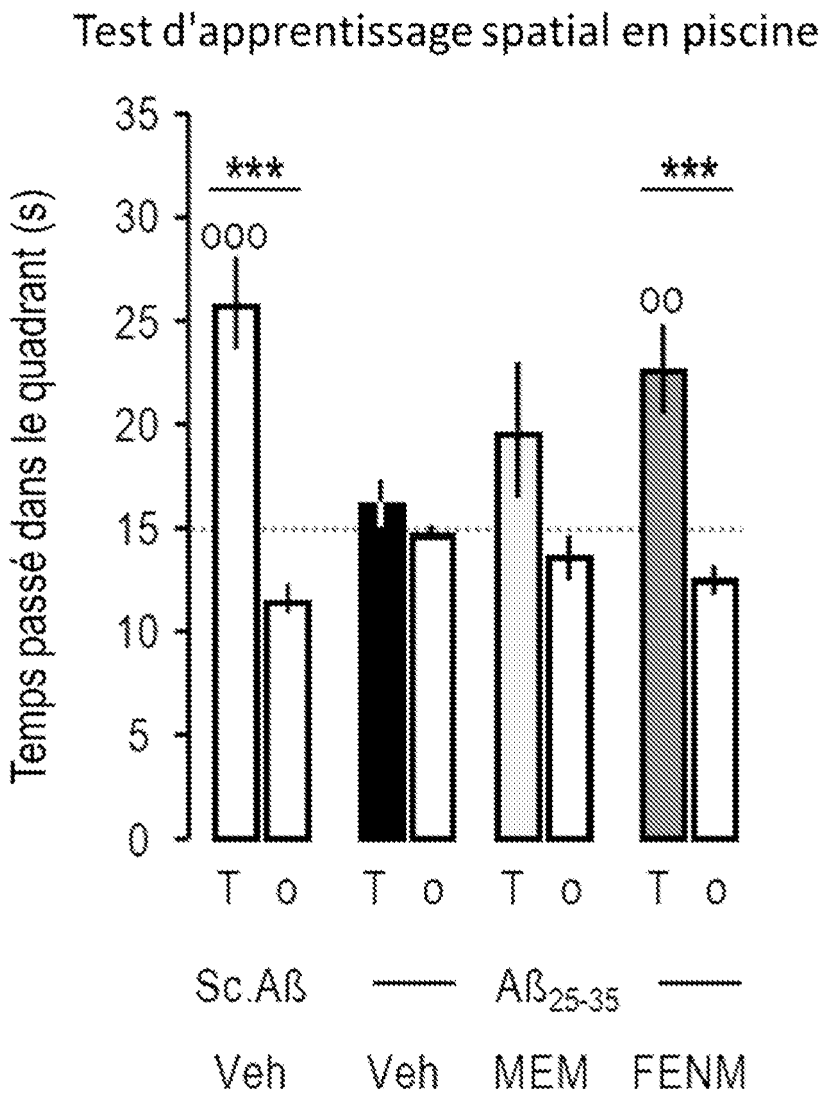
Test d'apprentissage spatial en piscine
Temps passé dans le quadrant (s)
35
30
25
20
15
10
5
0
***
***
ooo
oo
T o T o T o T o
Sc.Aß
$Aß_{25-35}$
Veh
Veh
MEM
FENM

**FIGURE 5**

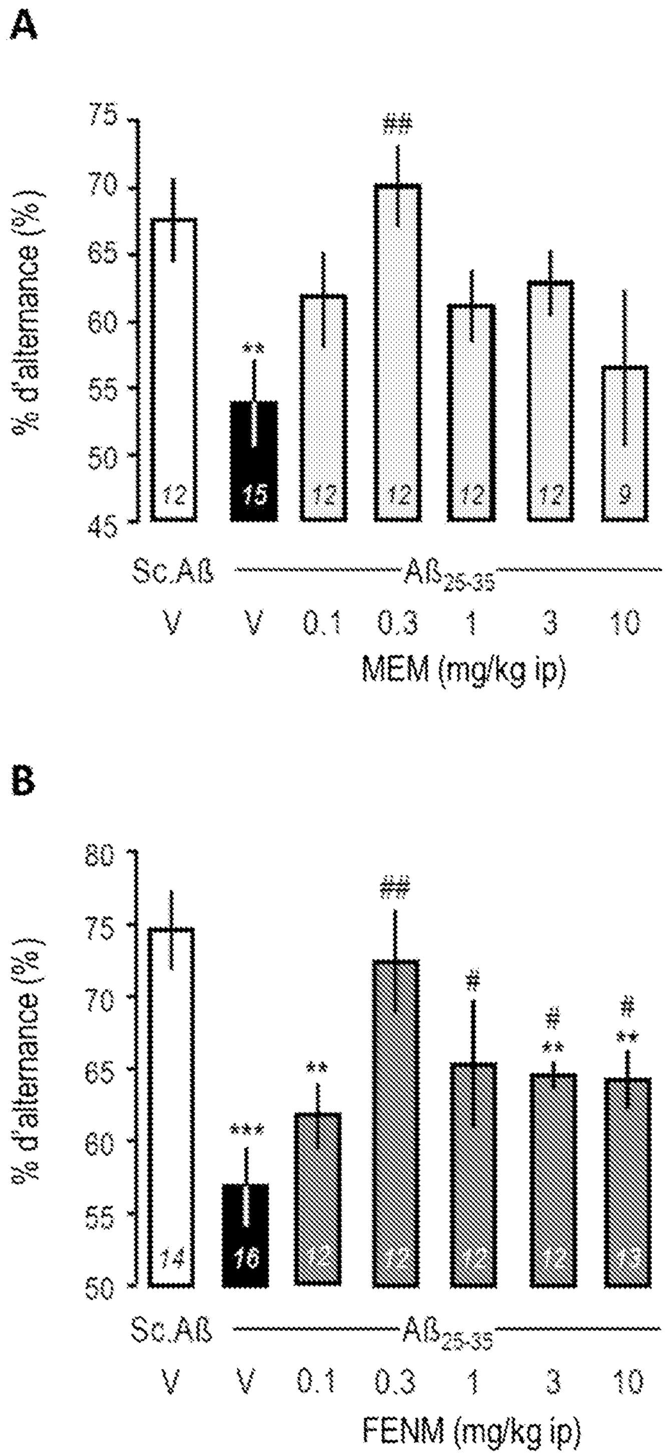
A
75
70
65
60
55
50
45
% d'alternance (%)
##
**
12
15
12
12
12
12
9
Sc.Aß
Aß25-35
V
V
0.1
0.3
1
3
10
MEM (mg/kg ip)
B
80
75
70
65
60
55
50
% d'alternance (%)
##
#
#
#
***
**
**
**
14
16
Sc.Aß
Aß25-35
V
V
0.1
0.3
1
3
10
FENM (mg/kg ip)

FIGURE 6

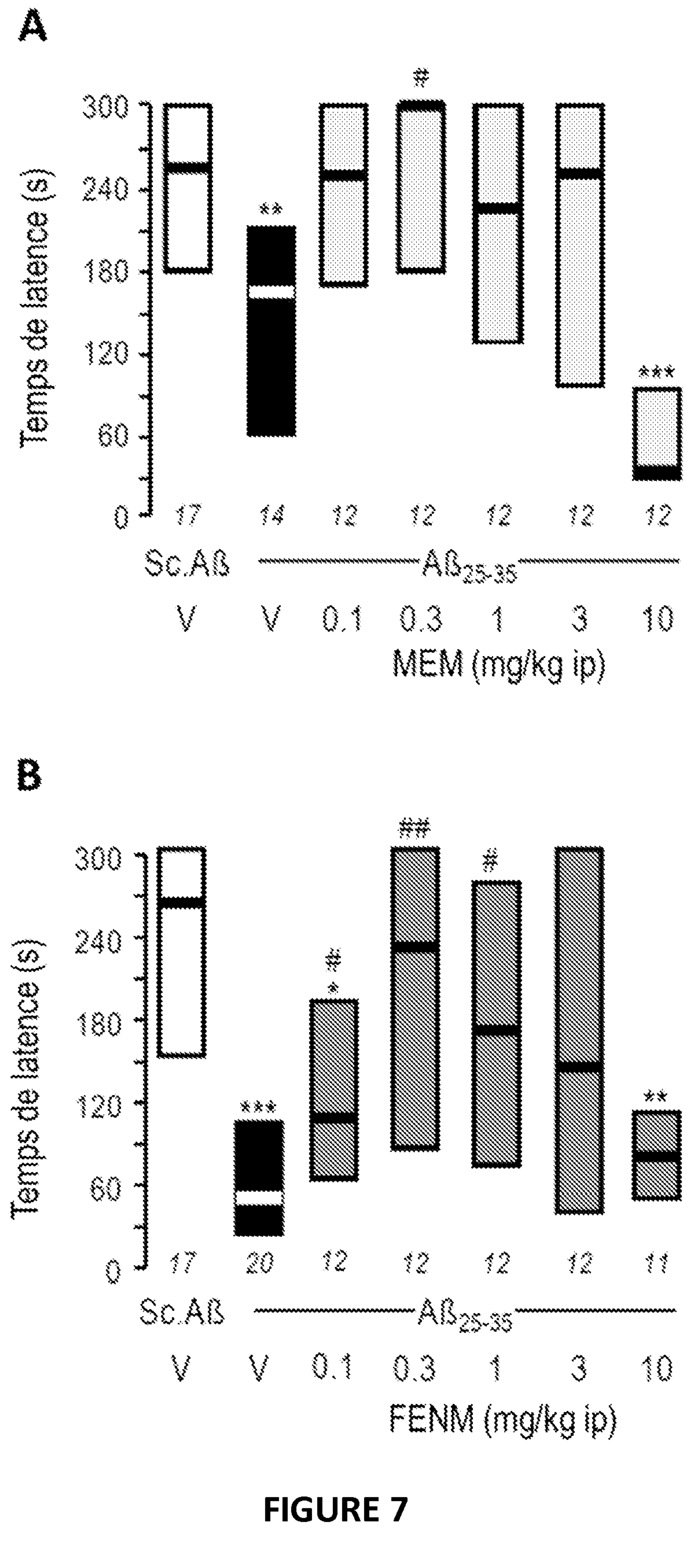
A
#
300
240
180
120
60
0
Temps de latence (s)
**
***
17
14
12
12
12
12
12
Sc.Aß
Aß25-35
V
V
0.1
0.3
1
3
10
MEM (mg/kg ip)
B
##
#
#
*
***
**
300
240
180
120
60
0
Temps de latence (s)
17
20
12
12
12
12
11
Sc.Aß
Aß25-35
V
V
0.1
0.3
1
3
10
FENM (mg/kg ip)

**FIGURE 7**

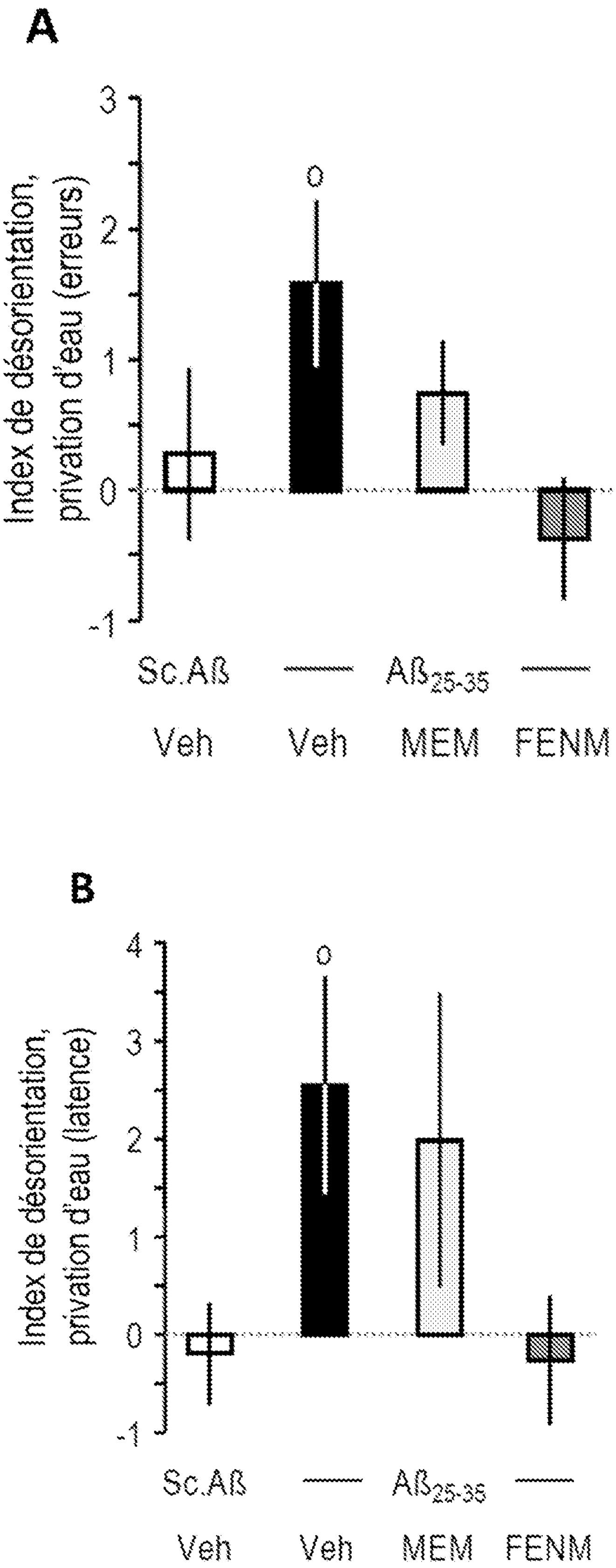
A
3
2
1
0
-1
o
Index de désorientation,
privation d'eau (erreurs)
Sc.Aß
Aß25-35
Veh
Veh
MEM
FENM
B
4
3
2
1
0
-1
o
Index de désorientation,
privation d'eau (latence)
Sc.Aß
Aß25-35
Veh
Veh
MEM
FENM

**FIGURE 8**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2009062391 A
- WO 2014191424 A
- WO 2019115833 A
- WO 2013064579 A
- WO 2017191034 A
- WO 2010029131 A


### Littérature non-brevet citée dans la description


- **ARNDT, J.W. ; QIAN, F. ; SMITH, B.A. et al.** Structural and kinetic basis for the selectivity of aducanumab for aggregated forms of amyloid-β. *Sci Rep,* 2018, vol. 8, 6412
- **BEAURAIN M. ; SALABERT A.S. ; RIBEIRO M.J. ; ARLICOT N. ; DAMIER P. ; LE JEUNE F. ; DEMONET J.F. ; PAYOUX P.** Innovative Molecular Imaging for Clinical Research, Therapeutic Stratification, and Nosography in Neuroscience. *Frontiers in Medicine,* 2019, vol. 6, 268
- **CREELEY C ; WOZNIAK DF ; LABRUYERE J ; TAYLOR GT ; OLNEY JW.** Low doses of memantine disrupt memory in adult rats. *J Neurosci.,* 2006, vol. 26 (15), 3923-3932
- **CROUZIER L ; GILABERT D ; ROSSE! M ; TROUSSE F ; MAURICE T.** Topographical memory analyzed in mice using the Hamlet Test, a novel complex maze. *Neurobiol Learn Mem.,* 2018, vol. 149, 118-134
- **CROUZIER L ; MAURICE T.** Assessment of topographie memory in mice in a complex environment using the Hamlet test. *Current Protoc Mouse Biol,* 2018, vol. 8, e43
- **GRUDEN MA ; DAVIDOVA TB ; MALISAUSKAS M ; SEWELL RD ; VOSKRESENSKAYA NI ; WILHELM K ; ELISTRATOVA EL ; SHERSTNEV VV ; MOROZOVA-ROCHE LA.** Differential neuroimmune markers to the onset of Alzheimer's disease neurodegeneration and dementia: autoantibodies to Abeta((25-35)) oligomers, S100b and neurotransmitters. *J Neuroimmunol.,* 02 Mai 2007, vol. 186 (1-2), 181-92
- **KNIGHT R ; KHONDOKER M ; MAGILL N ; STEWART R ; LANDAU S.** A Systematic Review and Meta-Analysis of the Effectiveness of Acetylcholinesterase Inhibitors and Memantine in Treating the Cognitive Symptoms of Dementia. *Dement Geriatr Cogn Disord,* 2018, vol. 45, 131-151
- **LIU CC ; LIU CC ; KANEKIYO T ; XU H ; BU G ; APOLIPOPROTEIN E.** Alzheimer disease: risk, mechanisms and therapy. *Nat Rev Neurol.,* 2013
- **LIU, CHIA-CHAN.** *Nat Rev Neurol.,* 2013, vol. 9 (2), 106-118
- **MAURICE T ; HIRAMATSU M ; ITOH J ; KAMEYAMA T ; HASEGAWA T ; NABESHIMA T.** Behavioral evidence for a modulating rôle of sigma ligands in memory processes. I. Atténuation of dizocilpine (MK-801)-induced amnesia. *Brain Res.,* 1994, vol. 647, 44-56
- **MAURICE T ; LOCKHART BP ; PRIVAT A.** Amnesia induced in mice by centrally administered β-amyloid peptides involves cholinergic dysfunction. *Brain Res.,* 1996, vol. 706, 181-93
- **MAURICE T ; MUSTAFA MH ; DESRUMAUX C ; KELLER E ; NAERT G ; DE LA C. GARCÍA-BARCELÓ M ; RODRÍGUEZ CRUZ Y ; GARCIA RODRÍGUEZ JC.** Intranasal formulation of erythropoietin (EPO) showed potent protective activity against amyloid toxicity in the Aβ25-35 nontransgenic mouse model of Alzheimer's disease. *J Psychopharmacol.,* 2013, vol. 27, 1044-57
- **MAURICE T ; VOLLE JN ; STREHAIANO M ; CROUZIER L ; PEREIRA C ; KALOYANOV N ; VIRIEUX D ; PIRAT JL.** Neuroprotection in non-transgenic and transgenic mouse models of Alzheimer's disease by positive modulation of σ1 receptors. *Pharmacol Res.,* 2019, vol. 144, 315-330
- **MEUNIER J ; LENI J ; MAURICE T.** The anti-amnesic and neuroprotective effects of donepezil against amyloid β25-35 peptide-induced toxicity in mice involve an interaction with the σ1 receptor. *Br J Pharmacol.,* 2006, vol. 149, 998-1012
- **MEUNIER J ; VILLARD V ; GIVALOIS L ; MAURICE T.** The γ-secretase inhibitor 2-[(1R)-1-[(4-chlorophenyl)sulfonyl] (2,5-difluorophenyl)amino]ethyl-5-fluorobenzenebutanoic acid (BMS-299897) alleviates Aβ1-42 seeding and short-term memory déficits in the AP25-35 mouse model of Alzheimer's disease. *Eur J Pharmacol.,* 2013, vol. 698, 193-9
- **PAXINOS G ; FRANKLIN KBJ.** The Mouse Brain in Stereotaxic Coordinates. Academic Press, 2004

- **PIKE CJ ; WALENCEWICZ AJ ; GLABE CG ; COTMAN CW.** In vitro aging of beta-amyloid protein causes peptide aggregation and neurotoxicity. *Brain Res.,* 01 Novembre 1991, vol. 563 (1-2), 311-4
- **RODRÍGUEZ CRUZ Y ; STREHAIANO M ; RODRÍGUEZ OBAYA T ; GARCÍA RODRÍGUEZ JC ; MAURICE T.** An intranasal formulation of erythropoietin (Neuro-EPO) prevents memory déficits and amyloid toxicity in the APPSwe transgenic mouse model of Alzheimer’s disease. *J Alz Dis.,* 2017, vol. 55, 231-248
- **STAHL HP ; WERMUTH CG.** Handbook of Pharmaceutical Salts: Properties, Sélection, and Use
- **VILLARD V ; ESPALLERGUES J ; KELLER E ; ALKAM T ; NITTA A ; YAMADA K ; NABESHIMA T ; VAMVAKIDES A ; MAURICE T.** Anti-amnesic and neuroprotective effects of the aminotetrahydrofuran derivative ANAVEX1-41 against amyloid ß25-35-induced toxicity in mice. *Neuropsychopharmacology.,* 2009, vol. 34, 1552-66
- **VILLARD V ; ESPALLERGUES J ; KELLER E ; VAMVAKIDES A ; MAURICE T.** Anti-amnesic and neuroprotective potentials of the mixed muscarinic receptor/sigma1 ($\sigma$1) ligand ANAVEX2-73, a novel aminotetrahydrofuran derivative. *J Psychopharmacol.,* 2011, vol. 25, 1101-17